# EUROPEAN PATENT APPLICATION

(11) **EP 4 223 864 A1**
(43) Date of publication of application: **09.08.2023**
(21) Application number: 21872588.5
(22) Date of filing: 24.09.2021
(51) Int. Cl.: C12M 1/26, C12N 1/00

(54) **METHOD FOR CONTROLLING LIVING BODY AND DEVICE FOR CONTROLLING LIVING BODY**

(30) Priority: 24.09.2020 JP 2020160261
(71) Applicant: NIKON CORPORATION, Minato-ku Tokyo 108-6290 (JP)
(72) Inventor: MORIYAMA Masaki, Tokyo 108-6290 (JP); ISHIZAWA Naoya, Tokyo 108-6290 (JP); KOBAYASHI Ryo, Tokyo 108-6290 (JP); NAKAMURA Taichi, Tokyo 108-6290 (JP); TANAKA Shuhei, Tokyo 108-6290 (JP); HAYASHI Seri, Tokyo 108-6290 (JP); TAKUBO Makiko, Tokyo 108-6290 (JP)
(74) Representative: Hoffmann Eitle
(86) International application number: PCT/JP2021/035201
(87) International publication number: WO 2022/065459

(57) **Abstract**

A first aspect of the present invention provides a manipulating method of an organism including forming an air bubble in liquid where the organism is immersed, attaching the organism to the air bubble, and controlling an airflow by generating the airflow in the air bubble and manipulating a position of the organism with the airflow.

## Description

### TECHNICAL FIELD

The present invention relates to a method of manipulating an organism and an organism manipulation device.

### BACKGROUND

In research on cell biology and the like, suction of a particular cell from many cells in a culture vessel has been conducted. Patent document 1 discloses a system for supporting suction work targeting a characteristic cell in a large quantity of cells.

Patent document 1: Japanese Patent Application Publication No. 2019-030263

### GENERAL DISCLOSURE

According to a first aspect of the present invention, a manipulating method of an organism is provided. The manipulating method of an organism may include an air bubble forming step for forming an air bubble in liquid in which the organism has been immersed. The manipulating method of an organism may include an organism attaching step for attaching the organism to the air bubble. The manipulating method of an organism may include an airflow controlling step for generating an airflow in the air bubble and manipulating a position of the organism with the airflow.

According to a second aspect of the present invention, the air bubble forming step may be performed by immersing an end part of a flow channel in liquid, and introducing gas into the liquid from the end part. Further, the airflow controlling step may include generating the airflow in the air bubble by moving a relative position between the flow channel and the liquid.

According to a third aspect of the present invention, the air bubble forming step may include forming an air bubble on the end part of the flow channel and maintaining the air bubble.

According to a fourth aspect of the present invention, the airflow controlling step may include generating the airflow in the air bubble by moving a relative position between the flow channel and a surface of a vessel storing the liquid that is in contact with the organism, in a direction within a range of ±20° from the horizontal direction.

According to a fifth aspect of the present invention, the airflow controlling step may include generating the airflow in the air bubble by moving the relative position between the flow channel and a surface of a vessel storing the liquid that is in contact with the organism, in a direction within a range of ±20° from the vertical direction.

According to a sixth aspect of the present invention, the liquid and the organism may be contained in a vessel, and the airflow controlling step may include moving the relative position between the flow channel and the liquid in a state where the air bubble is in contact with a surface of the vessel that is in contact with the organism.

According to a seventh aspect of the present invention, the airflow controlling step may include generating the airflow in the air bubble by changing a volume of the air bubble.

According to an eighth aspect of the present invention, the airflow controlling step may include generating the airflow in the air bubble by increasing the volume of the air bubble.

According to a ninth aspect of the present invention, the airflow controlling step may include generating the airflow in the air bubble by reducing the volume of the air bubble.

According to a tenth aspect of the present invention, the air bubble forming step may be performed by immersing an end part of a flow channel in liquid, and introducing gas into the liquid from the end part. Further, the flow channel may include a gas supply flow channel for supplying the gas and a gas recovery flow channel for recovering the gas. The airflow controlling step may include generating an airflow where the gas supplied from an end part of the gas supply flow channel passes through the air bubble and flows toward an end part of the gas recovery flow channel.

According to an eleventh aspect of the present invention, the flow channel may have a double tube structure, and the gas supply flow channel may be one flow channel out of an inner side or an outer side in the double tube structure, whereas the gas recovery flow channel may be the other flow channel out of the inner side or the outer side in the double tube structure.

According to a twelfth aspect of the present invention, a recovery step of recovering the organism from the liquid may be further included after the airflow controlling step.

According to a thirteenth aspect of the present invention, an organism manipulation device for manipulating an organism is provided. The organism manipulation device may include a flow channel that can introduce gas into liquid, an end part of the flow channel being immersed in the liquid where the organism is immersed. The organism manipulation device may include an air bubble control unit which generates an airflow in an air bubble formed by introducing the gas into the liquid from the end part and manipulates a position of the organism with the airflow.

According to a fourteenth aspect of the present invention, the air bubble control unit may control to maintain the air bubble formed on the end part.

According to a fifteenth aspect of the present invention, the air bubble control unit may have a flow channel position control unit which controls an actuator for moving the flow channel, thereby controlling the airflow in the air bubble.

According to a sixteenth aspect of the present invention, the air bubble control unit may have a stage position control unit which controls an actuator for moving a stage equipped with a vessel for containing the organism, thereby controlling the airflow in the air bubble.

According to a seventeenth aspect of the present invention, the air bubble control unit may have a volume control unit which controls a pump connected to the flow channel, thereby controlling the volume of the air bubble in the liquid.

According to an eighteenth aspect of the present invention, an organism manipulation device is provided. The organism manipulation device may include a flow channel having an end part arranged in liquid including an organism, the liquid being stored in a vessel. The organism manipulation device may include a pump for introducing gas into the flow channel to form an air bubble. The organism manipulation device may include a position control unit which is able to change a position of the vessel or the flow channel. The pump or the position control unit may attach the organism to a gas-liquid interface of the air bubble. The position control unit may move the flow channel in an opposing direction of the organism with a central axis of the flow channel being a reference, in a vertical directional view.

Note that, the summary clause does not necessarily describe all necessary features of the embodiments of the present invention. The invention may also include a sub-combination of the features described above.

### BRIEF DESCRIPTION OF THE DRAWINGS

FIG. 1A illustrates an example of a device configuration of an organism manipulation device 100 in the present embodiment.
FIG. 1B illustrates an example of the device configuration of the organism manipulation device 100 in the present embodiment.
FIG. 2A illustrates an example of a schematic view showing a structure of a nozzle 49 in the present embodiment.
FIG. 2B illustrates an example of the schematic view showing the structure of the nozzle 49 in the present embodiment.
FIG. 3A illustrates an example of the schematic view showing the structure of the nozzle 49 in the present embodiment.
FIG. 3B illustrates an example of the schematic view showing the structure of the nozzle 49 in the present embodiment.
FIG. 4 illustrates an example of a schematic view showing an example of a method of recovering an organism in the present embodiment.
FIG. 5 illustrates an example of a specific configuration of an information processing device 170 in the present embodiment.
FIG. 6 illustrates an example of a flow of a method of manipulating an organism in the present embodiment.
FIG. 7A illustrates an example of a GUI image displayed on an output unit 160 in the present embodiment.
FIG. 7B illustrates an example of the GUI image displayed on the output unit 160 in the present embodiment.
FIG. 7C illustrates an example of the GUI image displayed on the output unit 160 in the present embodiment.
FIG. 7D illustrates an example of the GUI image displayed on the output unit 160 in the present embodiment.
FIG. 7E illustrates an example of the GUI image displayed on the output unit 160 in the present embodiment.
FIG. 8 illustrates an example of a flow of a replacement or addition processing of liquid in S600 in the present embodiment.
FIG. 9A illustrates an example of a flow of moving a relative position between the nozzle 49 and a cell in S200 in the present embodiment.
FIG. 9B illustrates an example of the flow of moving a relative position between the nozzle 49 and a cell in S200 in the present embodiment.
FIG. 9C illustrates an example of the flow of moving a relative position between the nozzle 49 and a cell in S200 in the present embodiment.
FIG. 10A illustrates an example of a flow of forming an air bubble in S300 in the present embodiment.
FIG. 10B illustrates an example of the flow of forming an air bubble in S300 in the present embodiment.
FIG. 11A illustrates an example of a flow of performing a manipulation in S400 in the present embodiment.
FIG. 11B illustrates an example of recovering a cytoplasm and a cell membrane from a cell in the present embodiment.
FIG. 11C illustrates an example of attaching a cell to an air bubble and separating the cell in the present embodiment.
FIG. 11D illustrates an example of a schematic view showing a method of recovering a cell in the present embodiment.
FIG. 11E illustrates an example of subcultured cells in the present embodiment.
FIG. 11F illustrates an example of subcultured cells in the present embodiment.
FIG. 11G illustrates an example of analyzing a recovered cell in the present embodiment.
FIG. 11H illustrates an example of a schematic view showing a retained cell in the present embodiment.
FIG. 11I illustrates an example of a compressed cell in the present embodiment.
FIG. 12A is an example of a flow of removing an air bubble in S500 in the present embodiment.
FIG. 12B is an example of the flow of removing an air bubble in S500 in the present embodiment.
FIG. 13 illustrates an example of a flow of controlling an airflow in an air bubble 256 to which a manipulation target 35 is attached in the present embodiment.
FIG. 14 illustrates an example of a method of generating an airflow in the air bubble 256 and manipulating a position of the manipulation target 35 with the airflow in the present embodiment.
FIG. 15 illustrates an example of a schematic view describing a method 914 of moving the nozzle 49 in a horizontal direction in the present embodiment.
FIG. 16 illustrates an example of a schematic view describing a moving direction of the manipulation target 35 when the nozzle 49 is moved in the horizontal direction in the present embodiment.
FIG. 17 illustrates an example of a schematic view describing a method 915 of moving the nozzle 49 in a vertical direction in the present embodiment.
FIG. 18 illustrates an example of a schematic view describing a moving direction of the manipulation target 35 when the nozzle 49 is moved in the vertical direction in the present embodiment.
FIG. 19 illustrates an example of a schematic view describing a method 917 of increasing a volume of the air bubble 256, and a method 918 of depressurizing the air bubble 256 in the present embodiment.
FIG. 20 illustrates an example of a schematic view describing a moving direction of the manipulation target 35 when a volume of the air bubble 256 is changed in the present embodiment.
FIG. 21 illustrates an example of a schematic view describing a method 913 of generating an airflow by using a double tube nozzle 49a in the present embodiment.
FIG. 22 illustrates an example of a schematic view describing a moving direction of the manipulation target 35 when the double tube nozzle 49a is used in the present embodiment.
FIG. 23 is an experimental image showing a movement of the manipulation target 35 due to a movement of the air bubble 256 in the present embodiment.
FIG. 24 is an experimental image illustrating a position control on the manipulation target 35 due to an enlargement of the air bubble 256 in the present embodiment.
Fig. 25 illustrates an example of a hardware configuration of a computer.

### DESCRIPTION OF EXEMPLARY EMBODIMENTS

Hereinafter, the present invention will be described through embodiments of the invention, but the following embodiments do not limit the invention according to claims. In addition, not all combination of the features described in the embodiments are necessary for the solution of the invention. Note that in the drawings, same or similar parts are assigned with same reference signs, and duplicated descriptions may be omitted.

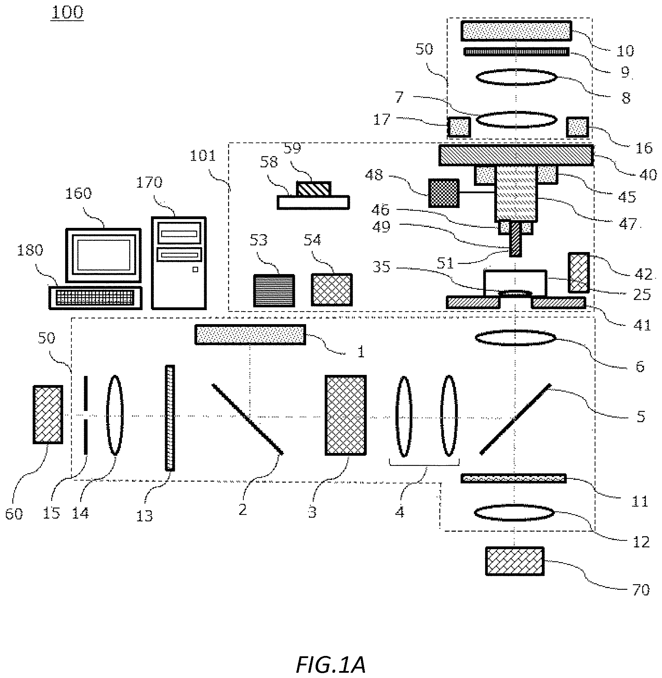

Fig. 1A illustrates an example of a device configuration of an organism manipulation device 100 in the present embodiment. The organism manipulation device 100 according to the present invention manipulates a minute organism such as a cell by using an interface between a gas and a liquid. For example, the organism manipulation device 100 can perform, on an organism, various manipulations such as attaching the organism to the interface and then separating the organism adhered on a solid phase. The organism manipulation device 100 includes a microscope unit 50, a camera 60, a camera 70, a gas-liquid interface manipulation unit 101, an output unit 160, an information processing device 170, and an input unit 180.

The microscope unit 50 is a device for observing or displaying a manipulation target 35 in an enlarged manner by using a microscope. The manipulation target 35 is an organism. The organism may be an organic creature. For example, the organism may be a cell. As an example, the cell may be an animal cell or a plant cell. As an example, the cell may be a living cell or a dead cell. In addition, for example, the organism may be a minute organism other than a cell. As an example, the minute organism may be a microorganism, a fungus, an alga, a biological tissue, a spheroid, or the like. In addition, the organism may contain an intracellular organelle.

The microscope unit 50 includes a fluorescence image observation light source 1, a dichroic mirror 2, an optical deflector 3, a relay lens 4, a dichroic mirror 5, an objective lens 6, a condenser lens 7, a condensing lens 8, a band pass filter 9, a transmission image observation light source 10, a barrier filter 11, a projection lens 12, a barrier filter 13, a projection lens 14, a pinhole 15, a light source 16, and a light source 17.

The fluorescence image observation light source 1 is a light source used when a fluorescence image of the manipulation target 35 is observed. The manipulation target 35 may be labeled with one type or two or more types of fluorescent substances, or may not be fluorescently labeled. The fluorescence image observation light source 1 emits, to the manipulation target 35, light to be excited or reflected.

The transmission image observation light source 10 is a light source used when a transmission image of the manipulation target 35 is observed. The transmission image observation light source 10 emits light to be transmitted through the manipulation target 35. The light to be transmitted through the manipulation target 35 may pass through the outside of the nozzle or may pass through the inside of the nozzle.

The configuration of the microscope unit 50 other than that described above will be described later. Note that the present invention is not limited to the example of the above description, and the microscope unit 50 may have a known configuration. For example, the configuration of the microscope unit 50 may have the configuration described in Japanese Patent Application Publication No. Hei 7-13083 or Japanese Patent No. 3814869.

The camera 60 captures a fluorescence image of the manipulation target 35 to generate an image. The image data generated by the camera 60 may be recorded in the information processing device 170 (for example, a recording unit 190 to be described later) and/or output to the output unit 160. For example, the camera 60 may be a camera that captures a fluorescence image, but is not limited thereto. In the following description, the camera 60 is a camera that captures a fluorescence image.

The camera 70 captures a transmission image of the manipulation target 35 to generate an image. The image data generated by the camera 70 may be recorded in the information processing device 170 (for example, the recording unit 190 to be described later) and/or output to the output unit 160. For example, the camera 70 may be a camera that captures a transmission image, but is not limited thereto. In the following description, the camera 70 is a camera that captures a transmission image.

The camera 60 and the camera 70 include an imaging sensor (not illustrated). The camera 60 and the camera 70 may be cooling cameras. The cooling camera is a camera that can cool the imaging sensor to suppress the noise generated by heat. The imaging sensor may be a CMOS imaging sensor (Complementary Metal Oxide Semiconductor) or a CCD imaging sensor (Charge Coupled Device). The camera 60 and the camera 70 may be housed in a housing different from the microscope unit 50.

The gas-liquid interface manipulation unit 101 manipulates the manipulation target 35 by using a gas-liquid interface between gas and liquid. For example, the gas-liquid interface manipulation unit 101 forms an air bubble in liquid, and manipulates an organism (for example, a cell) in the liquid. The gas-liquid interface manipulation unit 101 has all or at least some of a nozzle actuator 40, a sample actuator 41, a flow channel imaging camera 42, a light source 45, a light source 46, a pressure generating unit 47, a sensor unit 48, a nozzle 49, a flow channel 51, a flow channel exchange unit 53, a liquid storage part 54, a sample lid 58, and a sample lid keeping part 59.

The nozzle actuator 40 is equipped with the nozzle 49 via the pressure generating unit 47, and moves the nozzle 49. As will be described below, the flow channel 51 is formed inside the nozzle 49, and a gas-liquid interface 255 such as an air bubble is formed in an edge part of the flow channel 51. The nozzle actuator 40 may be operable in any direction of a longitudinal direction, a lateral direction, and upward and downward directions. The nozzle actuator 40 may be operable only in the upward and downward directions. In this case, movements of the nozzle actuator 40 in the longitudinal direction and the lateral direction may be operated by a stage of the microscope unit 50. The nozzle actuator 40 may be operable only in the longitudinal and lateral directions. In this case, movements of the nozzle actuator 40 in the upward and downward directions may be operated by the stage of the microscope unit 50. The nozzle actuator 40 may be fixed without operations. In this case, movements of the nozzle actuator 40 in the longitudinal and lateral directions and the upward and downward directions may be operated by the stage of the microscope unit 50. An operation of the nozzle actuator 40 is controlled by a nozzle position control unit (not illustrated) of an air bubble forming unit in the information processing device 170.

The sample actuator 41 moves a stage (not illustrated) that is equipped with a vessel 25. The sample actuator 41 may be operable in any direction of the longitudinal direction, the lateral direction, and the upward and downward directions. The stage may be equipped with the vessel 25, which is transparent, for storing the manipulation target 35. The vessel 25 may be a culture vessel that is filled with liquid. The sample actuator 41 may be equipped with one or a plurality of vessels and/or tubes, but the present invention is not limited thereto. An operation of the sample actuator 41 is controlled by a stage position control unit (not illustrated) of the air bubble forming unit in the information processing device 170. Note that, the stage may be provided for the gas-liquid interface manipulation unit 101, or may be provided for the microscope unit 50.

The flow channel imaging camera 42 images an edge part of the nozzle 49. The flow channel imaging camera 42 may image an air bubble formed in an edge part of the nozzle 49. The captured image may be sent to an image processing unit in the information processing device 170. An air bubble forming unit 200 may instruct, based on the captured image, the nozzle actuator 40 and/or the sample actuator 41 to move a relative position between the nozzle 49 and the manipulation target 35. Note that, instead of the flow channel imaging camera 42, an edge part of the nozzle 49 may be imaged with the camera 60, the camera 70, or the like. Not limited to this, in the following description, the flow channel imaging camera 42 may be a microscope accessory camera that is included in the microscope unit 50. A camera included in the microscope unit 50 may use the fluorescence image observation light source 1, the transmission image observation light source 10, the light source 16, the light source 17, the light source 45, and the light source 46, as illuminations. The light source 16 and the light source 17 may be ring illuminations, but the present invention is not limited thereto.

The light source 45 and the light source 46 illuminate the nozzle 49 and/or the manipulation target 35. The light source 45 and the light source 46 may be ring illuminations, but the present invention is not limited thereto.

The pressure generating unit 47 generates a pressure to be loaded on the flow channel 51. The pressure generating unit 47 is connected with an end of the flow channel 51 that does not contact liquid, and supplies preset gas to that end. For example, the pressure generating unit 47 may have an actuator that causes a reciprocating motion of a syringe pump and a plunger of the syringe pump. With the actuator pushing the plunger of the syringe pump toward the flow channel 51, the gas is supplied to the flow channel 51, and with the actuator pulling the plunger of the syringe pump from the flow channel 51 side, the gas may be taken in from the flow channel 51. A control on the pressure generating unit 47 is performed by the air bubble forming unit in the information processing device 170.

Liquid in which the manipulation target 35 is immersed may be a complete medium, a base medium, or a buffer solution, but the present invention is not limited thereto. A complete medium is a medium that includes a maintaining/proliferation factor necessary for maintenance/proliferation of a cell. A base medium is a culture medium that includes a very small proportion of protein, amino acid, or salt. A buffer solution is liquid that maintains pH and an osmotic pressure suitable for survival of a cell. Well-known liquids, complete media, base media, and buffer solutions can be used.

The gas may be air. The gas may include moisture.

The sensor unit 48 has one or a plurality of sensors, and senses states of the nozzle 49 and the liquid and the gas in the nozzle 49. For example, the sensor unit 48 may detect a position, a speed, and an acceleration of the nozzle 49. The sensor unit 48 may detect a position of the nozzle actuator 40, a pressure generated in the pressure generating unit 47, a position of the plunger of the syringe pump in the pressure generating unit 47, and the like. The sensor unit 48 may detect an environmental temperature, and a temperature of the liquid in the vessel 25. The sensor unit 48 may detect a humidity of the environment. In addition, the sensor unit 48 may detect pH of the liquid in the vessel 25. The sensor unit 48 may detect the temperature and the humidity of the gas in the nozzle 49. The sensor unit 48 sends the information to the information processing device 170 (for example, the air bubble forming unit 200 to be described later). A well-known sensor can be used for the sensor unit 48. Note that, the sensor unit 48 may be a different housing from the pressure generating unit 47, or may be stored inside the pressure generating unit 47.

The nozzle 49 is equipment that includes the flow channel 51 to be described later. The nozzle 49 may be rod-shaped or flat plate-shaped.

The liquid and the gas that are suctioned (taken in) or discharged (supplied) pass through the flow channel 51. The flow channel 51 is provided inside the nozzle 49 so as to penetrate the nozzle 49 in the longitudinal direction. The pressure generating unit 47 is connected to the other end of the flow channel 51.

The flow channel exchange unit 53 is equipment for keeping and discarding the nozzle 49. When exchanging the nozzle 49, the flow channel exchange unit 53 may remove the nozzle 49 mounted on the nozzle actuator 40 and discard it in a nozzle discarding part (not illustrated) of the flow channel exchange unit 53, and may instead mount the nozzle 49 that is kept in a nozzle keeping part (not illustrated) of the flow channel exchange unit 53 on the nozzle actuator 40. The flow channel exchange unit 53 may be omitted, and in that case, the nozzle 49 may be exchanged by the hands of a manipulator.

The liquid storage part 54 is equipment for keeping liquid to be supplied to the vessel 25, and recovering the liquid from the vessel 25 for discarding. When exchanging the liquid, the liquid storage part 54 may recover the liquid contained in the vessel 25 from the vessel 25 and discard it in a liquid discarding part (not illustrated) of the liquid storage part 54, and may replenish the liquid kept in a liquid keeping part (not illustrated) of the liquid storage part 54 to the vessel 25. Regarding the exchange of the liquid, the same type of liquids may be exchanged. Regarding the exchange of the liquid, different types of liquids may be exchanged. The liquid storage part 54 may be omitted, and in that case, the liquid may be exchanged by the hands of the manipulator.

The sample lid 58 is a lid to be attached to the vessel 25. The sample lid 58 may be attached to the vessel 25, or may be stored in the sample lid keeping part 59. The sample lid 58 may be, as necessary, taken out from the sample lid keeping part 59 and attached to the vessel 25, and removed from the vessel 25 and stored in the sample lid keeping part 59, with a sample lid actuator (not illustrated). In this case, an operation of the sample lid actuator may be controlled with a sample lid control unit (not illustrated) of the air bubble forming unit 200 in the information processing device 170. The sample lid 58 and the sample lid keeping part 59 may be omitted, and in that case, the sample lid 58 may be attached to the vessel 25 and removed from the vessel 25 with the hands of the manipulator.

The output unit 160 outputs a processing result of the information processing device 170. For example, the output unit 160 outputs an image on which image processing is performed inside the information processing device 170 (for example, an image processing unit 300 to be described later). For example, the output unit 160 is a monitor that is connected to the information processing device 170.

The information processing device 170 exchanges instructions and data with the microscope unit 50, the camera 60, the camera 70, the gas-liquid interface manipulation unit 101, the output unit 160, and the input unit 180. For example, the information processing device 170 is connected to the microscope unit 50 and the gas-liquid interface manipulation unit 101, and controls the microscope unit 50 and the gas-liquid interface manipulation unit 101.

Specifically, the information processing device 170 switches a combination of a type of the objective lens 6 and/or a type of a filter cube of a fluorescent filter arranged in an optical path of the microscope unit 50. For example, regarding transmission image observation and fluorescence image observation, both the type of the filter cube and the type of the objective lens 6 arranged in the optical path are different. In addition, regarding two types of fluorescence image observation, only the type of the filter cube arranged in the optical path is different. Furthermore, regarding transmission image observation and fluorescence image observation, light sources that are used (the transmission image observation light source 10 and the fluorescence image observation light source 1, respectively) are also different. Thus, an inside (for example, an imaging control unit 171 to be described later) of the information processing device 170 may switch one or more of a filter block, the objective lens 6, and a light source depending on whether to perform at least any one of or a plurality of transmission image observation and one type or two or more types of fluorescence image observation.

When performing fluorescence image observation, the information processing device 170 turns on the fluorescence image observation light source 1, and turns off the transmission image observation light source 10, to make the optical path of the fluorescence image observation light source 1 effective. When performing fluorescence image observation, a light emitted from the fluorescence image observation light source 1 illuminates the manipulation target 35 via the dichroic mirror 2, the optical deflector 3, the relay lens 4, the dichroic mirror 5, and the objective lens 6.

When the manipulation target 35 is fluorescently labeled, a fluorescent substance of the manipulation target 35 is excited, and fluorescence is produced. The fluorescence produced from the manipulation target 35 reaches a light receiving surface of the camera 60 via the objective lens 6, the dichroic mirror 5, the relay lens 4, the optical deflector 3, the dichroic mirror 2, the barrier filter 13, the projection lens 14, and the pinhole 15 (when the microscope unit 50 is a confocal microscope). At this time, a fluorescence image of the manipulation target 35 is formed in the camera 60. Note that, even when the manipulation target 35 is not fluorescently labeled, a light emitted from the fluorescence image observation light source 1 hits on the manipulation target 35, and the manipulation target 35 can be observed by using a light reflected from the manipulation target 35.

The information processing device 170 turns on the transmission image observation light source 10, and turns off the fluorescence image observation light source 1, to make the optical path of the transmission image observation light source 10 effective. When performing transmission image observation, a light emitted from the transmission image observation light source 10 illuminates the manipulation target 35 via the band pass filter 9, the condensing lens 8, and the condenser lens 7. The light transmitted through the manipulation target 35 reaches a light receiving surface of the camera 70 via the objective lens 6, the dichroic mirror 5, the barrier filter 11, and the projection lens 12. At this time, a transmission image of the manipulation target 35 is formed in the camera 70. Note that, when an end part of the nozzle 49 is hard to see with fluorescence observation, transmission image observation may also be performed.

In addition, the information processing device 170 controls a relative position between the nozzle 49 of the gas-liquid interface manipulation unit 101 and the stage. Furthermore, in addition to the control on the microscope unit 50 and the gas-liquid interface manipulation unit 101, the information processing device 170 may perform an image processing such as generating one composite image from a plurality of images upon receiving the manipulation target 35 captured with the camera 60 and/or the camera 70, and/or an image that is captured with the flow channel imaging camera 42 of the gas-liquid interface manipulation unit 101. The information processing device 170 may perform, as necessary, controls on other operations of the organism manipulation device 100, data processing, and the like. Configurations of the information processing device 170 will be described below.

The input unit 180 inputs instructions from the manipulator, data, or the like to the information processing device 170. For example, the input unit 180 inputs an instruction from the manipulator related to selection of a manipulation application with respect to the manipulation target 35. In addition, the input unit 180 inputs operation amounts of the nozzle actuator 40 and/or the sample actuator 41 from the manipulator to the information processing device 170. For example, the input unit 180 is a keyboard or a mouse connected to the organism manipulation device 100.

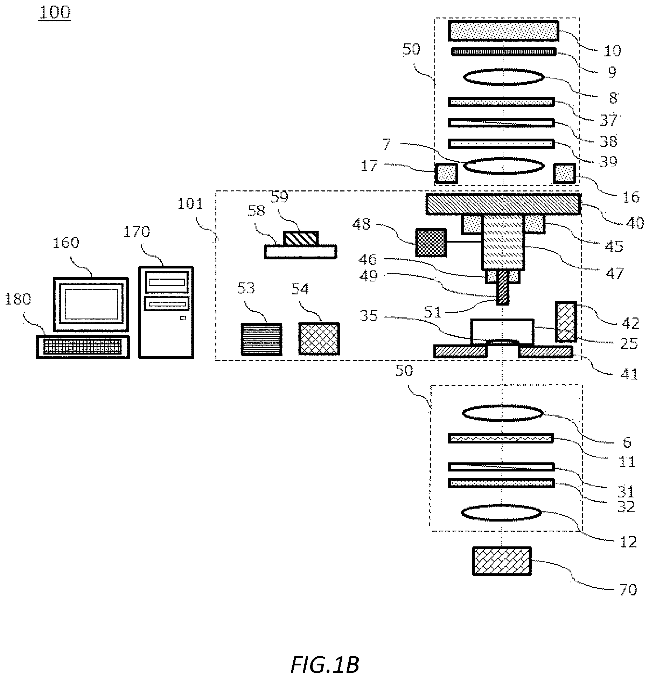

FIG. 1B illustrates another example of the device configuration of the organism manipulation device 100 in the present embodiment. FIG. 1B illustrates the organism manipulation device 100 when the microscope unit 50 is a phase difference microscope or a differential interference microscope. When the microscope unit 50 is the phase difference microscope, the microscope unit 50 may include the objective lens 6, the condenser lens 7, the condensing lens 8, the band pass filter 9, the transmission image observation light source 10, the barrier filter 11, the projection lens 12, the light source 16, the light source 17, and a ring diaphragm 39. When the microscope unit 50 is the differential interference microscope, the microscope unit 50 may include the objective lens 6, the condenser lens 7, the condensing lens 8, the band pass filter 9, the transmission image observation light source 10, the barrier filter 11, the projection lens 12, the light source 16, the light source 17, a Nomarski prism 31, an analyzer (polarizing plate) 32, a polarizer (polarizing plate) 37, and a Nomarski prism 38. In addition, not limited to these, the microscope unit 50 may include configurations other than those described above. Regarding the configurations of the organism manipulation device 100 other than the microscope unit 50, the descriptions in FIG. 1A may be applied.

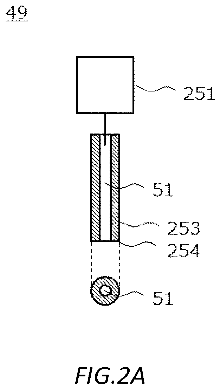

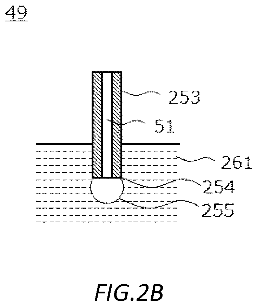

FIG. 2A and FIG. 2B are examples of a schematic view showing the structure of the nozzle 49 in the present embodiment. In FIG. 2A, the nozzle 49 includes a tubular portion 253 having the flow channel 51. The tubular portion 253 may have a hollow cylindrical shape. In this case, the shape of a cross section of the tubular portion 253 that is orthogonal to an axial direction is a circle. In addition, the flow channel 51 may be connected to a pump 251 (for example, the syringe pump of the pressure generating unit 47) on one end. Upon receiving an instruction from the information processing device 170 (for example, the air bubble forming unit 200 to be described later), the pump 251 regulates a pressure and/or a volume of an air bubble by regulating an amount of the gas to be supplied to the flow channel 51 or taken in from the flow channel 51.

In FIG. 2B, when an end part 254 that is on the side not connected to the pump (not illustrated: for example, the syringe pump of the pressure generating unit 47) of the tubular portion 253 is arranged in liquid 261, the pump can form an air bubble on the end part 254 by supplying gas to the flow channel 51. In this case, the gas-liquid interface 255 is formed in a boundary between the gas of the air bubble and the liquid 261. Note that, the shape of the air bubble is not limited to a spherical shape, and it may be deformed according to the shape of the end part 254. When gas is retained on the end part 254 of the flow channel 51, the gas-liquid interface 255 is formed on the end part 254 of the flow channel 51, but when both gas and liquid exist inside the flow channel 51, the gas-liquid interface 255 is formed on an interface of the gas and the liquid inside the flow channel 51.

When the gas-liquid interface 255 contacts an organism adhered on a solid phase such as an inner bottom surface of the vessel 25 in the liquid 261, by moving the gas-liquid interface 255, the gas-liquid interface 225 can apply force to the organism, thereby separating the organism from the solid phase and attaching the organism to the gas-liquid interface 255. A movement of the gas-liquid interface 255 may be performed by moving the nozzle 49 in which the air bubble is formed, with the nozzle actuator 40, or may be performed by moving the liquid, or may be performed by changing a volume of the air bubble. The solid phase may be a surface on which an adherent cell can be adhered and cultured. For example, the solid phase may be a glass; a resin such as polystyrene; a metal; a surface that is coated with one or more types of extracellular matrix components selected from collagen, fibronectin, laminin, polylysine, and the like; a surface that is coated with various types of polymers (as an example, a polymer having hydrophilicity and adhesiveness to a cell that are controllable); and the like, but the present invention is not limited thereto. Note that, in the present embodiment, the gas-liquid interface 255 is formed by an interface between gas and liquid, but the present invention is not limited thereto, and it may be changed according to a phase or a substance in contact with the interface. A method of separating an organism from a solid phase will be described below in detail.

An opening area of the flow channel 51 in the end part 254 is not particularly limited as long as it has a size that allows manipulation of an organism. For example, the opening area may be greater than an adhering area per one organism. The shape of the end part 254 is not particularly limited. In addition, an inner diameter of the flow channel 51 may be the same throughout an entire length of the tubular portion 253.

Furthermore, the flow channel 51 may be such channel where the pump 251 takes in gas in an air bubble to which an organism is attached, thereby taking the gas-liquid interface 225 in the flow channel 51 and further recovering the organism. Alternatively, besides the flow channel 51, the nozzle 49 may further include another flow channel for recovering the organism.

In the embodiments of FIG. 2A and FIG. 2B, since only one flow channel 51 is formed in the nozzle 49, and the pump 251 connected to the flow channel 51 is also only one, it is a minimal configuration that is very simple, and it is possible to reduce the maintenance and the cost of the organism manipulation device 100.

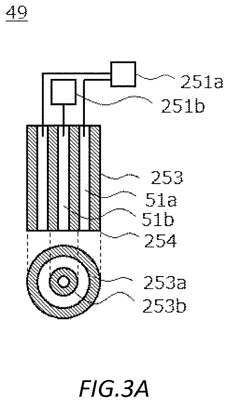

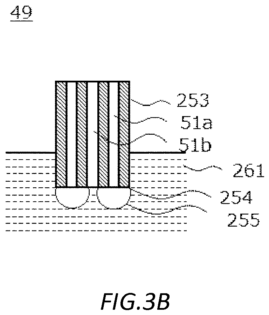

FIG. 3A and FIG. 3B are examples of the schematic view showing the structure of the nozzle 49, in other embodiments. The examples of FIG. 2A and FIG. 2B illustrated the case where the flow channel forming an air bubble and the flow channel recovering an organism are the same, but the examples of FIG. 3A and FIG. 3B illustrate a case that the flow channel forming an air bubble and the flow channel recovering an organism are different.

In FIG. 3A, the tubular portion 253 of the nozzle 49 has a double structure of an outer tube 253a and an inner tube 253b. A first flow channel 51a where gas flows, is between the outer tube 253a and the inner tube 253b, and the inside of the inner tube 253b is a second flow channel 51b. For example, the first flow channel 51a may be a gas supply flow channel, and the second flow channel 51b may be a gas recovery flow channel.

In addition, the first flow channel 51a and the second flow channel 51b of the nozzle 49 may be connected to a first pump 251a and a second pump 251b, respectively, on one ends. For example, the pressure generating unit 47 may have the first pump 251a and the second pump 251b as syringe pumps, and may individually control each of them with separate actuators. Regarding each of the first pump 251a and the second pump 251b, an actuator that has received an instruction from the air bubble forming unit 200 regulates a pressure and/or a volume of an air bubble by regulating an amount of the gas to be supplied or taken in from the first flow channel 51a and the second flow channel 51b. The shape of a cross section of the tubular portion 253 orthogonal to the axial direction is a doughnut shape in the first flow channel 51a and is a circle in the second flow channel 51b.

In FIG. 3B, when the end part 254 that is on the side not connected to the first pump 251a or the second pump 251b of the tubular portion 253 is arranged in the liquid 261, the first pump 251a can form an air bubble on the end part 254 by supplying gas to the first flow channel 51a. In this case, the gas-liquid interface 255 is formed in a boundary between the gas of the air bubble and the liquid 261.

When the gas-liquid interface 255 contacts an organism adhered on a solid phase in the liquid 261, by moving the gas-liquid interface 255, the organism can be separated from the solid phase and the organism can be attached to the gas-liquid interface 255. The second pump 251b may, by taking in the air bubble to which the organism is attached via the second flow channel 51b, take the gas-liquid interface 225 in the flow channel 51 and recover the organism.

Note that, in the above-described embodiment, the first pump 251a supplies gas to the first flow channel 51a to form an air bubble, and the second pump 251b recovers an organism by taking in the gas with the second flow channel 51b, but the second pump 251b may supply gas to the second flow channel 51b to form an air bubble, and the first pump 251a may recover an organism by taking in the gas with the first flow channel 51a. In addition, the first pump 251a and the second pump 251b may be the same syringe pump provided for the pressure generating unit 47. Either of the first pump 251a and the second pump 251b may be omitted.

In the embodiments of FIG. 3A and FIG. 3B, since one flow channel forms an air bubble to attach an organism to the gas-liquid interface 255, and the other flow channel can recover the attached organism at the same time, there is an effect that a time for recovering the cell is shortened. Note that, FIG. 3A and FIG. 3B illustrate the embodiments in which the shape of a cross section of the tubular portion 253 orthogonal to the axial direction is a doughnut shape in the first flow channel 51a and is a circle in the second flow channel 51b, but the shape of the cross section is not limited to the doughnut shape or the circle, and attachment and recovery of the organism can be performed at the same time as long as there are two flow channels.

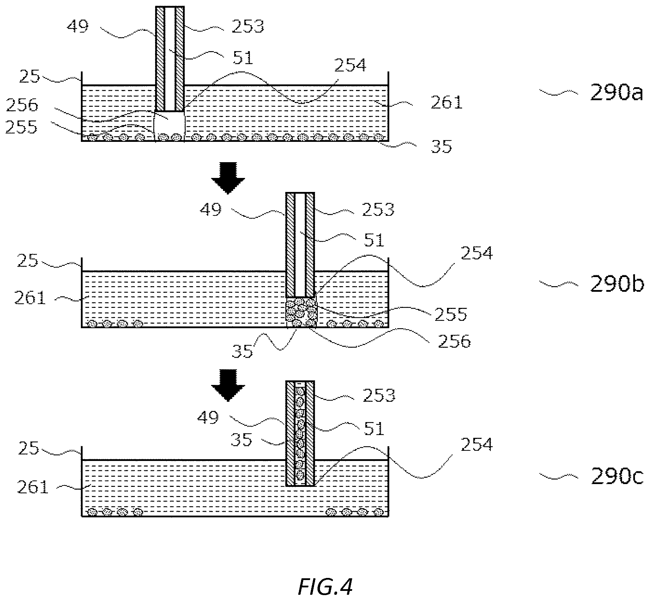

FIG. 4 is a schematic view showing an example of a method of recovering the manipulation target 35 in the present embodiment. In 290a, the manipulation target 35 is cultured at a solid phase on an inner bottom surface of the vessel 25. The manipulation target 35 may be cultured in the liquid 261. For example, the manipulation target 35 is an adherent cell. For example, the liquid may be a complete medium.

In 290a, the pump 251 supplies gas to the flow channel 51 of the nozzle 49, and forms the air bubble 256 on the end part 254 of the nozzle 49. By bringing the air bubble 256 into contact with the manipulation target 35, the gas-liquid interface 255 between the gas and the liquid 261 contacts the manipulation target 35. In this case, the pump 251 adjusts supply and intake of the gas, and maintains the air bubble 256 that is formed. In this manner, manipulations of the manipulation target 35 with the air bubble 256 can be facilitated.

Then, in 290b, the nozzle actuator 40 moves the nozzle 49 along a surface of the solid phase while keeping the air bubble 256 in contact with the manipulation target 35. FIG. 4 illustrates a behavior that the nozzle actuator 40 moves the nozzle 49 in a left-to-right direction, but the direction of moving the nozzle 49 is not limited as long as it is a direction parallel to the surface of the solid phase. With the nozzle actuator 40 moving the nozzle 49, the manipulation target 35 can be separated from the solid phase. At this time, the separated manipulation target 35 attaches to the gas-liquid interface 255 of the air bubble 256. With the air bubble forming unit 200 controlling the pump 251 to change a size of the air bubble 256 by regulating a pressure and/or a volume of gas to be supplied or taken in, the manipulation target 35 within a desired range can be separated. Note that, instead of moving the nozzle 49 along the surface of the solid phase, the stage may be moved.

Then, in 290c, with the pump 251 taking in the gas in the flow channel 51, the manipulation target 35 attached to the gas-liquid interface 255 may be recovered. In this manner, by using the air bubble 256 formed in the nozzle 49, the manipulation target 35 can be selectively separated from the solid phase and recovered.

Note that, if the manipulation target 35 is an adherent cell that is strongly adhered on the solid phase, adhesion of the adherent cell may be loosened in advance before performing the method in FIG. 4. The loosening of adhesion of the adherent cell can be performed by using a well-known method, as will be described below.

FIG. 4 described the example of moving the gas-liquid interface 255 by moving the nozzle 49, thereby separating and recovering the cell, but the movement of the gas-liquid interface 255 is not limited to the above-described example. For example, a volume of the air bubble 256 may be increased after bringing the air bubble 256 into contact with the manipulation target 35. In this case, a contact surface between the air bubble 256 and the solid phase will be broadened, and the manipulation target can be selectively separated from the solid phase and recovered. For example, after bringing the air bubble 256 into contact with the manipulation target 35, the nozzle actuator 40 may move the nozzle 49 closer to the solid phase. Also in this case, the contact surface between the air bubble 256 and the solid phase is broadened with the air bubble 256 being pressed against the solid phase, and the manipulation target can be selectively separated from the solid phase and recovered.

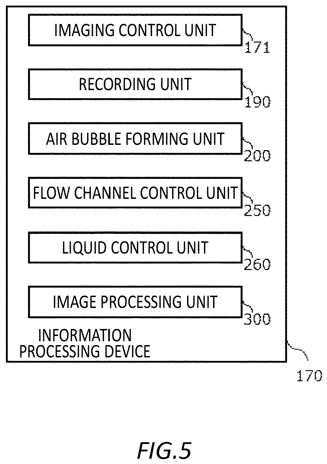

FIG. 5 illustrates an example of a specific configuration of the information processing device 170 in the present embodiment. The information processing device 170 has the imaging control unit 171, the recording unit 190, the air bubble forming unit 200, a flow channel control unit 250, a liquid control unit 260, and the image processing unit 300.

The imaging control unit 171 performs controls on the fluorescence image observation light source 1, the objective lens 6, the fluorescent filter, the transmission image observation light source 10, the flow channel imaging camera 42, the light source 45, the light source 46, the camera 60, the camera 70, and the like described in FIG. 1A and FIG. 1B. For example, once an imaging condition of the manipulation target 35 is input to the input unit 180, the imaging control unit 171 performs necessary adjustments per imaging according to the input imaging condition, from among switching of the camera, switching of the type of the objective lens 6 in the microscope unit 50, switching of the light source, switching of the type of the fluorescent filter, the position of the stage, and the height of the objective lens 6. After the imaging control unit 171 has performed the necessary adjustments, one or a plurality of cameras among the flow channel imaging camera 42, the camera 60, and the camera 70 perform imaging of the manipulation target 35 or the nozzle 49, and generate an image of the manipulation target 35 or the nozzle 49. The one or the plurality of cameras send generated image data to the image processing unit 300. In addition, the generated image data may also be recorded in the recording unit 190 and/or output to the output unit 160.

The recording unit 190 may be a memory, a built-in hard disk drive, or an external recording medium, but the present invention is not limited thereto. The information processing device 170 has a central processing unit (CPU), and the CPU achieves the information processing device 170 and the like by executing a computer program recorded in the recording unit 190.

The air bubble forming unit 200 controls a pressure and a volume of an air bubble formed in the flow channel 51, supply and intake of gas, a movement of the nozzle 49, and a movement of the stage. The air bubble forming unit 200 may include all or some of the nozzle position control unit, the stage position control unit, the volume control unit, a gas supply control unit, and an intake control unit.

The nozzle position control unit controls the nozzle actuator 40, and controls an operation of the nozzle 49, an airflow in an air bubble involved in the operation of the nozzle 49, and a movement of the gas-liquid interface 255 involved in the operation of the nozzle 49. In addition, the nozzle position control unit receives position information on the nozzle 49 from the sensor unit 48 or the nozzle actuator 40. Note that, an airflow may be a flow or a dynamic state of a fluid in an air bubble.

The stage position control unit controls the sample actuator 41, and controls an operation of the stage equipped with the vessel 25 containing the manipulation target 35, an airflow in an air bubble involved in the operation of the stage, and a movement of the gas-liquid interface 255 involved in the operation of the stage. In addition, the stage position control unit receives position information on the stage and the manipulation target 35 from the sensor unit 48 or the sample actuator 41.

The volume control unit controls the actuator of the pressure generating unit 47, and controls a pressure and/or a volume of an air bubble formed in the flow channel 51 by supplying gas from the syringe pump or taking the gas in the syringe pump. In addition, the volume control unit receives information on the pressure and/or the volume of the air bubble from the nozzle actuator 40, the pressure generating unit 47, or the sensor unit 48.

In addition, when the nozzle 49 includes the gas supply flow channel for supplying gas (gas supply) and the gas recovery flow channel for recovering the gas (gas intake), the volume control unit or the gas supply control unit controls the first pump 251a connected to the gas supply flow channel, thereby controlling a volume of the gas to be supplied to the gas supply flow channel. The volume control unit or the gas supply control unit receives information on an amount of the gas to be supplied to the gas supply flow channel from the nozzle actuator 40, the pressure generating unit 47, or the sensor unit 48.

In addition, when the nozzle 49 includes the gas supply flow channel for supplying gas (gas supply) and the gas recovery flow channel for recovering the gas (gas intake), the volume control unit or the intake control unit controls the second pump 251b connected to the gas recovery flow channel, thereby controlling an amount (volume) of the gas to be taken in from the gas recovery flow channel. The volume control unit or the intake control unit receives information on an amount of the gas to be taken in from the gas recovery flow channel, from the nozzle actuator 40, the pressure generating unit 47, or the sensor unit 48.

The flow channel control unit 250 controls keeping, mounting, and discarding of the nozzle 49. The flow channel control unit 250 receives, from the input unit 180, instructions from the manipulator on manipulations related to mounting and discarding of the nozzle 49. According to the received instructions, the flow channel control unit 250 sends an instruction to the flow channel exchange unit 53 to take out the nozzle 49 from a flow channel keeping part of the flow channel exchange unit 53 and mount the nozzle 49 on the nozzle actuator 40, or remove the nozzle 49 mounted on the nozzle actuator 40 and discard it in a flow channel discarding part of the flow channel exchange unit 53.

The liquid control unit 260 controls keeping, replenishment, and discarding of liquid. The liquid control unit 260 receives, from the input unit 180, instructions from the manipulator on manipulations related to replenishment and discarding of liquid. According to the received instructions, the liquid control unit 260 sends an instruction to the liquid storage part 54 to replenish liquid kept in the liquid keeping part of the liquid storage part 54 to the vessel 25, or recover the liquid contained in the vessel 25 from the vessel 25 and discard it in the liquid discarding part of the liquid storage part 54.

The image processing unit 300 receives images captured with the flow channel imaging camera 42, the camera 60, and the camera 70, from these cameras. The image processing unit 300 may use a plurality of images among the received images to compose these into one composite image. For example, the image processing unit 300 may compose a fluorescence image that is captured with the camera 60 and a transmission image that is captured with the camera 70 to generate a composite image. The image processing unit 300 may record the image and/or the composite image received from these cameras in the recording unit 190, and/or output such image and/or composite image to the output unit 160.

An energy control unit controls a difference (E1-E2) between a surface free energy E1 on an interface between gas and an organism, and a surface free energy E2 on an interface between the gas and liquid. A value of the difference (E1-E2) may be a positive value, 0, or a negative value. The smaller the value of the difference (E1-E2) is, the larger number of organisms attach to an air bubble. Since the value of the surface free energy E1 is constant, there is a little room for control by the energy control unit. Therefore, with the energy control unit controlling the surface free energy E2 on the interface between the gas and the liquid, the value of this difference (E1-E2) can be controlled to a preset value. The details will be described below.

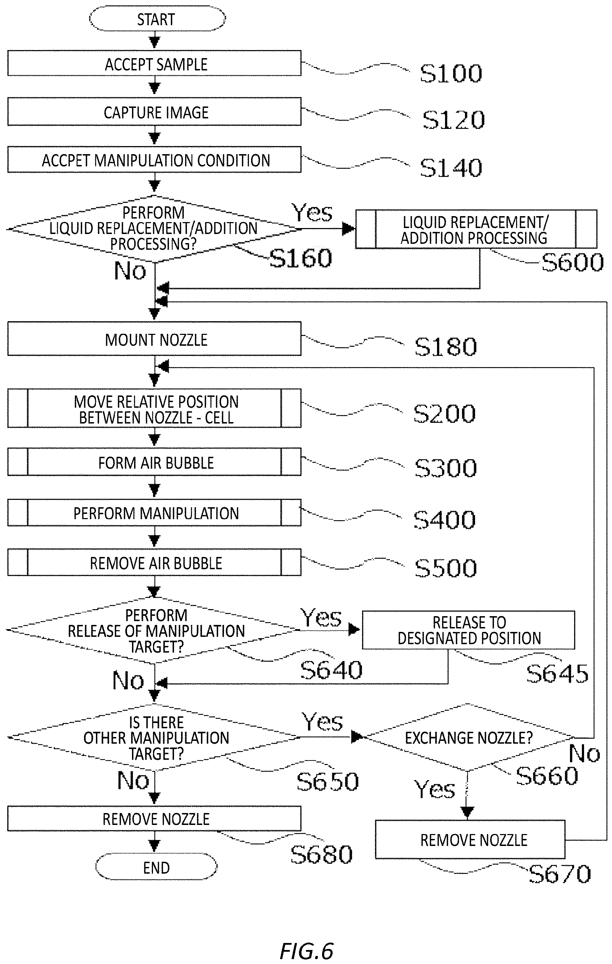

FIG. 6 is an example of a flow of a method of manipulating an organism in the present embodiment. An organism to be the manipulation target 35 in the present embodiment can be manipulated by performing the processing of S100 to S680 in FIG. 6. Note that, for convenience of description, the processing of S100 to the processing of S680 will be described in order; however, at least some processing may be executed in parallel, and each step may be interchanged and executed within a range not deviating from the spirit of the present invention.

First, in S100, the sample actuator 41 accepts an organism to be the manipulation target 35. For example, in S100, the sample actuator 41 equips a stage with the vessel 25 that contains the manipulation target 35 together with liquid. A lid of the vessel 25 may be removed to perform manipulations on the manipulation target 35. The lid may be exchanged by an actuator for exchanging the lid, or may be exchanged by the hands of the manipulator. After the sample actuator 41 has accepted the organism to be the manipulation target 35, the information processing device 170 proceeds the processing to S120.

Then, in S120, the camera 60 or the camera 70 images a wide range of an observation field including the manipulation target 35, and generates an image. The imaging control unit 171 sets the observation method to capturing of a low magnification transmission image, and sends an instruction to the camera 70 to image the observation field. The imaging control unit 171 may set the observation method to capturing of a fluorescence image, and send an instruction to the camera 60 to image the observation field. The imaging control unit 171 may receive an input of an imaging condition from the manipulator, via the input unit 180. The camera 60 or the camera 70 images the observation field. The image processing unit 300 may record the captured image in the recording unit 190 and/or output the captured image to the output unit 160. After the camera 60 or the camera 70 has imaged the observation field, the imaging control unit 171 proceeds the processing to S140.

Then, in S140, the information processing device 170 receives, via the input unit 180, an input from the manipulator related to the manipulation target 35 and the type of a manipulation. The manipulation target 35 may be a single cell, a population (colony) of cells, a cytoplasm and/or a cell membrane of a cell, or a spheroid, but the present invention is not limited thereto. The type of a manipulation may be recovery of the manipulation target 35, removal of the manipulation target 35, retention of the manipulation target 35, or compression of the manipulation target 35, but the present invention is not limited thereto.

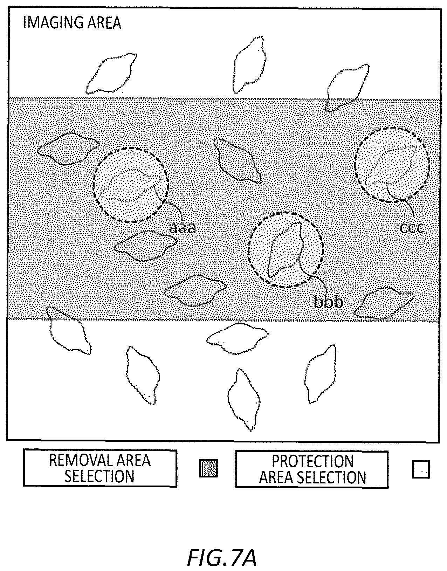

FIG. 7A is an example of a Graphical User Interface (GUI) image that is displayed on the output unit 160, obtained by imaging an observation field with the camera 60 or the camera 70. In FIG. 7A, cells aaa, bbb, and ccc to be manipulation targets are designated as the manipulation target 35, via the input unit 180. As illustrated in FIG. 7A, an organism to be the manipulation target 35 is optionally designated via the input unit 180. As illustrated in FIG. 7A, as in the case of the GUI image where a removal area and/or a protection area is selected, a removal area and/or a protection area may also be provided for the observation field. By providing the removal area, a risk of recovering cells other than a cell to be recovered can be reduced. In addition, by providing the protection area, a risk of mistakenly removing a recovery cell at the time of removing a cell in the removal area can be reduced.

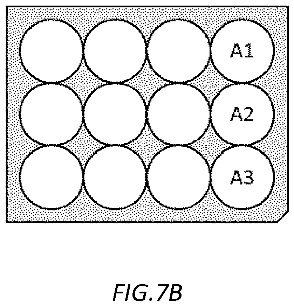

FIG. 7B is an example of a GUI image that is displayed on the output unit 160, in which recovering destinations and moving destinations of the cells aaa, bbb, and ccc to be the manipulation target 35 are designated as A1, A2, and A3 of a 12-hole plate, respectively. As illustrated in FIG. 7B, the moving destinations are optionally designated via the input unit 180. For example, the moving destinations may be the same plate, different plates, petri dishes, micro test tubes, PCR tubes, or conical tubes.

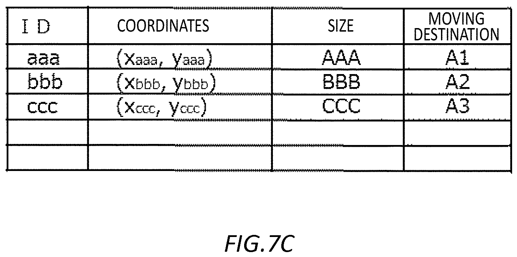

FIG. 7C is an example of a GUI image displayed on the output unit 160, the GUI image displaying a table that lists an ID number of a cell to be the manipulation target 35, xy coordinates of the manipulation target 35 on the sample actuator 41, a size of the manipulation target 35, and a moving destination of the manipulation target 35. The table illustrated in FIG. 7C uses ID numbers, xy coordinates, sizes, and moving destinations as the items, but the displayed items are not limited thereto. In this manner, with the manipulation target 35, the moving destination, and the like being designated via the input unit 180, the image processing unit 300 may output a table to the output unit 160.

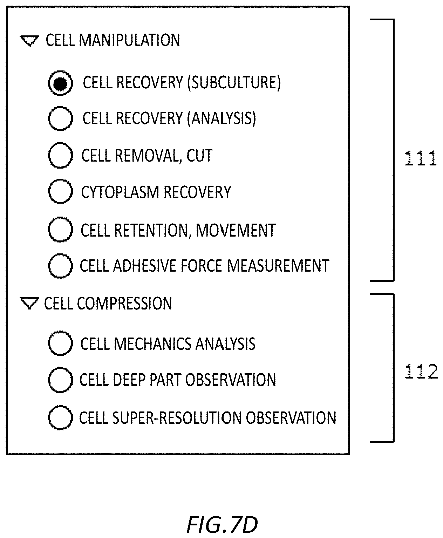

FIG. 7D is an example of a GUI screen that is displayed on the output unit 160, for selecting the type of a manipulation on the manipulation target 35. The input unit 180 receives, from the manipulator, an instruction as to the kind of manipulation desired to be performed on the manipulation target 35, and inputs the instruction to the information processing device 170. For example, as shown in a display area 111, the manipulation on a cell may be subculture, or retention or movement of the cell, but the present invention is not limited thereto. For example, as shown in a display area 112, the manipulation on a cell may be compression of the cell for observing a deep part of the cell, but the present invention is not limited thereto. FIG. 7D illustrates an example of selecting the type of the manipulation with a radio button, but the method of selection is not limited to the radio button.

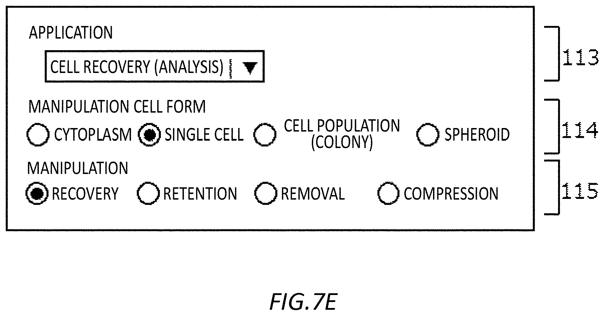

FIG. 7E is another example of the GUI screen displayed on the output unit 160, for selecting the type of a manipulation on the manipulation target 35. For example, as shown in a display area 113, the type of the manipulation on a cell may be selected with a pull-down menu. For example, as shown in the display area 113, a display area 114, and a display area 115, the pull-down menu and the radio button may be used in combination for selection of the type of the manipulation.

The input unit 180 may send an instruction input from the manipulator to the information processing device 170, based on the screens illustrated in FIG. 7A to FIG. 7E. After the information processing device 170 has received the instruction, the imaging control unit 171 proceeds the processing to S160.

Note that, in S160, if the manipulation target 35 is an adherent cell that is strongly adhered on a solid phase, a step of loosening adhesion of the adherent cell in advance may be additionally performed. In this case, in the step of accepting manipulation conditions in S140, the information processing device 170 may receive an input as to whether to perform the processing of loosening the adhesion.

The loosening of the adhesion of the adherent cell can be performed by using a well-known method. For example, the loosening of the adhesion of the adherent cell may be performed by removing liquid (for example, a medium), and treating the adherent cell with an adhesion loosening solution after washing with a buffer solution. For example, the adhesion loosening solution may be a proteolytic enzyme solution, a solution that does not include a metal ion, or a chelating agent solution. As an example, the adhesion loosening solution is a trypsin-EDTA solution. The loosening of the adhesion of the adherent cell may be performed by the liquid control unit 260, or may be performed with the hands of the manipulator. After the adherent cell is treated with the adhesion loosening solution and adhesive force is weakened, the processing may proceed to S160. Note that, not limited to the loosening of the adhesion of the adherent cell, when a step is performed with the hands of the manipulator, the processing may be started again from the step of accepting a sample in S100. In addition, instead of the treatment with the adhesion loosening solution, the adhesive force may be weakened by using a substrate for loosening adhesion. For example, the substrate for loosening adhesion may be such substrate that loosens adhesion by reacting to a temperature or an irradiation of a light.

Then, in S160, the information processing device 170 receives, via the input unit 180, an input from the manipulator related to a liquid replacement or addition treatment. For example, when attachment force between an organism to be the manipulation target 35 and an air bubble is desired to be adjusted, the information processing device 170 may receive an input to perform a liquid replacement or addition treatment. When the information processing device 170 has received the instruction to perform the liquid replacement or addition treatment, the information processing device 170 may proceed the processing to S600. When the information processing device 170 has received an instruction not to perform the liquid replacement or addition treatment, the information processing device 170 may proceed the processing to S180.

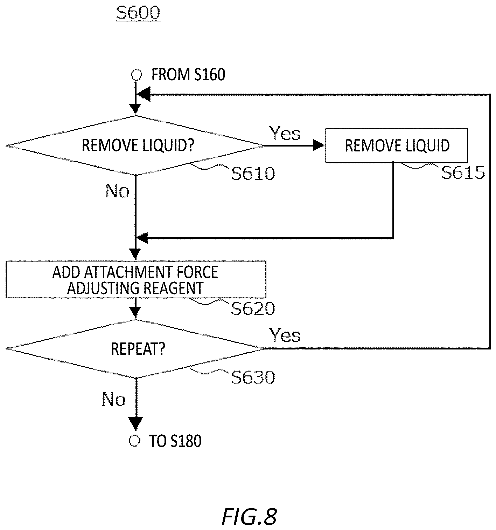

In S600, the liquid control unit 260 replaces liquid of the vessel 25 that contains the manipulation target 35, or adds another liquid to the liquid of the vessel 25. In S600, the step of performing the liquid replacement or addition treatment includes the steps from S610 to S630, as illustrated in FIG. 8.

First, in S610, the information processing device 170 receives, via the input unit 180, an input from the manipulator related to whether to remove liquid. When the information processing device 170 has received an instruction to remove the liquid, the processing proceeds to S615. When the information processing device 170 has received an instruction not to remove the liquid, the processing proceeds to S620.

In S615, the liquid control unit 260 removes the liquid by controlling the liquid storage part 54. For example, the liquid control unit 260 sends an instruction to the liquid storage part 54 to recover the liquid contained in the vessel 25 from the vessel 25 by a preset amount, and discard it in the liquid discarding part of the liquid storage part 54. At this time, the liquid storage part 54 may recover and discard the full amount of the liquid. Alternatively, the liquid storage part 54 may recover and discard a part (for example, the half amount) of the liquid. After the liquid storage part 54 has recovered the liquid, the liquid control unit 260 proceeds the processing to S620.

In S620, the liquid control unit 260 sends an instruction to the liquid storage part 54 to add an attachment force adjusting reagent to the vessel 25. The attachment force adjusting reagent is a reagent for adjusting attachment force between an organism and an air bubble. For example, the attachment force adjusting reagent may be such reagent that changes concentrations of inorganic salts and/or amphipathic substances in liquid. As an example, the attachment force adjusting reagent may be a buffer solution that includes or does not include at least either of calcium ion or magnesium ion, a basal medium, a complete medium, or a chelating agent. At this time, the liquid storage part 54 replenishes the attachment force adjusting reagent kept in the liquid keeping part of the liquid storage part 54 to the vessel 25.

Note that, the attachment force adjusting reagent is replenished to the vessel 25 in the above-described example, but instead of adding the attachment force adjusting reagent to the vessel 25, the attachment force between the organism and the air bubble may be adjusted by attaching inorganic salts, amphipathic substances, or the like included in the liquid to a filter or the like for removal.

In S630, the information processing device 170 receives, via the input unit 180, an instruction from the manipulator related to whether to repeat the above-described series of manipulations. When the information processing device 170 has received the instruction to repeat the series of manipulations, the information processing device 170 proceeds the processing to S610, and the liquid control unit 260 sends an instruction to the liquid storage part 54 to remove the liquid of the vessel 25. When the information processing device 170 has received an instruction not to repeat the series of manipulations, the information processing device 170 proceeds the processing to S180. Note that, the step of S600 and the sub-step may be performed with the hands of the manipulator, and in this case, the processing may be started again from the step of accepting a sample in S100.

In S180, the nozzle 49 is mounted on the nozzle actuator 40. For example, the information processing device 170 receives, via the input unit 180, an instruction from the manipulator to mount the nozzle 49 on the nozzle actuator 40. According to the instruction, the flow channel control unit 250 sends an instruction to the flow channel exchange unit 53 to take out the nozzle 49 from the flow channel keeping part of the flow channel exchange unit 53 and mount the nozzle 49 on the nozzle actuator 40. At this time, the nozzle 49 that is suitable may be selected according to a size of the manipulation target 35, the type of a manipulation, and the like. A selection of the nozzle 49 may be designated from the manipulator via the input unit 180, or may be automatically designated by the flow channel control unit 250. After the nozzle 49 is mounted on the nozzle actuator 40, the flow channel control unit 250 proceeds the processing to S200. Note that, when there is no need to mount the nozzle 49 on the nozzle actuator 40 such as a state where the nozzle 49 is mounted on the nozzle actuator 40 in advance, or a state where the nozzle actuator 40 and the nozzle 49 are integrally formed, the step of S180 may be omitted.

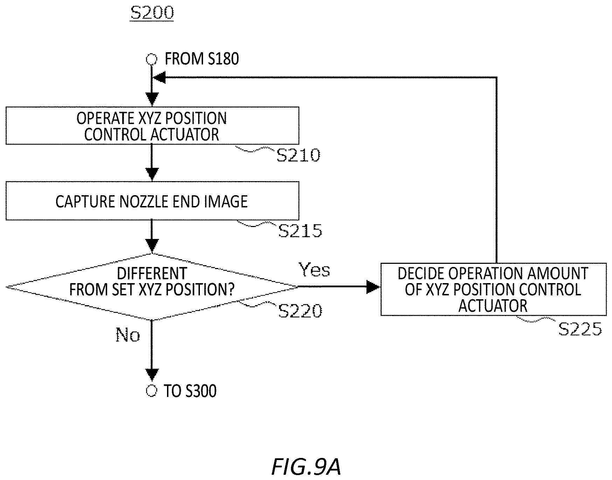

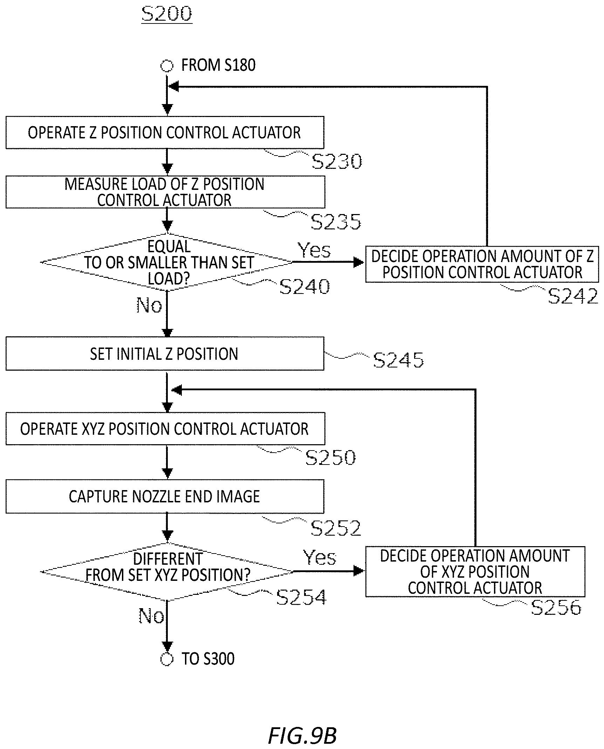

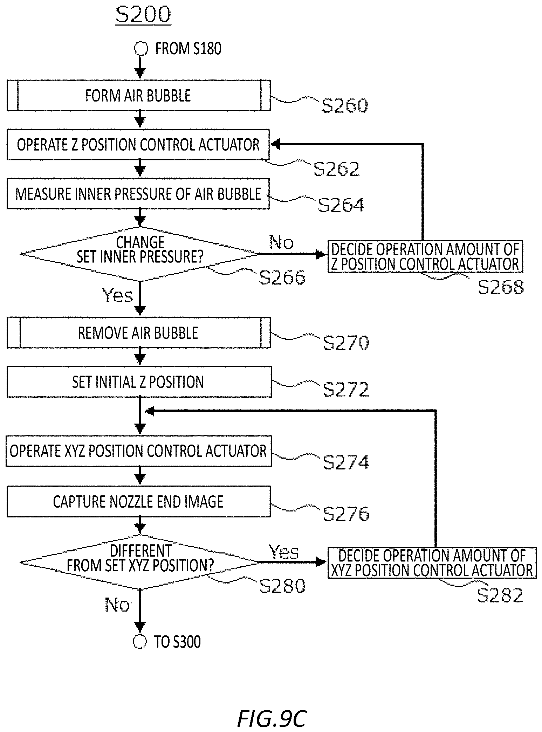

Then, in S200, the nozzle actuator 40 moves a relative position between the nozzle 49 and the manipulation target 35. For example, the air bubble forming unit 200 sends an instruction to the nozzle actuator 40 to move the relative position between the nozzle 49 and the manipulation target 35. In S200, the step of moving the relative position includes the steps from S210 to S225 as illustrated in FIG. 9A, or the steps from S230 to S256 as illustrated in FIG. 9B, or the steps from S260 to S282 as illustrated in FIG. 9C.

FIG. 9A is an example of a flow of moving the relative position between the nozzle 49 and the manipulation target 35, based on an image that is obtained by imaging a position of the end part 254 of the nozzle 49.

First, in S210, the air bubble forming unit 200 sends an instruction to the nozzle actuator 40 to cause an operation of the nozzle 49 to a preset position. The nozzle actuator 40 may be an actuator for controlling an x, y, and z position. A z position may be a position in the vertical direction (also referred to as the direction along gravity, the upward and downward directions, and the z direction), an x position may be any position in the x direction (also referred to as the longitudinal direction) that is perpendicular to the z direction, and a y position may be a position in the y direction (also referred to as the lateral direction) that is perpendicular to the x direction and the z direction.

The position of the nozzle 49 may be set to a z position by, first focusing the camera 60 and/or the camera 70 on a bottom surface of the vessel 25, subsequently moving a focus of the camera 60 and/or the camera 70 upward by an optional distance, and then matching an edge of the nozzle 49 with the focus of the camera 60 and/or the camera 70 by using the nozzle actuator 40. For example, the optional distance may be equal to or smaller than a radius of an air bubble formed on the end part 254 of the nozzle 49. If the distance between the edge of the nozzle 49 and the bottom surface of the vessel 25 is equal to or smaller than the radius of the formed air bubble, since the air bubble contacts the bottom surface, an organism that is positioned on the bottom surface can be manipulated by using an interface of the air bubble. In this case, an xy position of the nozzle 49 can be set by using the nozzle actuator 40 or the sample actuator 41, based on an image that is obtained by imaging the manipulation target 35 with the camera 60 and/or the camera 70. Note that, instead of the camera 60 and/or the camera 70, or in addition to the camera 60 and/or the camera 70, the position of the nozzle 49 may be set by using the flow channel imaging camera 42.

In addition, regarding adjustment of the z position of the nozzle 49, the z position may be set by imaging the edge of the nozzle 49 and the bottom surface of the vessel 25 from the lateral direction of the nozzle 49, with the flow channel imaging camera 42. Furthermore, the shape of the air bubble, the liquid quantity of the flow channel 51, or the like may be confirmed by performing imaging from the lateral direction of the nozzle 49 with the flow channel imaging camera 42. After the nozzle actuator 40 has moved the nozzle 49 to a preset position, the air bubble forming unit 200 proceeds the processing to S215.

Then, in S215, the flow channel imaging camera 42 captures an image of the end part 254 of the nozzle 49. The flow channel imaging camera 42 sends the captured image to the image processing unit 300. The image processing unit 300 may record the image in the recording unit 190 and/or output the image to the air bubble forming unit 200.

Then, in S220, the air bubble forming unit 200 determines, based on the image of the end part 254 of the nozzle 49 that is captured, whether the position of the nozzle 49 is different from the preset position. For example, the air bubble forming unit 200 calculates a difference between the positions from the image of the end part 254 of the nozzle 49 that is captured, and an image of the end part 254 of the nozzle 49 in a set x, y, and z position that is preset (that is, an initial position), and if the difference is equal to or greater than a threshold, determines that the position of the nozzle 49 is different from the initial position.

If the position of the nozzle 49 is determined to be different from the initial position, the air bubble forming unit 200 proceeds the processing to S225, and if not, proceeds the processing to S300.

In S225, the air bubble forming unit 200 decides an operation amount of the nozzle actuator 40. For example, the air bubble forming unit 200 decides an operation amount of the nozzle actuator 40, and sends an instruction to the nozzle actuator 40 to operate by the decided operation amount, for causing an operation of the nozzle 49 to the preset x, y, and z position (that is, the initial position). For example, the air bubble forming unit 200 may decide an operation amount of an amount according to the level of the difference calculated in S220. The nozzle actuator 40 receives the instruction, and the processing proceeds to S210. In S210 of the second and subsequent times, the air bubble forming unit 200 sends an instruction to the nozzle actuator 40 to operate by the amount according to the operation amount.

FIG. 9B is an example of a flow of moving the relative position between the nozzle 49 and the manipulation target 35, based on a load sensed with the nozzle actuator 40.

First, in S230, the air bubble forming unit 200 sends an instruction to the nozzle actuator 40 to cause an operation of the nozzle 49 to a preset position. The nozzle actuator 40 may be an actuator for controlling a z position. In this case, the z position is controlled based on a value of a load, contact or proximity information sensed with the nozzle actuator 40. The nozzle 49 may be positioned above a region where an organism does not exist on the bottom surface of the vessel 25. After the nozzle actuator 40 has moved the nozzle 49 to the preset position, the air bubble forming unit 200 proceeds the processing to S235.

Then, in S235, the sensor unit 48 measures an applied load, contact or proximity information of the nozzle actuator 40, and sends measurement values to the air bubble forming unit 200. As an example of load detection, once the nozzle 49 reaches a bottom portion of the vessel 25, a load sensed with the nozzle actuator 40 will be exponentially increased. Therefore, by measuring the value of the load sensed with the nozzle actuator 40, the air bubble forming unit 200 can determine whether the nozzle 49 has reached the bottom portion of the vessel 25. In addition, as another example of the load detection, the sensor unit 48 may sense a load while the nozzle actuator 40 is moving the nozzle 49 in the downward direction. Note that, instead of the sensor unit 48, the nozzle actuator 40 may send the value of the sensed load to the air bubble forming unit 200.

Then, in S240, the air bubble forming unit 200 determines whether the measured load value is equal to or smaller than a set load. If the measured load value is equal to or smaller than the set load, the air bubble forming unit 200 proceeds the processing to S242, and if not, proceeds the processing to S245. As described above, the air bubble forming unit 200 calculates a difference between the set load and the measured load, and if the value of the difference is equal to or greater than a threshold, determines that the nozzle 49 has not reached the bottom portion of the vessel 25.

In S242, the air bubble forming unit 200 decides an operation amount of the nozzle actuator 40. For example, the air bubble forming unit 200 decides an operation amount of the nozzle actuator 40, and sends an instruction to the nozzle actuator 40 to operate by the decided operation amount, for causing an operation of the nozzle 49 to the preset position. For example, the air bubble forming unit 200 may decide an operation amount of an amount according to the level of the difference calculated in S240. The nozzle actuator 40 receives the instruction, and the processing proceeds to S230. In S230 of the second and subsequent times, the air bubble forming unit 200 sends an instruction to the nozzle actuator 40 to operate by the amount according to the operation amount.

In S245, the air bubble forming unit 200 sets an initial z position of the nozzle 49. For example, the air bubble forming unit 200 may stop moving the z position of the nozzle 49 after the last S230, and set this position as the initial z position. Alternatively, the air bubble forming unit 200 may move the nozzle 49 in the z direction from the bottom surface of the vessel 25 by an optional distance that is preset, and set this position as the initial z position. In this manner, the initial z position of the nozzle 49 is positioned above the bottom surface by the optional distance. For example, the optional distance may be equal to or smaller than a radius of an air bubble formed on the end part 254 of the nozzle 49. After the air bubble forming unit 200 has set the initial z position of the nozzle 49, the air bubble forming unit 200 proceeds the processing to S250.

Then, in S250, the air bubble forming unit 200 sends an instruction to the nozzle actuator 40 to cause an operation of the nozzle 49 to a set x, y, and z position (that is, the initial position) that is preset. The nozzle 49 may move on an xy plane. In this case, the z position is controlled based on a value of a load sensed with the nozzle actuator 40. Furthermore, the movement of the nozzle 49 may include moving to the z direction as necessary, in addition to the movement on the xy plane. After the nozzle actuator 40 has moved the nozzle 49 to the set x, y, and z position, the air bubble forming unit 200 proceeds the processing to S252.

Then, in S252, the flow channel imaging camera 42 captures an image of the end part 254 of the nozzle 49. The flow channel imaging camera 42 sends the captured image to the image processing unit 300. The image processing unit 300 may record the image in the recording unit 190 and/or output the image to the air bubble forming unit 200.

Then, in S254, the air bubble forming unit 200 determines, based on the image of the end part 254 of the nozzle 49 that is captured, whether the position of the nozzle 49 is different from the preset position. For example, the air bubble forming unit 200 calculates a difference between the positions from the image of the end part 254 of the nozzle 49 that is captured, and an image of the end part 254 of the nozzle 49 in a set x, y, and z position that is preset (that is, the initial position), and if the difference is equal to or greater than a threshold, the air bubble forming unit 200 determines that the position of the nozzle 49 is different from the initial position.

If the position of the nozzle 49 is determined to be different from the initial position, the air bubble forming unit 200 proceeds the processing to S256, and if not, proceeds the processing to S300.

In S256, the air bubble forming unit 200 decides an operation amount of the nozzle actuator 40. For example, the air bubble forming unit 200 decides an operation amount of the nozzle actuator 40, and sends an instruction to the nozzle actuator 40 to operate by the decided operation amount, for causing an operation of the nozzle 49 to the set x, y, and z position that is preset (that is, the initial position). For example, the air bubble forming unit 200 may decide an operation amount of an amount according to the level of the difference calculated in S254. The nozzle actuator 40 receives the instruction, and the air bubble forming unit 200 proceeds the processing to S250. In S250 of the second and subsequent times, the air bubble forming unit 200 sends an instruction to the nozzle actuator 40 to operate by the amount according to the operation amount.

FIG. 9C is an example of a flow of moving the relative position between the nozzle 49 and the manipulation target 35, based on an inner pressure of an air bubble formed on the end part 254 of the nozzle 49.

First, in S260, the air bubble forming unit 200 controls the pressure generating unit 47 to form an air bubble on the end part 254 of the nozzle 49. Prior to forming the air bubble, the air bubble forming unit 200 may send an instruction to the nozzle actuator 40 to cause an operation of the end part 254 of the nozzle 49 into liquid. The step of forming the air bubble in S260 and the sub-step may be the same as the step of S300 and the sub-step to be described later.

Then, in S262, the air bubble forming unit 200 sends an instruction to the nozzle actuator 40 to cause an operation of the nozzle 49 to a preset position. The nozzle actuator 40 may be an actuator for controlling a z position. In this case, the z position is controlled based on a value of the inner pressure measured with the sensor unit 48. The nozzle 49 may be positioned above a region where an organism does not exist on the bottom surface of the vessel 25. After the nozzle actuator 40 has moved the nozzle 49 to the preset position, the air bubble forming unit 200 proceeds the processing to S264.

Note that, in S262, an edge of the nozzle 49 may sense a liquid surface and control the z position, based on the value of the inner pressure measured with the sensor unit 48. The air bubble forming unit 200 maintains the inner pressure of the nozzle to a pressure equal to or greater than atmospheric pressure, or a pressure equal to or smaller than atmospheric pressure, and once the edge of the nozzle 49 reaches the liquid surface, the value of the inner pressure measured with the sensor unit 48 varies due to external force produced by deformation of the gas-liquid interface caused by the contact to the liquid surface. Therefore, the air bubble forming unit 200 can determine whether the edge of the nozzle 49 has reached the liquid surface by measuring the value of the inner pressure of the air bubble. In this manner, even when a position to move the nozzle 49 is not previously set, the air bubble forming unit 200 can send the nozzle actuator 40 an instruction of a z position control with the liquid surface as a reference position, and move the position of the nozzle 49.

Then, in S264, the sensor unit 48 measures an inner pressure of the formed air bubble, and sends a measured value of the inner pressure of the air bubble to the air bubble forming unit 200. Once the air bubble reaches the bottom portion of the vessel 25, the shape of the air bubble deforms due to an interaction with the bottom portion, and the inner pressure of the air bubble varies exponentially. Therefore, the air bubble forming unit 200 can determine whether the air bubble has reached the bottom portion of the vessel 25 by measuring the value of the inner pressure of the air bubble. In addition, as another example of inner pressure measurement, while the nozzle 49 being moved in the downward direction with the nozzle actuator 40, the sensor unit 48 may measure the inner pressure, or the air bubble forming unit 200 may control the pressure, or the pressure generating unit 47 may be actuated. By simultaneously operating in this manner, it is possible to speed up detection of the bottom portion and the like, and use a variation process of pressures in a formation process of an air bubble as a detection index. Note that, instead of the sensor unit 48, the nozzle actuator 40 may measure an inner pressure of the air bubble, and send a measured value of the inner pressure to the air bubble forming unit 200.

Then, in S266, the air bubble forming unit 200 determines whether the measured value of the inner pressure of the air bubble is within a range of a preset inner pressure. If the measured value of the inner pressure is outside the range of the preset inner pressure, the air bubble forming unit 200 proceeds the processing to S268, and if not, proceeds the processing to S270. As described above, the air bubble forming unit 200 calculates an absolute value of a difference between the set inner pressure and the measured inner pressure of the air bubble, and if the difference is equal to or greater than a threshold, the air bubble forming unit 200 determines that the nozzle 49 has reached the bottom portion of the vessel 25.

In S268, the air bubble forming unit 200 decides an operation amount of the nozzle actuator 40. For example, the air bubble forming unit 200 decides an operation amount of the nozzle actuator 40, and sends an instruction to the nozzle actuator 40 to operate by the decided operation amount, for causing an operation of the nozzle 49 to the preset position. For example, the air bubble forming unit 200 may decide an operation amount of an amount according to the level of difference calculated in S266. The nozzle actuator 40 receives the instruction, and the processing proceeds to S262. In S262 of the second and subsequent times, the air bubble forming unit 200 sends an instruction to the nozzle actuator 40 to operate by the amount according to the operation amount.

In S270, the air bubble forming unit 200 controls the pressure generating unit 47 to remove the air bubble from the end part 254 of the nozzle 49. The step of removing the air bubble in S270 and the sub-step may be the same as the step of S500 and the sub-step to be described later.

Then, in S272, the air bubble forming unit 200 sets the initial z position of the nozzle 49. The step of S272 may be the same as the step of S245. After the air bubble forming unit 200 has set the initial z position of the nozzle 49, the air bubble forming unit 200 proceeds the processing to S274.

Then, in S274, the air bubble forming unit 200 sends an instruction to the nozzle actuator 40 to cause an operation of the nozzle 49 to a set x, y, and z position that is preset (that is, the initial position). A movement of the nozzle 49 may be a movement on the xy plane, and it may include moving in the z direction as necessary. After the nozzle actuator 40 has moved the nozzle 49 to the set x, y, and z position that is preset, the air bubble forming unit 200 proceeds the processing to S276.

Then, in S276, the flow channel imaging camera 42 captures an image of the end part 254 of the nozzle 49. The flow channel imaging camera 42 sends the captured image to the image processing unit 300. The image processing unit 300 may record the image in the recording unit 190 and/or output the image to the air bubble forming unit 200.

Then, in S280, the air bubble forming unit 200 determines whether the position of the nozzle 49 is different from the preset position, based on the image of the end part 254 of the nozzle 49 that is captured. For example, the air bubble forming unit 200 calculates a difference between the positions from the image of the end part 254 of the nozzle 49 that is captured, and an image of the end part 254 of the nozzle in a set x, y, and z position that is preset (that is, the initial position), and if the difference is equal to or greater than a threshold, the air bubble forming unit 200 may determine that the position of the nozzle 49 is different from the initial position.

If the position of the nozzle 49 is determined to be different from the initial position, the air bubble forming unit 200 proceeds the processing to S282, and if not, proceeds the processing to S300.

In S282, the air bubble forming unit 200 decides an operation amount of the nozzle actuator 40. For example, the air bubble forming unit 200 decides an operation amount of the nozzle actuator 40, and sends an instruction to the nozzle actuator 40 to operate by the decided operation amount, for causing an operation of the nozzle 49 to the set x, y, and z position that is preset (that is, the initial position). For example, the air bubble forming unit 200 may decide an operation amount of an amount according to the level of the difference calculated in S280. The nozzle actuator 40 receives the instruction, and the air bubble forming unit 200 proceeds the processing to S274. In S274 of the second and subsequent times, the air bubble forming unit 200 sends an instruction to the nozzle actuator 40 to operate by the amount according to the operation amount.

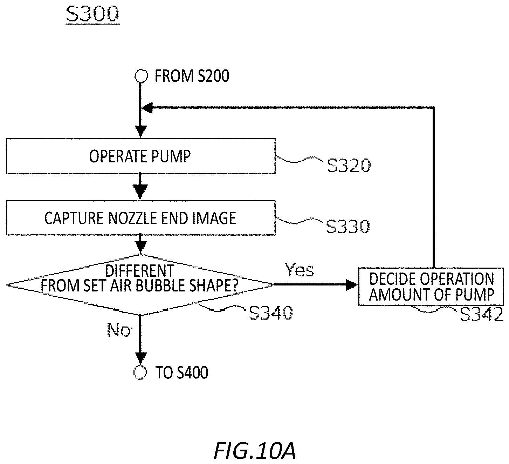

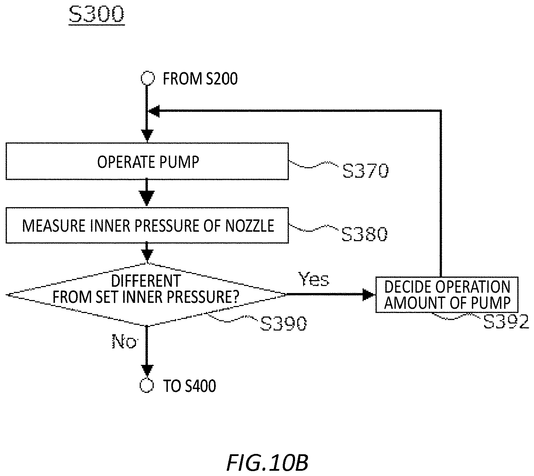

In S300, the air bubble forming unit 200 controls the pressure generating unit 47 to enlarge the gas-liquid interface 255. For example, enlarging the gas-liquid interface 255 may include forming an air bubble. Prior to forming the air bubble, the air bubble forming unit 200 may send an instruction to the nozzle actuator 40 to cause an operation of the end part 254 of the nozzle 49 into liquid. In S300, the step of enlarging the gas-liquid interface 255 includes the steps from S320 to S342 as illustrated in FIG. 10A, or includes the steps from S370 to S392 as illustrated in FIG. 10B.

FIG. 10A is an example of a flow of enlarging the gas-liquid interface 255, based on an image that is obtained by imaging the position of the end part 254 of the nozzle 49.

In S320, the air bubble forming unit 200 sends an instruction to the pressure generating unit 47 connected to the flow channel 51, to enlarge the gas-liquid interface 255 at the edge of the flow channel 51 (form an air bubble). For example, the air bubble forming unit 200 sends an instruction to the pressure generating unit 47 such that the actuator of the pressure generating unit 47 pushes the plunger of the syringe pump by a preset distance, or pushes the plunger of the syringe pump until a preset pressure is achieved. As a result, the gas pushed out from the syringe pump is supplied to the flow channel 51, and the gas-liquid interface 255 at the edge of the flow channel 51 is enlarged (an air bubble is formed). After the gas-liquid interface 255 has been enlarged (an air bubble is formed), the air bubble forming unit 200 proceeds the processing to S330.

Then, in S330, the flow channel imaging camera 42 captures an image of the air bubble formed on the end part 254 of the nozzle 49. The flow channel imaging camera 42 sends the captured image to the image processing unit 300. The image processing unit 300 may record the image in the recording unit 190 and/or output the image to the air bubble forming unit 200.

Then, in S340, the air bubble forming unit 200 determines whether the shape of the formed air bubble is different from a preset air bubble shape, based on the image of the air bubble that is captured. The air bubble forming unit 200 predicts the shape of the air bubble formed on the end part 254 of the nozzle 49, from information such as a set inner pressure in the nozzle 49, an inner diameter of the end part 254 of the nozzle 49, wettability of the nozzle 49 (a contact angle of the liquid), the type of the liquid, and the type of the gas. For example, the air bubble forming unit 200 may determine whether the shape of the formed air bubble is different from the set air bubble shape, by comparing the image of the air bubble that is captured, with the shape of the air bubble predicted from the above-described information.

If the shape of the formed air bubble is different from the set air bubble shape, the air bubble forming unit 200 proceeds the processing to S342, and if not, proceeds the processing to S400.

In S342, the air bubble forming unit 200 decides an operation amount of the plunger of the syringe pump of the pressure generating unit 47. For example, the air bubble forming unit 200 decides an operation amount of the plunger of the syringe pump of the pressure generating unit 47 (for example, the distance of pushing or drawing the plunger of the syringe pump) such that the air bubble formed on the edge of the nozzle 49 is formed in a preset shape. The air bubble forming unit 200 sends an instruction to the pressure generating unit 47 to operate by the decided operation amount. For example, the air bubble forming unit 200 may decide an operation amount of an amount according to the level of the difference calculated in S340.

The operation amount may be an operation amount of the actuator of the pressure generating unit 47, or may be an additional pressure loaded on the syringe pump. The pressure generating unit 47 receives the instruction, and the air bubble forming unit 200 proceeds the processing to S320. In S320 of the second and subsequent times, the pressure generating unit 47 performs an operation of an amount according to the operation amount.

FIG. 10B is an example of a flow of enlarging the gas-liquid interface 255, based on an inner pressure in the nozzle 49.

The step of S370 may be the same as the step of S320. Upon completion of S370, the air bubble forming unit 200 proceeds the processing to S380.

Then, in S380, the sensor unit 48 measures an inner pressure in the nozzle 49, and sends a measured value of the inner pressure in the nozzle 49 to the air bubble forming unit 200. Note that, instead of the sensor unit 48, the nozzle actuator 40 may measure an inner pressure in the nozzle 49, and send a measured value of the inner pressure to the air bubble forming unit 200.

Then, in S390, the air bubble forming unit 200 determines whether the measured value of the inner pressure in the nozzle 49 is within a preset range of the inner pressure. If the measured value of the inner pressure is outside the set range of the inner pressure, the air bubble forming unit 200 proceeds the processing to S392, and if not, proceeds the processing to S400. For example, the air bubble forming unit 200 calculates a difference between the preset inner pressure and the measured inner pressure in the nozzle 49, and if the difference is equal to or greater than a threshold, may determine that the set inner pressure has not been achieved.

In S392, the air bubble forming unit 200 decides an operation amount of the plunger of the syringe pump of the pressure generating unit 47 (for example, the distance of pushing or drawing the plunger of the syringe pump) to achieve the set inner pressure in the nozzle 49. The air bubble forming unit 200 sends an instruction to the pressure generating unit 47 to operate by the decided operation amount. For example, the air bubble forming unit 200 may decide an operation amount of an amount according to the level of the difference calculated in S390.

The operation amount may be an operation amount of the actuator of the pressure generating unit 47, or may be an additional pressure loaded on the syringe pump. The pressure generating unit 47 receives the instruction, and the air bubble forming unit 200 proceeds the processing to S370. In S370 of the second and subsequent times, the pressure generating unit 47 performs an operation of an amount according to the operation amount.

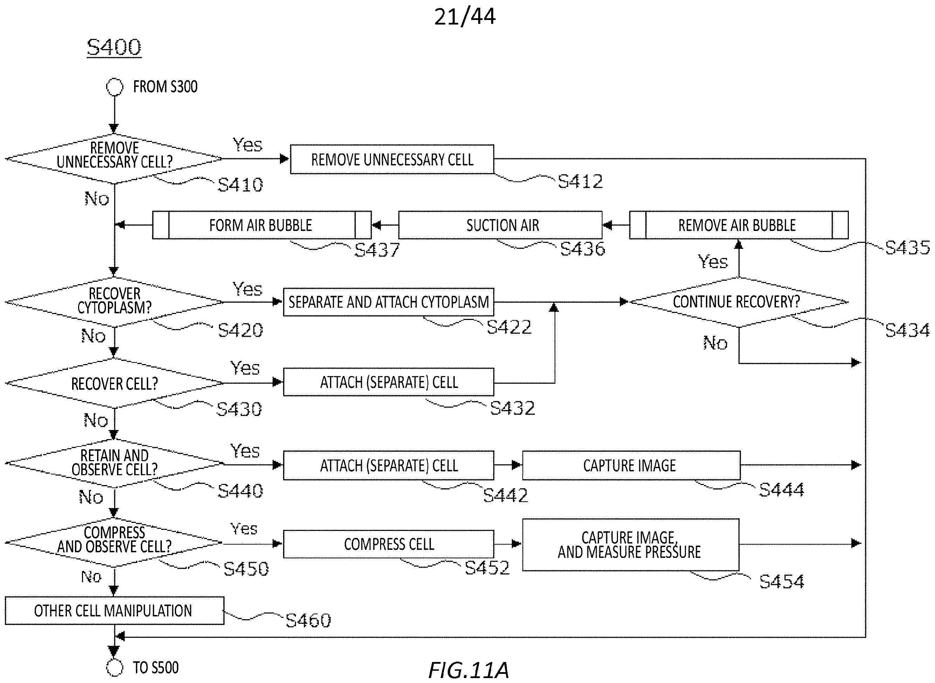

In S400, the gas-liquid interface manipulation unit 101 performs a manipulation on the manipulation target 35. For example, the air bubble forming unit 200 sends an instruction to the gas-liquid interface manipulation unit 101 to perform a manipulation on the manipulation target 35, based on an instruction received via the input unit 180. In S400, the step of performing the manipulation includes the steps from S410 to S460 as illustrated in FIG. 11A. For example, the manipulation may be removal of an unnecessary cell, recovery or movement of a cell membrane and/or a cell membrane, recovery of a cell, retention of a cell, or compression of a cell.

FIG. 11A is an example of a flow of performing a manipulation on the manipulation target 35. FIG. 11A describes an example when the manipulation target 35 is a cell. Note that, the manipulation target 35 may be other organisms, not limited to cells.

First, in S410, if an instruction to remove an unnecessary cell has been received in S140, the information processing device 170 proceeds the processing to S412. In S410, if an instruction not to remove an unnecessary cell has been received in S140, the information processing device 170 proceeds the processing to S420.

In S412, the air bubble forming unit 200 attaches the cell to the formed air bubble for removal.

For example, the air bubble forming unit 200 sends an instruction to the nozzle actuator 40 to move a position of the nozzle 49, in the x, y, and z directions, to a position where a target cell is present. After the nozzle actuator 40 has moved the nozzle 49 to the target position, the air bubble forming unit 200 may move the nozzle 49 and/or the stage, and bring the gas-liquid interface 255 of the air bubble into contact with the cell.

As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the cell is brought into contact with the gas-liquid interface 255 of the air bubble, as illustrated in 290a to 290c of FIG. 4, the nozzle actuator 40 may recover the target cell in the flow channel 51, and the liquid storage part 54 may discard the cell to the liquid discarding part of the liquid storage part 54.

In addition, as another example, the air bubble forming unit 200 may form the gas-liquid interface 255 in a size that is predetermined in the step of S300 and the sub-step, and may attach the cell to the gas-liquid interface 255 and separate the cell by performing a control to enlarge the gas-liquid interface 255. After the liquid control unit 260 has removed the unnecessary cell, the air bubble forming unit 200 proceeds the processing to S500.

In S420, if an instruction to recover a cytoplasm and/or a cell membrane has been received in S140, the air bubble forming unit 200 proceeds the processing to S422, and if not, proceeds the processing to S430.

In S422, the air bubble forming unit 200 uses the formed air bubble to separate the cytoplasm and/or the cell membrane, and attach these to the air bubble for recovery. For example, the air bubble forming unit 200 controls the pressure generating unit 47 to form an air bubble on the edge of the flow channel 51, and attaches the target cell to the air bubble. Then, the air bubble forming unit 200 compresses the cell with the air bubble, and cuts off only the cytoplasm and/or the cell membrane portion, and attaches it to the air bubble. For example, the air bubble forming unit 200 compresses the cell by raising the inner pressure of the air bubble or enlarging the air bubble by controlling the pressure generating unit 47, or by pressing the air bubble against the cell by controlling the nozzle actuator 40 to move the nozzle 49 toward the cell. Subsequently, the air bubble forming unit 200 moves a part of the cell swollen to the outside of the cell due to the compression, in a manner such that it is cut off from the cell on the gas-liquid interface, thereby cutting off the cytoplasm and/or the cell membrane portion from the cell. In this manner, the air bubble forming unit 200 controls the nozzle actuator 40 or the pressure generating unit 47 to cut out a necessary cytoplasm and/or cell membrane among the manipulation target 35.

For example, the air bubble forming unit 200 may send an instruction to the nozzle actuator 40 to move the nozzle 49 from the initial position, in the x, y, and z directions, to a position where the target cell is present. After the nozzle actuator 40 has moved the nozzle 49 to the target position, the air bubble forming unit 200 may move the nozzle 49 and/or the stage with the nozzle actuator 40 or the sample actuator 41, and may bring the gas-liquid interface 255 of the air bubble into contact with the cell. As an example, the air bubble forming unit 200 identifies the position where the target cell is present from an image that is obtained by imaging the target cell with the camera 60 or the camera 70, and controls the nozzle actuator 40 and moves the nozzle 49 to match the center of the nozzle 49 with the target position. The position where the target cell is present may be identified by the manipulator. In this case, the air bubble forming unit 200 may receive, from the input unit 180, an input by the manipulator as to the position where the target cell is present, and identify the position.

A cell membrane has been known to have a portion showing a relatively soft physical property and a portion showing a relatively hard physical property, due to a difference in the composition of lipids constituting the membrane component. The air bubble forming unit 200 controls the nozzle actuator 40 or the pressure generating unit 47, and compresses the cell with the air bubble. The cell may be compressed with the air bubble by the air bubble forming unit 200 forming the gas-liquid interface 255 of a predetermined size in the step of S300 and the sub-step, and performing a control to enlarge the gas-liquid interface 255, thereby attaching the cell to the gas-liquid interface 255. Then, by using swelling of the portion showing a relatively soft physical property of the cell membrane to the outside, the gas-liquid interface 255 may be used to attach the swollen portion, and move it in a direction away from the cell to cut off the cell membrane, thereby recovering the cell membrane to the flow channel 51 while it is attached to the gas-liquid interface 255. In addition, when cutting out the cell membrane in this manner, since the cell membrane swells by being pushed from the inside by the cytoplasm, the cut-off cell membrane includes a cytoplasmic component on the inside, and thus the cytoplasmic component can also be recovered to the flow channel 51. After the nozzle actuator 40 has cut off the necessary cytoplasm and/or cell membrane portion, the air bubble forming unit 200 proceeds the processing to S434.

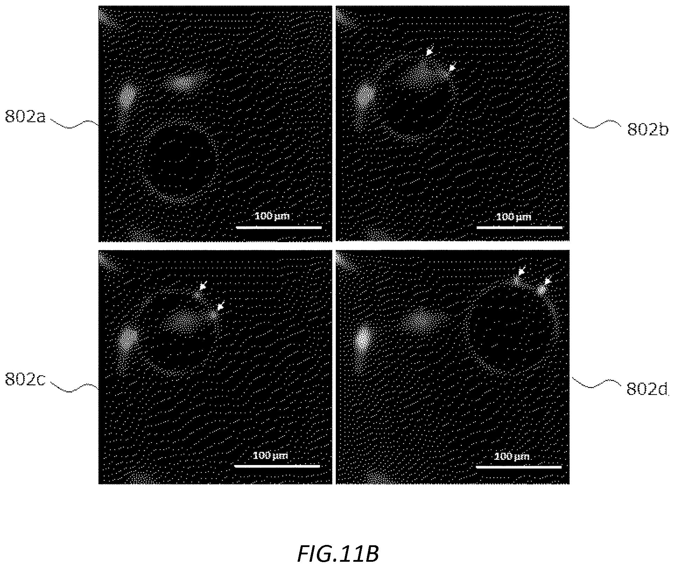

FIG. 11B illustrates a behavior of recovering a cytoplasm and a cell membrane from a cell, according to the present embodiment. To bring a HeLa cell (human cervical cancer cell) cultured on a solid phase of the vessel 25 into contact with the gas-liquid interface 255 of an air bubble, with the air bubble forming unit 200 controlling the pressure generating unit 47 and the nozzle actuator 40, a relative position between the cell and the nozzle 49 was brought close (802a). Then, with the air bubble forming unit 200 performing a control to press the gas-liquid interface 255 against the cell, the cell was compressed (802b). At this time, due to the compression, a behavior that a soft portion of the cell membrane swells to the outside was observed (the arrow of 802b). Then, regarding this swollen portion, a cytoplasm and a cell membrane in the swollen portion were cut off by moving the gas-liquid interface 255 in a direction away from the cell (the arrow of 802c). Finally, the cut-off cytoplasm and cell membrane were recovered by being attached to the gas-liquid interface 255 (802d). Note that, when performing the operation of FIG. 11B, the operation may be performed by enlarging the gas-liquid interface 255, or may be performed by moving the nozzle 49, or may be performed by moving the stage.

Then, in S434, the air bubble forming unit 200 determines whether the instruction received in S140 includes continuously recovering the cytoplasm and/or the cell membrane of the manipulation target 35. The air bubble forming unit 200 proceeds the processing to S435 if the determination is positive, and to S500 if the determination is negative.

In S435, the air bubble forming unit 200 controls the pressure generating unit 47 to remove the air bubble formed on the end part 254 of the nozzle 49. When removing the air bubble, recovery of the manipulation target 35 may be performed at the same time. For example, the manipulation target 35 may be recovered into liquid existing in the flow channel 51. The step of removing the air bubble in S435 and the sub-step may be the same as the step of S500 and the sub-step, and the details thereof will be described below.

Then, in S436, the air bubble forming unit 200 controls the pressure generating unit 47 and the nozzle actuator 40 to perform intake of gas for forming a new air bubble on the end part 254 of the nozzle 49. The air bubble forming unit 200 sends an instruction to the nozzle actuator 40 to take out the nozzle 49 from the liquid. After the nozzle actuator 40 has taken out the nozzle 49 from the liquid, the pressure generating unit 47 may draw the plunger of the syringe pump to take in a necessary amount of the gas.

Then, in S437, the air bubble forming unit 200 controls the pressure generating unit 47 to form a new air bubble on the end part 254 of the nozzle 49. For the step of S437 and the sub-step, the content of S300 that is already described may be applied. After the pressure generating unit 47 has formed an air bubble on the end part 254 of the nozzle 49, the air bubble forming unit 200 proceeds the processing to the step of S420.

In S430, the air bubble forming unit 200 determines whether the instruction to recover a cell has been received in S140. The air bubble forming unit 200 proceeds the processing to S432 if the determination is positive, and the air bubble forming unit 200 proceeds the processing to S440 if the determination is negative.

In S432, the air bubble forming unit 200 controls the pressure generating unit 47 to form an air bubble, and attaches the cell to the air bubble. The air bubble forming unit 200 may, as necessary, separate the cell attached to the air bubble from the solid phase.

For example, the air bubble forming unit 200 sends an instruction to the nozzle actuator 40 to move the nozzle 49 from the initial position, in the x, y, and z directions, to a position where a target cell is present. After the nozzle actuator 40 has moved the nozzle 49 to the target position, the air bubble forming unit 200 controls the pressure generating unit 47 to supply gas to the flow channel 51, thereby forming an air bubble on the edge of the flow channel 51. Then, the air bubble forming unit 200 may control the nozzle actuator 40 or the sample actuator 41 to move the nozzle 49 or the stage, thereby bringing the gas-liquid interface 255 of the air bubble into contact with the cell.

As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the nozzle actuator 40 or the sample actuator 41 has moved the nozzle 49 to the target position, the air bubble forming unit 200 controls the pressure generating unit 47 to supply gas to the flow channel 51, thereby forming an air bubble on the edge of the flow channel 51. Then, the air bubble forming unit 200 may move the nozzle 49 and/or the stage with the nozzle actuator 40 or the sample actuator 41, and bring the gas-liquid interface 255 of the air bubble into contact with the cell. For example, after bringing the cell into contact with the gas-liquid interface 255 of the air bubble, the nozzle actuator 40 may separate the cell, as necessary, by a means such as moving the gas-liquid interface 255.

After the air bubble forming unit 200 has controlled the pressure generating unit 47 to form an air bubble, and attached a cell to the air bubble, the air bubble forming unit 200 proceeds the processing to S434. For the steps of S434 and onwards, the content that is already described may be applied. Note that, in this case, in the step of S434, an object to be continuously recovered is not limited to only a cytoplasm or only a cell, but it may be both the cytoplasm and the cell.

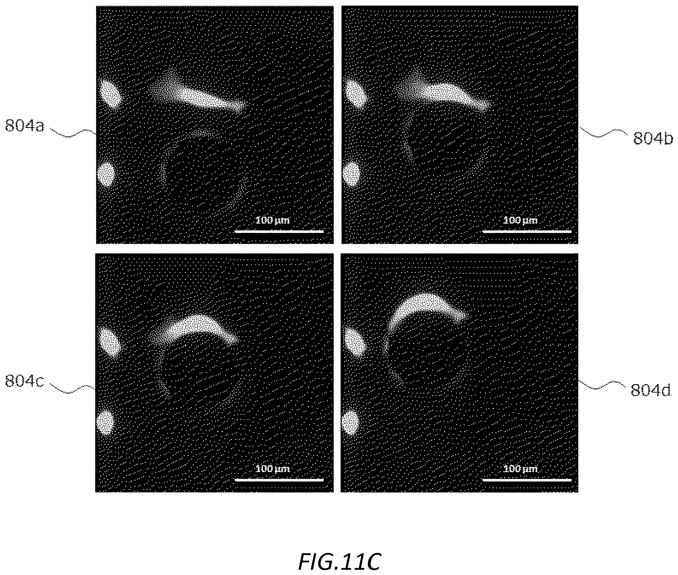

FIG. 11C illustrates a behavior of attaching an established cell line to an air bubble and separating the established cell line, according to the present embodiment. To bring a HeLa cell cultured on a solid phase of the vessel 25 into contact with the gas-liquid interface 255 of an air bubble, with the air bubble forming unit 200 controlling the pressure generating unit 47 and the nozzle actuator 40 (or the sample actuator 41 instead of the nozzle actuator 40), a relative position between the cell and the nozzle 49 was brought close (804a). Then, the cell contacted the gas-liquid interface 255 of the air bubble (804b). Then, the nozzle actuator 40 moved the gas-liquid interface 255 (804c), and the cell was attached to and separated from the gas-liquid interface 255 (804d). Note that, when performing the operation of FIG. 11C, the operation may be performed by enlarging the gas-liquid interface 255, or may be performed by moving the nozzle 49, or may be performed by moving the stage.

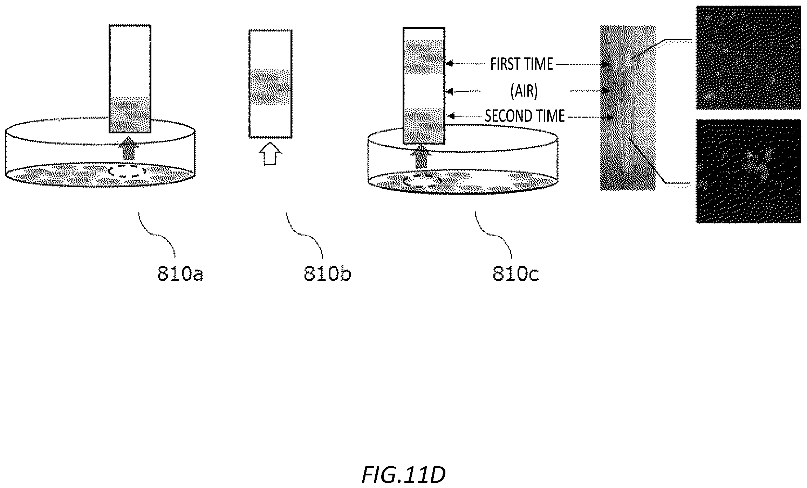

FIG. 11D illustrates a schematic view when repeating S430 → S432 → S434 → S435 → S436 → S437. When continuously recovering a cytoplasm and/or a cell membrane, and when continuously recovering a cell, these may be performed as shown in the schematic view of FIG. 11D. In 810a, after the nozzle actuator 40 has put the nozzle 49 in liquid, the air bubble forming unit 200 controls the pressure generating unit 47, and forms an air bubble on the end part 254 of the nozzle 49 by supplying gas to the syringe pump (not illustrated). Then, a cell adhered on a bottom surface of the vessel 25 is attached to the gas-liquid interface 255 of the air bubble and separated, and is recovered to the flow channel 51 by taking in the gas with the syringe pump. Then, in 810b, the nozzle actuator 40 pulls the nozzle 49 up from the liquid, and the syringe pump suctions the gas (for example, the air) to the flow channel 51. Then, in 810c, after the nozzle actuator 40 has put the nozzle 49 in the liquid, the syringe pump supplies gas to the end part 254 of the nozzle 49 to form a new air bubble, and a cell adhered on a bottom surface of the vessel 25 is recovered by being attached to the gas-liquid interface 255 of the air bubble. In this manner, with the air bubble forming unit 200 controlling the pressure generating unit 47 and the nozzle actuator 40, two cells (a population) can be continuously recovered in the flow channel 51, via the gas, without being mixed. A photograph showing two cells (a population) that are actually recovered with the air placed therebetween, by using this method, is also presented. Note that, FIG. 11D described the example of moving the nozzle 49, but instead of moving the nozzle 49, the operation of FIG. 11D may be performed by moving the stage.

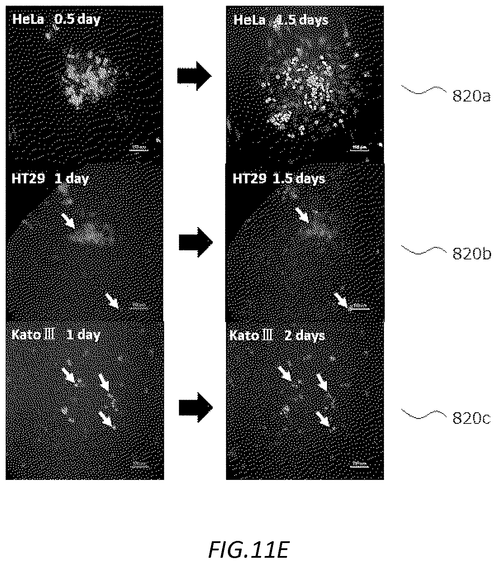

FIG. 11E illustrates a behavior of subculture in which an established cell line is recovered, and the recovered established cell line is cultured, according to the present embodiment. With the air bubble forming unit 200 controlling the pressure generating unit 47 and the nozzle actuator 40, a HeLa cell (human cervical cancer cell, 820a), a HT29 cell (human large bowel cancer cell, 820b), and a Kato III cell (human gastric signet ring cell cancer cell, 820c) that are established cell lines were attached to, and separated and recovered from a solid phase of the vessel 25 by using the gas-liquid interface 255 of an air bubble, and were released to a medium in another vessel to be cultured for 1.5 day (820a and 820b) and for 2 days (820c). For each cell, a cell proliferation was confirmed after the subculture. That is, according to the present embodiment, even when an established cell line was separated by using the gas-liquid interface 255 of an air bubble, the cell could be proliferated without damaging the viability of the cell. Note that, when performing the operation of FIG. 11E, the operation may be performed by moving the nozzle 49, or may be performed by moving the stage.

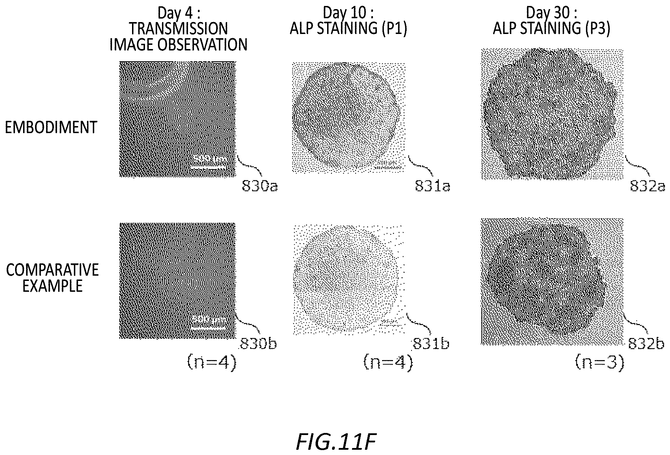

FIG. 11F illustrates a behavior of recovering an iPS cell, and subculturing the recovered iPS cell, according to the present embodiment. With the air bubble forming unit 200 controlling the pressure generating unit 47 and the nozzle actuator 40, a colony of the cultured iPS cells was attached to, and separated and recovered from a solid phase of the vessel 25 by using the gas-liquid interface 255 of an air bubble, and was released in a liquid culture medium in another vessel for observation of a transmission image after culturing for 4 days. In addition, as a comparative example, cells obtained by subculturing an iPS cell with an ordinary cell subculturing method (mechanical passage) was used. As a result, no morphological difference was recognized between the iPS cells of the present embodiment (830a) and the iPS cells of the comparative example (830b). In addition, after culturing for 10 days, staining of alkaline phosphatase was performed on the iPS cells of the present embodiment and the comparative example. Furthermore, after subculturing the iPS cell of the present embodiment and the comparative example for three times, and culturing the iPS cell for 30 days, staining of alkaline phosphatase was performed on the iPS cells of the present embodiment and the comparative example. As a result, no difference in stainability was recognized between the iPS cells of the present embodiment (831a is those cultured for 10 days, and 832a is those cultured for 30 days) and the iPS cells of the comparative example (831b is those cultured for 10 days, and 832b is those cultured for 30 days). It has been known that alkaline phosphatase is highly expressed in an iPS cell maintaining a self-replication ability that is undifferentiated. That is, according to the present embodiment, even when an iPS cell was separated and recovered by using the gas-liquid interface 255, the iPS cell could be proliferated while maintaining an undifferentiated state, without affecting maintainability of undifferentiation ability. Note that, when performing the operation of FIG. 11F, the operation may be performed by moving the nozzle 49, or may be performed by moving the stage.

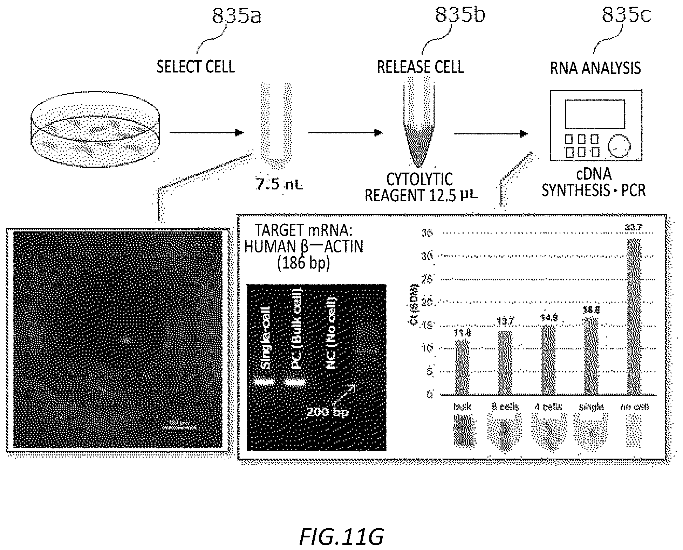

FIG. 11G illustrates a behavior of recovering an established cell line, and analyzing the recovered cell, according to the present embodiment. A HeLa cell that is an established cell line was separated and recovered from a solid phase of the vessel 25 by using the gas-liquid interface 255 of an air bubble. With the air bubble forming unit 200 controlling the pressure generating unit 47 and the nozzle actuator 40, one cell, four cells, and eight cells of the HeLa cells were selected and separated from the solid phase by using the gas-liquid interface 255 of the air bubble, and these cells were each recovered together with a liquid culture medium of 7.5 nL (835a). Then, the recovered HeLa cells were released to a 12.5 µL cytolytic reagent, and the cells were lysed (835b). In the cell lysate in which the HeLa cells are lysed, from mRNA of β-actin, a cDNA was synthesized, and a PCR reaction was performed (835c). As a result, an amount of cDNAs generally proportional to the number of recovered cells could be detected. That is, according to the present embodiment, cells could be recovered by one cell or an optional number of cells by using the gas-liquid interface 255, and a molecular biological analysis could be performed. Note that, when performing the operation of FIG. 11G, the operation may be performed by moving the nozzle 49, or may be performed by moving the stage.

Then, in S440, the air bubble forming unit 200 determines whether the instruction to retain and image the cell has been received in S140. The air bubble forming unit 200 proceeds the processing to S442 if the determination is positive, and to S450 if the determination is negative.

In S442, the air bubble forming unit 200 performs a control to attach the cell to the formed air bubble, and retain the attached cell. For example, the air bubble forming unit 200 sends an instruction to the nozzle actuator 40 to move the nozzle 49 from the initial position, in the x, y, and z directions, to a position where a target cell is present. After the nozzle actuator 40 has moved the nozzle 49 to the target position, the air bubble forming unit 200 controls the pressure generating unit 47 to supply gas to the flow channel 51, thereby forming an air bubble on the edge of the flow channel 51. The air bubble forming unit 200 may move the nozzle 49 and/or the stage with the nozzle actuator 40 or the sample actuator 41, and may bring the gas-liquid interface 255 of the air bubble into contact with the cell. As an example, the nozzle actuator 40 or the sample actuator 41 identifies the position where the target cell is present, from an image that is obtained by imaging the target cell with the camera 60 or the camera 70, and moves the center of the nozzle 49 to match the target position. After the cell has been brought into contact with the gas-liquid interface 255 of the air bubble, the air bubble forming unit 200 proceeds the processing to S444.

The position where the target cell is present may be identified by the manipulator. In this case, the air bubble forming unit 200 may receive, from the input unit 180, an input by the manipulator as to the position where the target cell is present, and identify the position. In addition, an edge surface of the air bubble is brought into contact with the cell in the above-described example, but the contact between the air bubble and the cell may be performed by bringing a side surface of the air bubble into contact with the cell. In this case, the nozzle actuator 40 or the sample actuator 41 may move the center of the nozzle 49 to match a vicinity of the target cell. In addition, for example, after bringing the cell into contact with the gas-liquid interface 255 of the air bubble, the nozzle actuator 40 may separate the cell as necessary, by a means such as moving the gas-liquid interface 255.

Then, in S444, the imaging control unit 171 sends an instruction to the camera 60 or the camera 70 to image the cell retained in the air bubble. The camera 60 or the camera 70 captures an image, and sends the image to the image processing unit 300. The image processing unit 300 may record the image in the recording unit 190 and/or output the image to the air bubble forming unit 200.

After the camera 60 or the camera 70 has imaged the retained cell, the air bubble forming unit 200 proceeds the processing to S500.

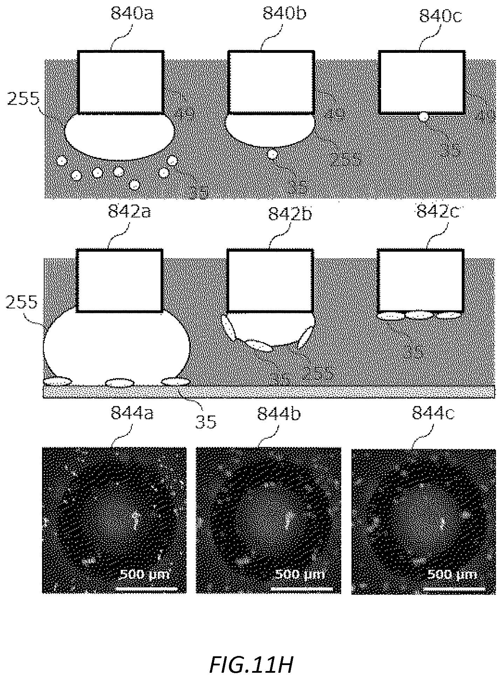

FIG. 11H is a schematic view showing a behavior of retaining an established cell line in the gas-liquid interface 255 of an air bubble, and performing an observation. Since a floating cell or a cell that is weakly adhered freely moves in a culture solution due to a slight vibration or the like, it is difficult to observe such cell as it is by using a microscope or the like. In 840a, the air bubble forming unit 200 controls the nozzle actuator 40 to put the end part 254 of the nozzle 49 in a liquid culture medium in the vessel 25 where a floating cell (the manipulation target 35) is cultured. Then, the pressure generating unit 47 supplies gas to the flow channel 51 to form an air bubble on the edge of the nozzle 49, thereby forming the gas-liquid interface 255. The air bubble forming unit 200 attaches a floating cell to become the manipulation target 35 to the gas-liquid interface 255, which is formed by controlling the nozzle actuator 40 or the pressure generating unit 47. Then, in 840b, the pressure generating unit 47 controls an inner pressure of the air bubble to temporarily retain the cell on the gas-liquid interface 255 of the air bubble. Then, in 840c, the pressure generating unit 47 takes in the gas from the flow channel 51 to shrink the air bubble. The cell retained in that state can be observed by using a microscope or the like. Alternatively, the pressure generating unit 47 may retain the cell without shrinking the air bubble, and the retained cell may be observed by using a microscope or the like. In this manner, according to the present embodiment, by retaining a cell on the gas-liquid interface 255 of an air bubble, an observation can be performed without moving the cell.

In addition, in the case of adherent cells that are dispersed on a solid phase of the vessel 25, when performing an observation with a microscope or the like, it is necessary to move the stage and observe with a wide field. In 842a, the air bubble forming unit 200 controls the nozzle actuator 40 to put the end part 254 of the nozzle 49 in a liquid culture medium of the vessel 25 where an adherent cell (the manipulation target 35) is cultured. Then, the pressure generating unit 47 supplies gas to the flow channel 51 to form an air bubble on the edge of the nozzle 49, thereby forming the gas-liquid interface 255. Then, the air bubble forming unit 200 controls the nozzle actuator 40 or the pressure generating unit 47 to control the gas-liquid interface 255, thereby attaching the cells to the gas-liquid interface 255 and separating the cells. Then, in 842b, the pressure generating unit 47 temporarily retains the cells on the gas-liquid interface 255 of the air bubble. Then, in 842c, the pressure generating unit 47 takes in the gas from the flow channel 51 to shrink the air bubble. The cells retained in such state are present in a narrow range on a plane surface of the same z position, and thus the cells can be observed all at once by using a microscope or the like. In this manner, according to the present embodiment, a field to be observed can be reduced by retaining cells on a gas-liquid interface of an air bubble.

As an example of such observation technique, Kato III cells that are floating cells were dispersed on a solid phase, and some of the cells were attached to the gas-liquid interface 255 (844a). Subsequently, the air bubble forming unit 200 controls an inner pressure of the air bubble via the pressure generating unit 47 to retain the cells on the gas-liquid interface 255 while shrinking the air bubble, and when the retained cells were focused, surrounding cells became out of focus (844b). At this time, when moving the stage, the surrounding cells will move but the cells retained on the gas-liquid interface 255 will not move, and thus the retained cells can be easily observed with a microscope (844c).

In S450, the air bubble forming unit 200 determines whether the instruction to compress and image the cell has been received in S140. The air bubble forming unit 200 proceeds the processing to S452 if the determination is positive, and to S460 if the determination is negative.

In S452, the air bubble forming unit 200 controls the pressure generating unit 47 and the nozzle actuator 40 to compress the cell by using the air bubble. For example, the air bubble forming unit 200 controls the pressure generating unit 47 to form an air bubble on the edge of the flow channel 51, and brings the air bubble into contact with the cell to become the manipulation target 35. Note that, when performing the operation of FIG. 11H, the operation may be performed by moving the nozzle 49, or may be performed by moving the stage.

As an example, the air bubble forming unit 200 sends an instruction to the nozzle actuator 40 to move the nozzle 49 from the initial position, in the x, y, and z directions, to a position where the target cell is present. As an example, the air bubble forming unit 200 identifies the position where the target cell is present from an image that is obtained by imaging the target cell with the camera 60 or the camera 70, and controls the nozzle actuator 40 and moves the nozzle 49 to match the center of the nozzle 49 with the target position.

The position where the target cell is present may be identified by the manipulator. In this case, the air bubble forming unit 200 may receive, from the input unit 180, an input by the manipulator as to the position where the target cell is present, and identify the position. In addition, the contact between the air bubble and the cell may be performed by bringing an edge surface of the air bubble into contact with the cell, or may be performed by bringing a side surface of the air bubble into contact with the cell. When bringing the edge surface of the air bubble into contact with the cell, the nozzle actuator 40 or the sample actuator 41 may move the center of the nozzle 49 to match immediately above the target cell. When bringing the side surface of the air bubble into contact with the cell, the nozzle actuator 40 or the sample actuator 41 may move the center of the nozzle 49 to match a vicinity of the target cell, and in this case, an air bubble can be formed on the side of the cell, and the cell can be gradually compressed from the side by moving the nozzle 49.

Then, after the nozzle actuator 40 has moved the nozzle 49 to the target position, the air bubble forming unit 200 controls the pressure generating unit 47 to supply gas to the flow channel 51, thereby forming an air bubble on the edge of the flow channel 51 in the target position. The air bubble forming unit 200 may move the nozzle 49 and/or the stage with the nozzle actuator 40 or the sample actuator 41, and may bring the gas-liquid interface 255 of the air bubble into contact with the cell. When the position of the nozzle 49 is fixed, the gas-liquid interface 255 and the cell may be brought into contact by moving the stage. The cell may be brought into contact with the gas-liquid interface 255 with a control by the air bubble forming unit 200 to enlarge the gas-liquid interface 255 via the pressure generating unit 47.

As an example, the air bubble forming unit 200 operates the plunger of the syringe pump of the pressure generating unit 47 with a preset operation amount to form an air bubble of a preset volume, and then uses the nozzle actuator 40 or the sample actuator 41 to move the nozzle 49 and/or the stage such that the nozzle 49 is positioned at a position where the air bubble compresses the cell. Then, the air bubble forming unit 200 may compress the cell by enlarging the air bubble formed on the edge of the flow channel 51 by controlling the pressure generating unit 47, or by pressing the air bubble against the cell by controlling the nozzle actuator 40 to move the nozzle 49 toward the cell.

In addition, as an example, the air bubble forming unit 200 may compress the cell by, after moving the nozzle 49 very near the cell, controlling the pressure generating unit 47 to form an air bubble of a preset volume on the edge of the flow channel 51.

Then, in S454, the imaging control unit 171 sends an instruction to the camera 60 or the camera 70 to image the compressed cell. The camera 60 or the camera 70 captures an image, and sends the image to the image processing unit 300. The image processing unit 300 may record the image in the recording unit 190 and/or output the image to the output unit 160.

Furthermore, instead of/in addition to this, the sensor unit 48 or the nozzle actuator 40 may measure a pressure of compressing the cell with the air bubble, and send a measured value of the pressure to the air bubble forming unit 200. The camera 60 or the camera 70 may repeat capturing of an image while changing a pressure of compressing the cell with the air bubble forming unit 200. The pressure of compressing the cell can be changed by the air bubble forming unit 200 controlling the pressure generating unit 47 and changing an inner pressure and/or a volume of the air bubble.

The air bubble forming unit 200 may, after moving the nozzle 49 very near the cell, control the pressure generating unit 47 to form an air bubble on the edge of the flow channel 51, and change the inner pressure and/or the volume of the air bubble to compress the entire cell or various parts of the cell while maintaining or changing the pressure to compress the cell. It is expected that hardness of various parts of a cell becomes different depending on the composition and distribution of a cell membrane or an intracellular organelle. In this manner, by using a pressure and/or an observed image at the time of compression with the air bubble as an index, the composition and distribution of the cell membrane in the cell or the intracellular organelle can be analyzed. By compressing the cell, the thickness of the cell becomes thin, and a structural object in a deep part of the cell can be clearly observed. Further, since the cell is broadened in the lateral direction, proximate structures become separate, and these can be individually separated and observed. By observing the cell while performing compression, information related to force applied to the cell and form variation amounts on an inside and an outside of the cell can be obtained, and it is possible to analyze information related to mechanics of the cell. After the camera 60 or the camera 70 has imaged the compressed cell, the air bubble forming unit 200 proceeds the processing to S500.

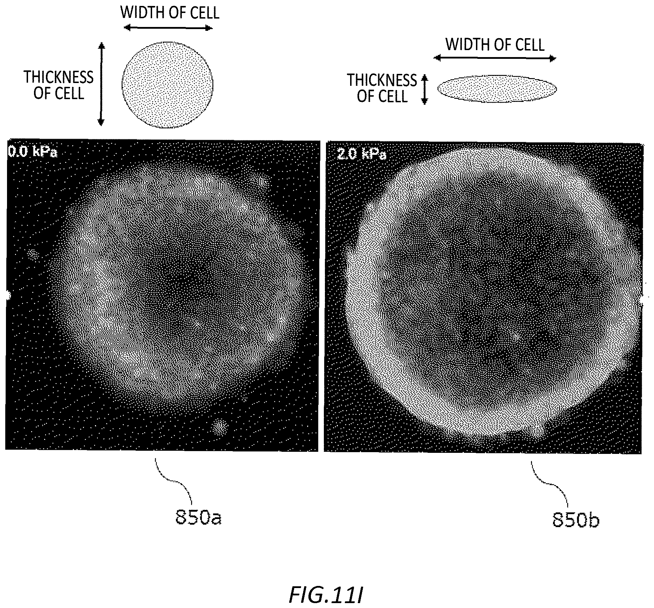

FIG. 11I illustrates a behavior of compressing an established cell line by using an air bubble, and observing a deep part of the cell, according to the present embodiment. By staining a nucleus and a cytoplasm of a spheroid (850a) formed from a HT29 cell in a living state, and performing compression by using an air bubble, a structure in a deep part of the cell such as the nucleus could be observed (850b). When comparing the center portion where the thickness is especially thick, the nucleus cannot be seen in the center portion in 850a before compression, while the nucleus can be confirmed in the center portion in 850b which is observed with compression. Conventionally, an observation of a structure in a deep part of a cell has been performed by fixing the cell with formalin, methanol, or the like, and thinly slicing the cell, or by using a special microscope that is specialized in a deep part observation. According to the present embodiment, a structure in a deep part of a cell can be observed in the deep part in a living state, without the use of a special microscope. Furthermore, nuclei are close to each other and it is difficult to recognize an individual nucleus in the spheroid before compression (850a), but since the spheroid is broadened in the longitudinal and lateral directions in the spheroid observed with compression (850b), a sufficient interval is generated between nuclei, and thus a nucleus can be independently recognized. Conventionally, a microscopic system that makes use of an optical system or a fluorescence labeling method has been researched and developed to independently recognize two or more proximate organelles inside the cell, and this is called a super-resolution microscope. Although the resolution is raised in such microscope techniques, the observation field becomes narrow, and the imaging time becomes longer. According to the present embodiment, two or more proximate organelles inside a cell can be independently recognized in a living state, with a short imaging time while maintaining an observation field, without the use of a special microscope. In addition, it is also possible to reproduce a three-dimensional structure of the original cell from an observed image of the compressed cell and mechanics information.

In S460, the air bubble forming unit 200 controls the gas-liquid interface manipulation unit 101 such that, among the instructions received in S140, necessary manipulations other than S410 to S450 are performed on the manipulation target 35. For example, the manipulations may be an evaluation on adhesiveness of the cell, induction of cell differentiation, and the like, but the manipulations will be described below. Upon completion of S460, the air bubble forming unit 200 proceeds the processing to S500.

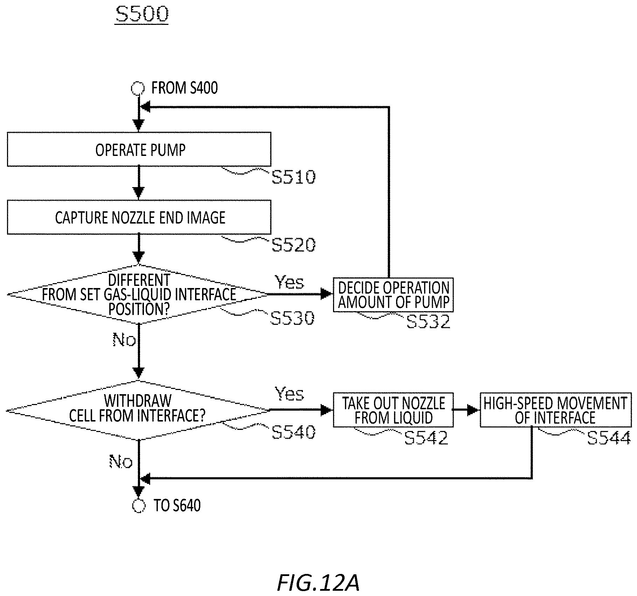

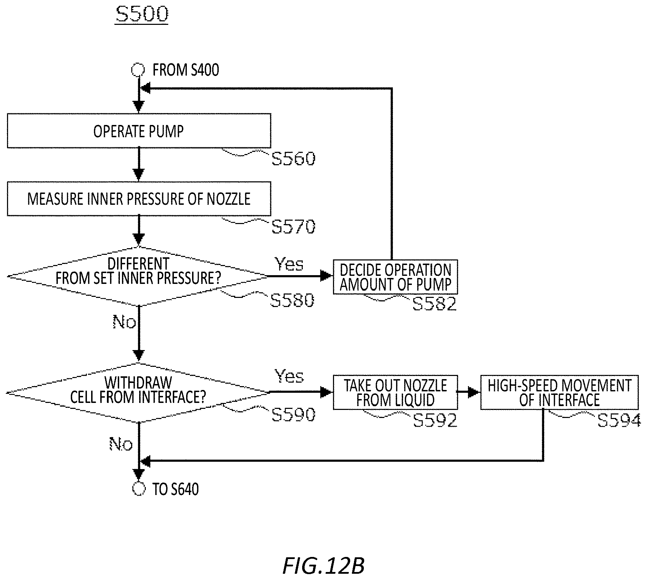

In S500, the air bubble forming unit 200 controls the pressure generating unit 47 to shrink the gas-liquid interface 255. Shrinkage of the gas-liquid interface 255 may include removal of an air bubble. When shrinking or removing an air bubble, recovery of the manipulation target 35 may be performed at the same time. In S500, the step of shrinking the gas-liquid interface 255 includes the steps from S510 to S544 as illustrated in FIG. 12A, or includes the steps from S560 to S594 as illustrated in FIG. 12B.

FIG. 12A is an example of a flow of shrinking the gas-liquid interface 255, based on an image that is obtained by imaging a position of the end part 254 of the nozzle 49.

In S510, the air bubble forming unit 200 controls the pressure generating unit 47 to perform a suction operation on the flow channel 51, and takes in the gas-liquid interface 255 from the edge of the flow channel 51. At this time, liquid is also taken in at the same time. The liquid to be taken in may be used to withdraw the manipulation target 35 from the gas-liquid interface 255. The liquid to be taken in may be liquid or the like (for example, a medium) contained in the vessel 25, or may be another liquid that is kept in the liquid storage part 54.

For example, the air bubble forming unit 200 sends an instruction to the pressure generating unit 47 such that the actuator of the pressure generating unit 47 draws the plunger of the syringe pump by a preset distance, or draws the plunger of the syringe pump until a preset pressure is achieved. Upon receiving the instruction from the volume control unit or the intake control unit in the air bubble forming unit 200, the pressure generating unit 47 takes in gas. As a result, the gas-liquid interface 255 is shrunk (the air bubble is removed), and the gas-liquid interface 255 is taken in the flow channel 51. After the gas-liquid interface 255 has been shrunk (the air bubble has been removed), the air bubble forming unit 200 proceeds the processing to S520.

Then, in S520, the flow channel imaging camera 42 captures an image of the end part 254 of the nozzle 49, and sends the image to the image processing unit 300. The image processing unit 300 may record the image in the recording unit 190 and/or output the image to the air bubble forming unit 200.

Then, in S530, the air bubble forming unit 200 determines whether a position of the gas-liquid interface 255 taken in the flow channel 51 is different from a preset position in the air bubble forming unit 200, based on the image of the end part 254 of the nozzle 49 that is captured. If the position is different, the air bubble forming unit 200 proceeds the processing to S532, and if not, proceeds the processing to S540. For example, the air bubble forming unit 200 calculates a difference between the position of the gas-liquid interface 255 calculated based on the image of the end part 254 of the nozzle 49 that is captured, and the preset position, and if the difference is equal to or greater than a threshold, the air bubble forming unit 200 may determine that the position of the gas-liquid interface 255 taken in the flow channel 51 is different from the set position.

In S532, the air bubble forming unit 200 decides an operation amount of the plunger of the syringe pump of the pressure generating unit 47. For example, the air bubble forming unit 200 decides an operation amount of the plunger of the syringe pump of the pressure generating unit 47 (for example, the distance of pushing or drawing the plunger of the syringe pump) to take in the gas-liquid interface 255 to the preset position of the gas-liquid interface 255. The air bubble forming unit 200 sends an instruction to the pressure generating unit 47 to operate by the decided operation amount. For example, the air bubble forming unit 200 may decide an operation amount of an amount according to the level of the difference calculated in S530.

The operation amount may be an operation amount of the actuator of the pressure generating unit 47, or may be an additional pressure loaded on the syringe pump. The pressure generating unit 47 receives the instruction, and the air bubble forming unit 200 proceeds the processing to S510. In S510 of the second and subsequent times, the pressure generating unit 47 performs an operation of an amount according to the operation amount.

In S540, if the instruction received in S140 includes withdrawal of the cell from the interface (for example, cell recovery), the air bubble forming unit 200 proceeds the processing to S542, and if not, the air bubble forming unit 200 proceeds the processing to S640.

In S542, the air bubble forming unit 200 sends an instruction to the nozzle actuator 40 to take out the nozzle 49 from the liquid. After the nozzle actuator 40 has taken out the nozzle 49 from the liquid by moving the nozzle 49 upward by a preset distance, the air bubble forming unit 200 proceeds the processing to S544.

Then, in S544, the air bubble forming unit 200 controls the pressure generating unit 47 to move the gas-liquid interface 255 between the gas in the flow channel 51 and the liquid at a high speed. In this manner, the cell attached to the gas-liquid interface 255 is withdrawn from the gas-liquid interface 255, and moves to the liquid. For example, the air bubble forming unit 200 causes the pressure generating unit 47 to rapidly perform a reciprocating operation of the plunger of the syringe pump, thereby moving the gas-liquid interface 255 at a high speed with repetition of gas supply and gas intake in the flow channel 51. Furthermore, in addition to/instead of this, the air bubble forming unit 200 may cause the nozzle actuator 40 to perform a reciprocating motion of the nozzle 49 at a high speed in the upward and downward directions (the ± z direction) and/or the longitudinal and lateral directions (the ± xy directions), thereby moving the gas-liquid interface 255 in the flow channel 51 at a high speed.

The air bubble forming unit 200 may cause a high-speed movement of the gas-liquid interface 255 at that location (the location where S542 was performed), or may cause that movement once the nozzle actuator 40 puts the nozzle 49 in the liquid at a designated moving destination. The air bubble forming unit 200 can appropriately withdraw the cell attached to the gas-liquid interface 255 from the gas-liquid interface 255, by controlling the moving speed of the liquid and the like from information such as an inner pressure in the nozzle 49 received from the sensor unit 48. In addition, the air bubble forming unit 200 may vibrate the gas-liquid interface 255 by forming an electromagnetic field in the nozzle 49.

Furthermore, the air bubble forming unit 200 may perform a control to withdraw the cell from the gas-liquid interface 255 by bringing the air bubble into contact with a filter. The air bubble forming unit 200 may control the liquid storage part 54 to add liquid that reduces free energy of the interface, thereby withdrawing the cell from the gas-liquid interface 255. Moreover, the air bubble forming unit 200 may control the nozzle actuator 40 and the pressure generating unit 47 to form an air bubble on the edge of the nozzle 49 at a designated moving destination, and scrape the cell on a bottom surface of the vessel 25 at the designated moving destination, thereby withdrawing the cell. In addition, the air bubble forming unit 200 may control the pressure generating unit 47 to raise an inner pressure of the air bubble, thereby pushing out and withdrawing the cell. Furthermore, the cell may be withdrawn from the gas-liquid interface 255 by using liquid that reduces free energy of the interface, as the liquid at the moving destination. After the cell has been withdrawn from the interface, the air bubble forming unit 200 proceeds the processing to S640.

In addition, an influence on an airflow can be controlled by changing the type and/or the composition of the gas. For example, when using heavy gas or gas having a high viscosity, an airflow becomes difficult to move, and an influence on the manipulation target 35 becomes large. On the other hand, when using light gas or gas having a low viscosity, an airflow becomes easy to move, and an influence on the manipulation target 35 becomes little. The gas may be selected by considering these influences of the type and/or the composition of the gas.

FIG. 12B is an example of a flow of shrinking the gas-liquid interface 255, based on an inner pressure in the nozzle 49.

In S560, the air bubble forming unit 200 controls the pressure generating unit 47 to cause a suction operation of the flow channel 51, and takes in the gas-liquid interface 255 from the edge of the flow channel 51. The step of S560 may be the same as the step of S510. Then, the air bubble forming unit 200 proceeds the processing to S570.

Then, in S570, the sensor unit 48 measures an inner pressure in the nozzle 49, and sends a measured value of the inner pressure of the nozzle 49 to the air bubble forming unit 200. Note that, instead of the sensor unit 48, the nozzle actuator 40 may measure an inner pressure in the nozzle 49, and send a measured value of the inner pressure to the air bubble forming unit 200.

Then, in S580, the air bubble forming unit 200 determines whether the measured value of the inner pressure in the nozzle 49 is within a preset range of the inner pressure. The air bubble forming unit 200 proceeds the processing to S582 if the measured value of the inner pressure is outside the preset range of the inner pressure, and if not, proceeds the processing to S590. For example, the air bubble forming unit 200 calculates a difference between the preset inner pressure and the measured inner pressure in the nozzle 49, and if the difference is equal to or greater than a threshold, may determine that the set inner pressure has not been achieved.

In S582, the air bubble forming unit 200 decides an operation amount of the plunger of the syringe pump of the pressure generating unit 47 (for example, the distance of pushing or drawing the plunger of the syringe pump) to achieve the set inner pressure in the nozzle 49. The air bubble forming unit 200 sends an instruction to the pressure generating unit 47 to operate by the decided operation amount. For example, the air bubble forming unit 200 may decide an operation amount of an amount according to the level of the difference calculated in S580.

The operation amount may be an operation amount of the actuator of the pressure generating unit 47, or may be an additional pressure loaded on the syringe pump. The pressure generating unit 47 receives the instruction, and the air bubble forming unit 200 proceeds the processing to S560. In S560 of the second and subsequent times, the pressure generating unit 47 performs an operation of an amount according to the operation amount.

In S590, if the instruction received in S140 includes withdrawal of the cell from the gas-liquid interface 255 (for example, cell recovery), the air bubble forming unit 200 proceeds the processing to S592. The steps from S590 to S594 may be the same as the steps from S540 to S544. Upon completion of S594, the air bubble forming unit 200 proceeds the processing to S640. If the instruction received in S140 does not include withdrawal of the cell from the gas-liquid interface 255 from the gas-liquid interface 255, the air bubble forming unit 200 proceeds the processing to S640.

Then, in S640, the information processing device 170 receives, via the input unit 180, an input from the manipulator related to release of the manipulation target 35. If the information processing device 170 has received an instruction to release the manipulation target 35, the information processing device 170 proceeds the processing to S645, and if not, proceeds the processing to S650.

In S645, the air bubble forming unit 200 may send, to the nozzle actuator 40, an instruction related to a moving destination of the recovered manipulation target 35. For example, the moving destination of the manipulation target 35 may be designated by a display area of a GUI by the manipulator, as illustrated in FIG. 7B. The nozzle actuator 40 may immerse the nozzle 49 including the manipulation target 35 attached to or withdrawn from the gas-liquid interface 255 in the liquid at the moving destination, and may perform release in the liquid at the moving destination. After the nozzle actuator 40 has released the recovered cell in the liquid at the moving destination, the air bubble forming unit 200 proceeds the processing to S650. Note that, the cell released in the liquid at the moving destination may be observed by using the microscope unit 50.

In S650, if there is another manipulation target 35, the air bubble forming unit 200 proceeds the processing to S660. In S650, if there is no other manipulation target 35, the air bubble forming unit 200 proceeds the processing to S680.

In S660, when manipulating the another manipulation target 35, if there is a need to exchange the nozzle 49, the air bubble forming unit 200 proceeds the processing to S670, and if there is no need to exchange the nozzle 49, proceeds the processing to S200.

In S670, the flow channel control unit 250 sends an instruction to the flow channel exchange unit 53 to remove the nozzle 49 mounted on the nozzle actuator 40, and discard it in the nozzle discarding part of the flow channel exchange unit 53. Note that, the nozzle may be kept with the cell still being taken in the nozzle 49 without being released, and an analysis on the cell that has been taken in may be subsequently performed. In this case, the nozzle 49 may not be discarded, and may be kept in the nozzle keeping part of the flow channel exchange unit 53. After the flow channel exchange unit 53 has discarded the nozzle 49, the processing proceeds to S180.

In S680, discarding of the nozzle 49 may be performed in the same procedure as S670. Upon discarding of the nozzle 49 by the flow channel exchange unit 53, the flow is completed.

In the above-described flow, examples of the manipulation on the manipulation target 35 are described to be removal of an unnecessary cell, recovery of a cytoplasm and/or a cell membrane, recovery and subculture of a cell, retention of a cell, and compression of a cell. The examples of the manipulation can include some manipulations other than those described above.

One example of the manipulation can be applying a culture substrate or a medical agent to a solid phase on a bottom surface of the vessel 25, and evaluating adhesiveness of a cell to such culture substrate or medical agent. Since adhesiveness to a cell can be evaluated by using an inner pressure of an air bubble, a moving speed of a nozzle, and a load when separating the cell as indexes, effectiveness of the culture substrate or the medical agent on the cell adhesion can be evaluated.

Another example of the manipulation can be sorting out of a cell. According to the flow described above, compression is performed on a cell with an air bubble. The constituent element or the physical property in a cell membrane or a cell is considered to be different depending on the type of the cell. Therefore, when the air bubble forming unit 200 controls the nozzle actuator 40 and/or the pressure generating unit 47, a shape variation process of the cell and a shape of the cell are considered to be different after starting, stopping, or releasing compression on the cell with the air bubble. In addition, it is considered that a cell that ruptures by being compressed may appear depending on the type of the cell. By using these as the indexes, the cell can be sorted out.

Another example of the manipulation can be observing a shape variation in the process of compressing a cell. According to the flow described above, compression is performed on a cell with an air bubble. For example, in the process of compressing the cell, the shape variation of the cell may be imaged when compressing the entire cell or various parts of the cell while changing the pressure to compress the cell.

Another example of the manipulation can be rupture or cutoff of a cell with compression. A cell can be ruptured or cut off by applying a large pressure on the cell. By rupturing or cutting off the cell, a cell membrane, a cytoplasm, and/or an organelle, etc. can be recovered, and connection between cells (for example, a synapse which is connection between nerve cells) can be cut.

Another example of the manipulation can be induction of cell differentiation. Osteoblastic cells, muscle cells, vascular endothelial progenitor cells, and the like are known to be cells in which its differentiation is induced by giving a mechanical stimulation. With the pressure generating unit 47 performing compression on these cells with an air bubble according to the flow described above, the differentiation can be induced.

Another example of the manipulation can be gene transfer to a cell. With the air bubble forming unit 200 using an air bubble to attach a vesicle-like object such as a cell membrane to the gas-liquid interface 255, and bringing it into contact with a cell membrane, according to the flow described above, a content of the vesicle is taken in the cell due to membrane fusion. At this time, by including a gene in the vesicle, the gene can be taken in the cell. In addition, other high polymers can also be taken in the cell through a pore, not limited to genes. Furthermore, when the air bubble forming unit 200 uses an air bubble to compress a cell on the gas-liquid interface 255 according to the flow described above, a tiny gap is readily generated in a part of a membrane in the process of cell deformation. At this time, by adding a gene in a medium of the cell, the gene can be taken in the cell through the gap. In addition, other high polymers can also be taken in the cell through the gap, not limited to genes.

Another example of the manipulation can be cooperation with an external apparatus, for example, a cell culture apparatus such as a fermenter or a cell analysis apparatus such as a cell sorter. With the air bubble forming unit 200 using an air bubble to take a cell in the flow channel 51, and releasing the cell to a designated position of a cooperating external apparatus, according to the flow described above, the cell may be moved. In addition, with the flow channel 51 being directly connected to the external apparatus, the cell taken in the flow channel 51 may be sent to the external apparatus, thereby moving the cell.

Another example of the manipulation can be a manipulation of an emulsion. An emulsion is a liquid droplet in oil liquid, or an oil droplet in an aqueous solution. To stabilize an emulsion that is formed, a surfactant that is an amphiphilic substance or the like may be included in the emulsion. The surfactant or the like is arranged so as to surround a liquid droplet or an oil droplet, and it forms a monolayer on an interface. Such monolayer is also seen in a part of an intracellular organelle such as an endosome or a lipid droplet. According to the flow described above, the air bubble forming unit 200 may use an air bubble to attach the emulsion to the gas-liquid interface 255, and may further perform a manipulation.

Methods of separating and/or recovering a cell include a method of separating a cell by locally denaturing a substrate by using a special substrate that reacts to a temperature or a light, and a method of separating a cell with ultrasonic waves, but these methods require a means of recovering the cell. However, the method of the present invention does not require a special substrate, and it includes both the means of separating a cell and the means of recovering the cell. In addition, the means of recovering a separated cell includes a method of recovering the cell by a liquid current that suctions liquid such as an aspirator, but there is a risk of recovering the cell and a large quantity of liquid at the same time, or involving a cell other than the target cell. However, according to the method of the present invention, a cell that is attached to a gas-liquid interface can be recovered by taking the gas-liquid interface in a nozzle, and it is possible to easily recover the target cell in a very little liquid quantity without involving a cell other than the target cell. In addition, although it has been known that an adverse effect is exerted on the cell by giving a strong liquid current at the time of suction of the liquid, it is possible to avoid such adverse effect by using the method of the present invention.

A method of separating the manipulation target 35 from the inner bottom surface of the vessel 25 in the method of recovering the manipulation target 35 illustrated in FIG. 4 will be described in detail below. In the present embodiment, by controlling an airflow in the air bubble 256 to which the manipulation target 35 is attached, the manipulation target 35 is moved along the airflow in the air bubble 256, and the manipulation target 35 is manipulated. In this manner, for example, by separating the manipulation target 35 from the inner bottom surface of the vessel 25, or by moving the manipulation target 35 in a separated state away from the bottom surface, the manipulation target 35 can be easily recovered.

Note that, by performing the method of the present embodiment, not only the manipulation target 35 is separated and recovered from the inner bottom surface of the vessel 25, but it is possible to perform other manipulations such as, for example, moving the manipulation target 35 to a predetermined location of an air bubble (for example, the center portion) in advance before compressing the manipulation target 35 from the top portion for observation, or moving the manipulation target 35 in the lateral direction of the air bubble to enable the use of a gas-liquid interface portion where the manipulation target 35 was attached for a manipulation of another manipulation target.

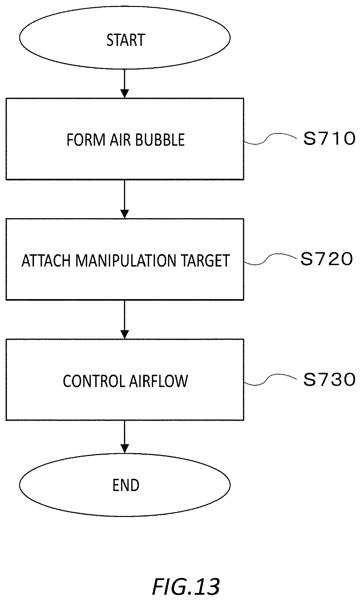

FIG. 13 is an example of a flow of controlling an airflow in the air bubble 256 to which the manipulation target 35 is attached in the present embodiment. In the present embodiment, by performing the processing from S710 to S730 in this order, the manipulation target 35 is moved along an airflow, and the manipulation target 35 is manipulated. However, the processing from S710 to S730 may not be performed in this order. For example, the processing of S710 and the processing of S720 may be performed at the same time, or the processing of S720 and S730 may be performed at the same time, or the processing of S710 and the processing of S730 may be performed at the same time, or the processing from S710 to S730 may be performed at the same time. Note that, the control on the airflow illustrated in FIG. 13 may be performed in the step of attaching a cell, a cytoplasm, or a cell membrane to the air bubble 256 and/or separating them from the air bubble 256 represented by S422, S432, and S442 in FIG. 11A, or may be performed in the step of taking them in the nozzle 49 represented by S435 in FIG. 11A and S500 in FIG. 6, or may be performed in the step of compressing the cell represented by S452 in FIG. 11A, or may be performed in the step of observing the cell represented by S444 and S454, or may be performed in the step of other cell manipulations represented by S460 in FIG. 11A.

First, in S710, the liquid 261 and the manipulation target 35 are contained in the vessel 25, and the end part 254 of the nozzle 49 is arranged in the liquid 261 to introduce gas into the flow channel 51 of the nozzle 49 with a pump, thereby forming the air bubble 256 in the flow channel 51 of the nozzle 49 or on the end part 254 of the nozzle 49. By forming the air bubble 256, the gas-liquid interface 255 is formed between the liquid 261 and the gas. The step of forming the air bubble 256 in S710 includes maintaining the air bubble 256 formed on the end part 254 of the flow channel 51 of the nozzle 49. The step of forming the air bubble 256 in S710 can be performed by the method shown in the above-described descriptions for FIG. 2B or FIG. 4.

Then, in S720, the manipulation target 35 is attached to the gas-liquid interface 255 of the air bubble 256. In the present embodiment, the air bubble forming unit 200 controls the nozzle actuator 40 to move the flow channel 51 of the nozzle 49, or controls the pressure generating unit 47 to change an inner pressure and/or a volume of an air bubble, thereby moving the gas-liquid interface 255 formed on the end part 254 of the nozzle 49, and bringing the gas-liquid interface 255 into contact with the manipulation target 35 adhered on the bottom portion of the vessel 25. In this manner, the manipulation target 35 adhered on the solid phase is attached to or/and separated from the gas-liquid interface 255 of the air bubble 256. Note that, the gas-liquid interface 255 of the air bubble 256 to which the manipulation target 35 is attached may not be a complete spherical surface.

Then, in S730, an airflow is generated in the air bubble 256, and a position of the manipulation target 35 is manipulated by controlling the airflow. In the present embodiment, the air bubble forming unit 200 functions as an air bubble control unit which generates an airflow in an air bubble formed by introducing gas into the liquid 261 from the end part 254 of the nozzle 49 and manipulating a position of the manipulation target 35 with the airflow. Note that, the step of controlling the airflow in S730 may include maintaining the air bubble 256 formed on the end part 254 of the flow channel 51 of the nozzle 49. In this manner, the control on the airflow in the air bubble 256 becomes easy, and a position control on the attached manipulation target 35 becomes more certain.

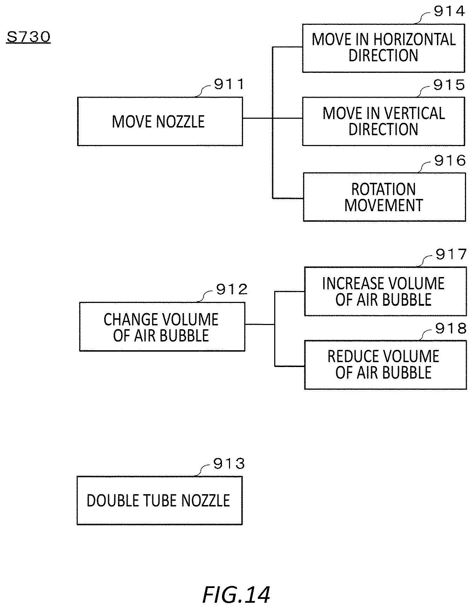

FIG. 14 illustrates an example of a method of generating an airflow in the air bubble 256 and manipulating a position of the manipulation target 35 with the airflow in the present embodiment. As illustrated in FIG. 14, the method of generating an airflow in the air bubble 256 and manipulating a position of the manipulation target 35 with the airflow includes a method 911 of generating an airflow with a movement of the nozzle 49, a method 912 of generating an airflow by changing a volume of the air bubble 256, and a method 913 of generating an airflow by using a double tube nozzle. These methods 911 to 913 can be used in combinations.

The method 911 of generating an airflow with a movement of the nozzle 49 is a method of generating an airflow in the air bubble 256 by moving a relative position between the flow channel 51 of the nozzle 49 and the liquid 261. The method 911 of generating an airflow with a movement of the nozzle 49 includes a method 914 of causing a parallel movement of the nozzle 49 in a direction parallel to a bottom portion 25a of the vessel 25 (the horizontal direction), a method 915 of causing a parallel movement of the nozzle 49 in a direction perpendicular to the bottom portion 25a of the vessel 25 (the vertical direction), and a method 916 of rotationally moving the nozzle 49. These methods 914 to 916 can be used in combinations. Note that, the movement of the relative position between the flow channel 51 and the liquid 261 may include moving a relative position between the flow channel 51 and the manipulation target 35.

In the present embodiment, the air bubble forming unit 200 functions as a flow channel position control unit which moves the flow channel 51 of the nozzle 49 by controlling the nozzle actuator 40, thereby controlling the airflow in the air bubble 256. Note that, the air bubble forming unit 200 may function as a stage position control unit which controls an actuator for moving a stage that is equipped with the vessel 25 containing the manipulation target 35, thereby controlling the airflow in the air bubble 256.

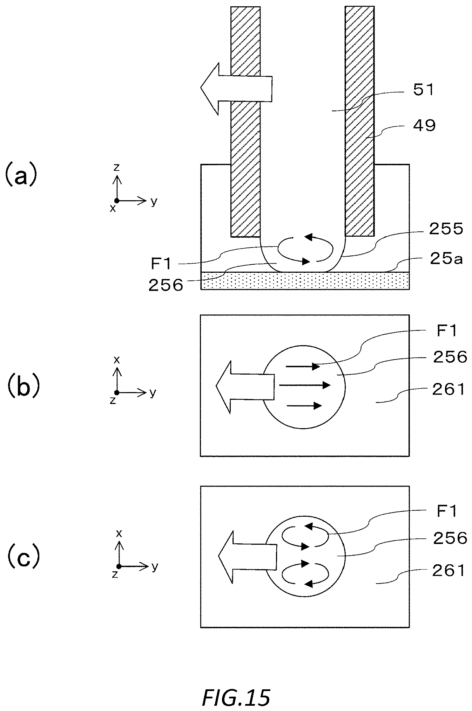

FIG. 15 illustrates an example of a schematic view describing the method 914 of moving the nozzle 49 in the horizontal direction in the present embodiment. FIG. 15 illustrates an example of moving the nozzle 49 in the leftward direction (the -Y direction). Note that, not limited to the complete horizontal direction (gravitational direction and perpendicular direction), a relative position between the flow channel 51 of the nozzle 49 and the bottom portion 25a of the vessel 25 may be moved in a direction within a range of ±20° from the horizontal direction (the ± Y direction). Note that, also when the nozzle 49 is obliquely installed, the relative position may be moved by using the horizontal direction as a reference. In addition, a moving speed of the nozzle 49 in the horizontal direction is desirably a speed of 50 to 500 µm/ second.

FIG. 15(a) illustrates a cross-sectional view of the nozzle 49 and the air bubble 256 in the length direction of the nozzle 49 (the Z direction), FIG. 15(b) illustrates a cross-sectional view of an edge portion (a portion near the bottom portion 25a) of the air bubble 256 in a direction parallel to the bottom portion 25a of the vessel 25 (the XY direction), and FIG. 15(c) illustrates a cross-sectional view of a middle portion of the air bubble 256 in a direction parallel to the bottom portion 25a of the vessel 25 (the XY direction). In addition, in FIG. 15(a) to (c), an arrow F1 illustrated inside the air bubble 256 shows a flow of an airflow generated inside the air bubble 256.

As illustrated in FIG. 15(a), when the nozzle 49 is moved in the leftward direction, a counterclockwise airflow F1 is generated inside the air bubble 256 formed on the end part 254 of the nozzle 49. This airflow F1 is generated by a convection flow that is caused by frictional force due to a movement of gas inside the air bubble 256 while contacting the bottom portion 25a of the vessel 25.

As illustrated in FIG. 15(b), when seen from the + Z direction, this airflow F1 is generated in an edge portion (a portion near the bottom portion 25a) of the air bubble 256, in the + Y direction which is the direction opposite to the moving direction of the nozzle 49 (the - Y direction). Since an air bubble is a closed system, as illustrated in FIG. 15(a), this airflow F1 is generated in the + Z direction which is the upward direction on the back side of the moving direction of the air bubble 256 (the - Y direction), while being generated in the - Z direction which is the downward direction on the front side of the moving direction of the air bubble 256 (the - Y direction), and is rotating. Similarly, as illustrated in FIG. 15(c), since an influence of frictional force of a bottom surface becomes little at a position away from the bottom surface, this airflow F1 makes clockwise and counterclockwise symmetric circles in a middle portion of the air bubble 256.

Note that, FIG. 15(a) and (b) illustrate the example of moving the nozzle 49 in the horizontal direction in a state where the edge portion of the air bubble 256 is in contact with the bottom portion 25a of the vessel 25, but the nozzle 49 may be moved in the horizontal direction in a state where the edge portion of the air bubble 256 is not in contact with the bottom portion 25a of the vessel 25.

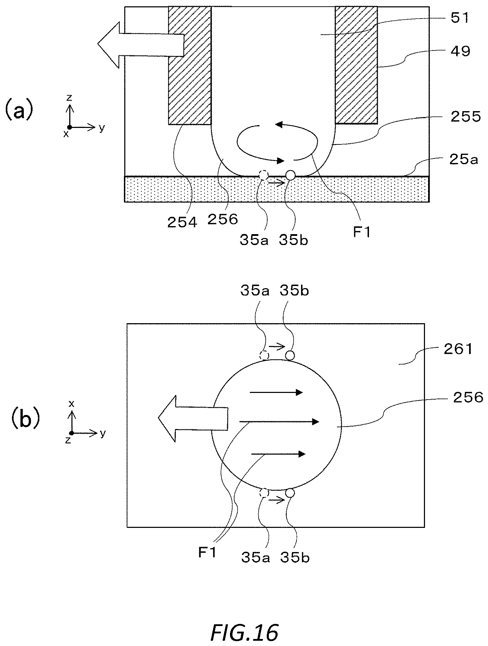

FIG. 16 illustrates an example of a schematic view describing a moving direction of the manipulation target 35 when the nozzle 49 is moved in the horizontal direction in the present embodiment. FIG. 16(a) illustrates a partially enlarged view near the air bubble 256 in FIG. 15(a), and FIG. 16(b) is a drawing similar to FIG. 15(b). FIG. 16(a) and (b) illustrate the manipulation target 35 attached to the gas-liquid interface 255. In FIG. 16(a) and (b), the manipulation target 35 before movement is shown with a circle 35a in a broken line, and the manipulation target 35 after movement is shown with a circle 35b in a solid line.

As illustrated in FIG. 16(a) and (b), when the nozzle 49 is moved in the leftward direction, the airflow F1 is generated in the + Y direction which is opposite to the moving direction of the nozzle 49 in an edge portion of the air bubble 256 (a portion near the bottom portion 25a), and thus the manipulation target 35 moves to a position 35b in the + Y direction due to an influence of the airflow F1.

As described above, by moving the nozzle 49 in the leftward direction in FIG. 15 and FIG. 16, the airflow F1 in the direction opposite to the moving direction of the nozzle 49 is generated in the edge portion of the air bubble 256, and the manipulation target 35 attached to the edge portion of the air bubble 256 can be moved in the direction of the airflow F1. Note that, when the nozzle 49 is moved in the rightward direction in FIG. 15 and FIG. 16, the direction of the airflow F1 generated inside the air bubble 256 and the moving direction of the manipulation target 35 attached to the edge portion of the air bubble 256 become directions opposite to the directions illustrated in FIG. 15 and FIG. 16.

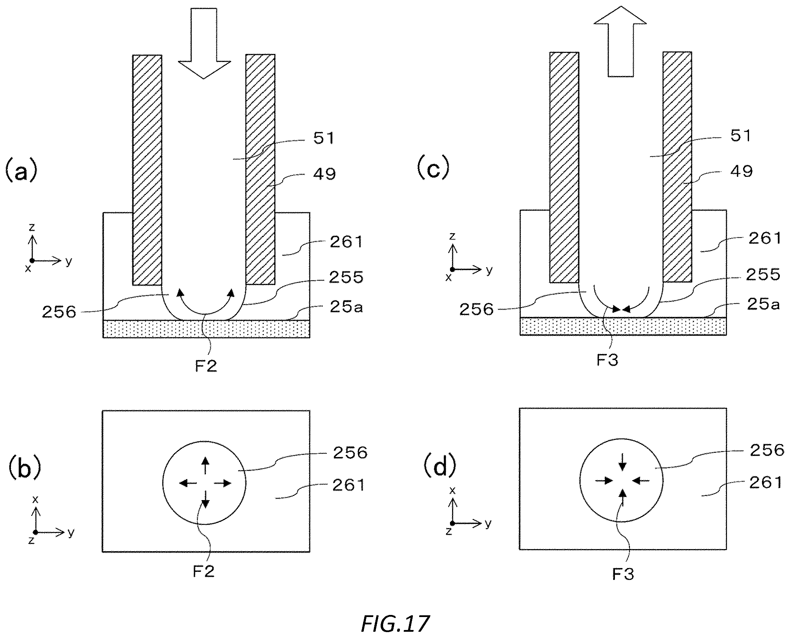

FIG. 17 illustrates an example of a schematic view describing the method 915 of moving the nozzle 49 in the vertical direction in the present embodiment. FIG. 17(a) and (b) illustrate the example of moving the nozzle 49 in the downward direction (the - Z direction), and FIG. 17(c) and (d) illustrate the example of moving the nozzle 49 in the upward direction (the + Z direction). Note that, not limited to the vertical direction (the direction along the gravitational direction), a relative position between the flow channel 51 of the nozzle 49 and the bottom portion 25a of the vessel 25 may be moved in a direction within a range of ± 20° from the vertical direction (the ± Z direction). Note that, also when the nozzle 49 is obliquely installed, the relative position may be moved by using the vertical direction as a reference. In addition, a moving speed of the nozzle 49 in the vertical direction is desirably a speed of 10 to 100 µm/ second.

FIG. 17(a) and (c) illustrate cross-sectional views of the nozzle 49 and the air bubble 256 in the length direction of the nozzle 49 (the Z direction), and FIG. 17(b) and (d) illustrate cross-sectional views of an edge portion (a portion near the bottom portion 25a) of the air bubble 256 in a direction parallel to the bottom portion 25a of the vessel 25 (the XY direction). In addition, in FIG. 17(a) to (d), arrows F2 and F3 illustrated inside the air bubble 256 show flows of an airflow generated inside the air bubble 256.

Note that, FIG. 17(a) to (d) illustrate the example of moving the nozzle 49 in the vertical direction in a state where the edge portion of the air bubble 256 is in contact with the bottom portion 25a of the vessel 25, but the nozzle 49 may be moved in the vertical direction in a state where the edge portion of the air bubble 256 is not in contact with the bottom portion 25a of the vessel 25. Note that, the bottom portion 25a of the vessel 25 may be a surface of a culture substrate.

As illustrated in FIG. 17(a), when the nozzle 49 is moved in the downward direction, the airflow F2 that rises along the gas-liquid interface 255 is generated inside the air bubble 256 formed on the end part 254 of the nozzle 49. As illustrated in FIG. 17(b), when seen from the + Z direction, the airflow F2 is in a direction toward a radially outward of the air bubble 256. This airflow F2 is generated mainly due to the air bubble 256 being crushed to the outside. Note that, also when the nozzle 49 is moved in a state where the edge portion of the air bubble 256 is not in contact with the bottom portion 25a of the vessel 25, an airflow in the same direction as the airflow F2 is generated due to a shape deformation of the air bubble caused by resistance of liquid, or generation of a convection flow caused by frictional force with the liquid.

As illustrated in FIG. 17(c), when the nozzle 49 is moved in the upward direction, an airflow F3 descending along the gas-liquid interface 255 is generated inside the air bubble 256 formed on the end part 254 of the nozzle 49. As illustrated in FIG. 17(d), when seen from the + Z direction, the airflow F3 is in a direction toward a radially inward of the air bubble 256. This airflow F3 is generated mainly due to a crushed portion of the air bubble 256 trying to go back to an original shape. Note that, also when the nozzle 49 is moved in a state where the edge portion of the air bubble 256 is not in contact with the bottom portion 25a of the vessel 25, an airflow in the same direction as the airflow F3 is generated due to a shape deformation of the air bubble caused by resistance of liquid, or generation of a convection flow caused by frictional force with the liquid.

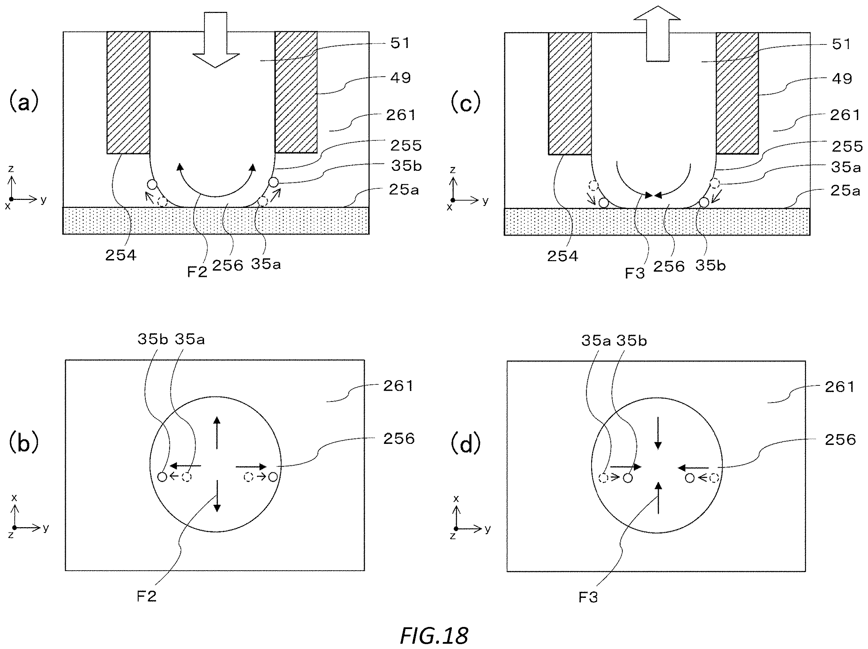

FIG. 18 illustrates an example of a schematic view describing a moving direction of the manipulation target 35 when the nozzle 49 is moved in the vertical direction in the present embodiment. FIG. 18(a) and (c) illustrate partially enlarged views near the air bubble 256 in FIG. 17(a) and (c), and FIG. 18(b) and (d) are drawings similar to FIG. 17(b) and (d). FIG. 18(a) and (b) illustrate the manipulation target 35 attached to the gas-liquid interface 255. In FIG. 18(a) and (b), the manipulation target 35 before movement is shown with the circle 35a in a broken line, and the manipulation target 35 after movement is shown with the circle 35b in a solid line.

As illustrated in FIG. 18(a) and (b), when the nozzle 49 is moved in the downward direction, since the airflow F2 rising along the gas-liquid interface 255 is generated inside the air bubble 256, the manipulation target 35 moves to the position 35b in the + Z direction that is radially outward of the air bubble 256 due to an influence of the airflow F2. As illustrated in FIG. 18(c) and (d), when the nozzle 49 is moved in the upward direction, since the airflow F3 descending along the gas-liquid interface 255 is generated inside the air bubble 256, the manipulation target 35 moves to the position 35b in the - Z direction that is radially inward of the air bubble 256 due to the influence of the airflow F2.

As described above, by moving the nozzle 49 in the vertical direction, the airflow F2 in a radially outward direction or the airflow F3 in a radially inward direction is generated inside the air bubble 256, and the manipulation target 35 attached to the gas-liquid interface 255 can be moved in the radially outward direction or the radially inward direction.

In addition, an airflow can be generated inside the air bubble 256 also with the method 916 of rotationally moving the nozzle 49. For example, when the nozzle 49 is rotationally moved in a clockwise direction as seen from the + Z direction, an airflow in a counterclockwise direction as seen from the + Z direction is generated in the flow channel 51 of the nozzle 49, and an airflow in a counterclockwise direction is similarly generated also inside the air bubble 256. With this airflow, the manipulation target 35 attached to the gas-liquid interface 255 of the air bubble 256 can be moved in the direction of the airflow.

The above-described method 911 of generating an airflow with a movement of the nozzle 49 may include other moving forms. For example, the nozzle 49 may be vibrated for a particular number of vibrations in the horizontal direction or the vertical direction to generate an airflow, or the nozzle 49 may be caused to circulate around a particular rotation center, or the nozzle 49 may be moved in an oblique direction with respect to the bottom portion 25a of the vessel 25, or the nozzle 49 may be inclined by a particular angle, or the nozzle 49 may be caused to make a pendulum motion. Furthermore, this plurality of moving forms may be used in combinations.

Returning to FIG. 14, the method 912 of generating an airflow by changing a volume of the air bubble 256 includes a method 917 of increasing a volume of the air bubble 256 and a method 918 of reducing a volume of the air bubble 256.

A change in the volume of the air bubble 256 is performed with the volume control unit, the gas supply control unit, and the intake control unit. When increasing the volume of the air bubble 256, the volume control unit or the gas supply control unit controls a pump of the pressure generating unit 47 connected to the gas supply flow channel, thereby controlling the volume of gas to be supplied to the gas supply flow channel. The volume control unit or the gas supply control unit receives information on an amount of the gas to be supplied to the gas supply flow channel from the nozzle actuator 40, the pressure generating unit 47, or the sensor unit 48. In addition, a pressurization acceleration of the air bubble 256 is desirably 0.01 to 10 kPa/ second.

When reducing the volume of the air bubble 256, the volume control unit or the intake control unit controls the pump of the pressure generating unit 47 connected to the gas recovery flow channel, thereby controlling the volume of gas to be taken in from the gas recovery flow channel. The volume control unit or the intake control unit receives information on an amount of the gas to be taken in from the gas recovery flow channel, from the nozzle actuator 40, the pressure generating unit 47, or the sensor unit 48. In addition, a depressurization acceleration of the air bubble 256 is desirably 0.005 to 5 kPa/ second. Note that, the same pump may be connected to the gas supply flow channel and the gas recovery flow channel, or different pumps may be connected thereto.

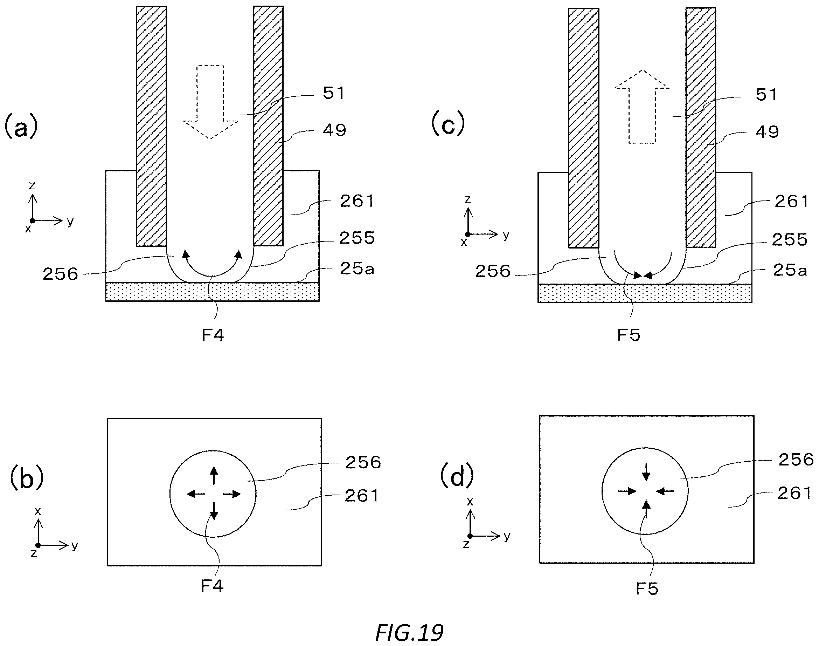

FIG. 19 illustrates an example of a schematic view for describing the method 917 of increasing a volume of the air bubble 256 and the method 918 of reducing a volume of the air bubble 256 in the present embodiment. FIG. 19(a) and (b) illustrate examples of increasing a volume of the air bubble 256, and FIG. 19(c) and (d) illustrate examples of reducing a volume of the air bubble 256.

FIG. 19(a) and (c) illustrate cross-sectional views of the nozzle 49 and the air bubble 256 in the length direction of the nozzle 49 (the Z direction), and FIG. 19(b) and (d) illustrate cross-sectional views of an edge portion (a portion near the bottom portion 25a) of the air bubble 256 in a direction parallel to the bottom portion 25a of the vessel 25 (the XY direction). In addition, in FIG. 19(a) to (d), arrows F4 and F5 illustrated inside the air bubble 256 show flows of an airflow generated inside the air bubble 256.

Note that, FIG. 19(a) to (d) illustrate the example of changing a volume of the air bubble 256 in a state where the edge portion of the air bubble 256 is in contact with the bottom portion 25a of the vessel 25, but the volume of the air bubble 256 may be changed in a state where the edge portion of the air bubble 256 is not in contact with the bottom portion 25a of the vessel 25.

As illustrated in FIG. 19(a), when increasing the volume of the air bubble 256, the airflow F4 rising along the gas-liquid interface 255 is generated inside the air bubble 256 formed on the end part 254 of the nozzle 49. As illustrated in FIG. 19(b), when seen from the + Z direction, the airflow F4 is in a direction toward a radially outward of the air bubble 256. This airflow F4 is generated by gas in a surrounding of the gas-liquid interface 255 being pushed back to the upward direction along the gas-liquid interface 255, due to descending of gas supplied from the pump through a center portion of the flow channel 51.

As illustrated in FIG. 19(c), when reducing the volume of the air bubble 256, the airflow F5 descending along the gas-liquid interface 255 is generated inside the air bubble 256 formed on the end part 254 of the nozzle 49. As illustrated in FIG. 19(d), when seen from the + Z direction, the airflow F5 is in a direction toward a radially inward of the air bubble 256. This airflow F5 is generated by gas in a surrounding of the gas-liquid interface 255 being brought back to the downward direction along the gas-liquid interface 255, due to rise of gas taken in the pump rising through a center portion of the flow channel 51.

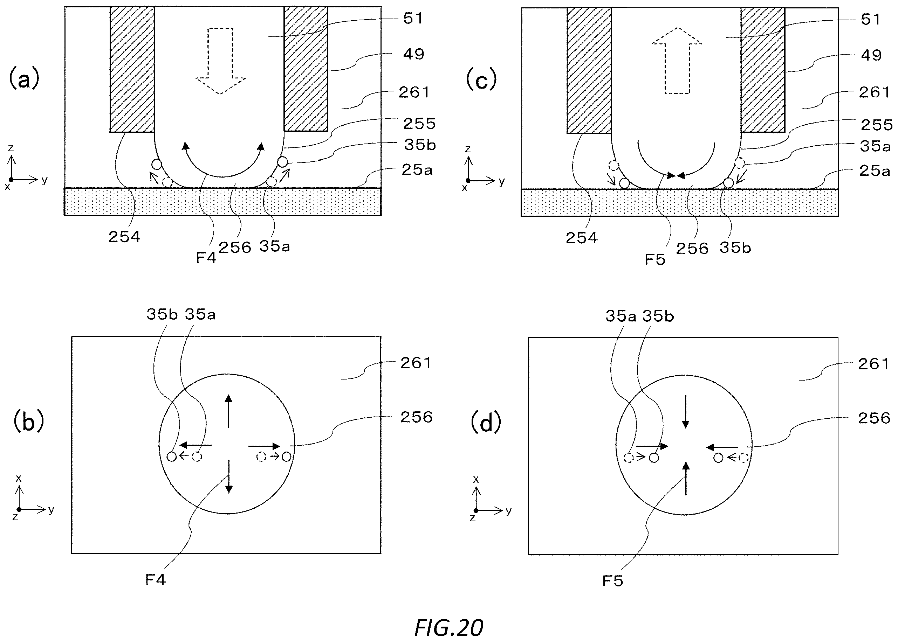

FIG. 20 illustrates an example of a schematic view describing a moving direction of the manipulation target 35 when a volume of the air bubble 256 is changed in the present embodiment. FIG. 20(a) and (c) illustrate partially enlarged views near the air bubble 256 in FIG. 19(a) and (c), and FIG. 20(b) and (d) are drawings similar to FIG. 19(b) and (d). FIG. 20(a) and (b) illustrate the manipulation target 35 attached to the gas-liquid interface 255. In FIG. 20(a) and (b), the manipulation target 35 before movement is shown with the circle 35a in a broken line, and the manipulation target 35 after movement is shown with the circle 35b in a solid line.

As illustrated in FIG. 20(a) and (b), when increasing the volume of the air bubble 256, since the airflow F4 rising along the gas-liquid interface 255 is generated inside the air bubble 256, the manipulation target 35 moves to the position 35b in the + Z direction that is radially outward of the air bubble 256 due to an influence of the airflow F4. As illustrated in FIG. 20(c) and (d), when reducing the volume of the air bubble 256, since the airflow F5 descending along the gas-liquid interface 255 is generated inside the air bubble 256, the manipulation target 35 moves to the position 35b in the - Z direction that is radially inward of the air bubble 256 due to the influence of the airflow F5.

As described above, by changing the volume of the air bubble 256, the airflow F4 in a radially outward direction or the airflow F5 in a radially inward direction is generated inside the air bubble 256, and the manipulation target 35 attached to the gas-liquid interface 255 can be moved in the radially outward direction or the radially inward direction.

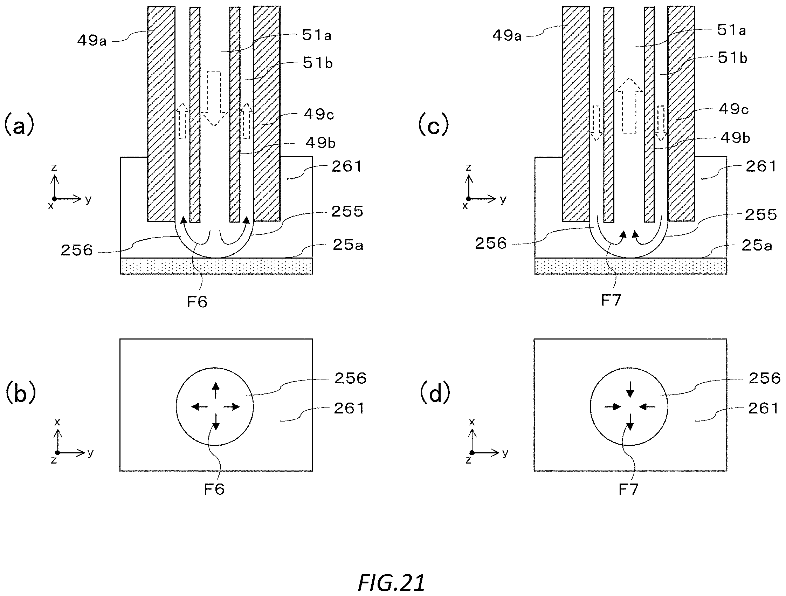

FIG. 21 illustrates an example of a schematic view describing the method 913 of generating an airflow by using a double tube nozzle 49a in the present embodiment. In the present embodiment, the double tube nozzle 49a has a double tube structure having an inner tube 49b and an outer tube 49c. The double tube nozzle 49a has the flow channel 51a formed on an inside of the inner tube 49b, and the flow channel 51b formed between the inner tube 49b and the outer tube 49c.

In the present embodiment, a control to circulate gas in the double tube nozzle 49a by using the flow channel 51a and the flow channel 51b is performed. The flow channel 51a and the flow channel 51b function as the gas supply flow channel for supplying gas, or the gas recovery flow channel for recovering the gas. FIG. 21(a) and (b) illustrate a control example of supplying gas from the flow channel 51a, and recovering the gas with the flow channel 51b, and FIG. 21(c) and (d) illustrate a control example of supplying gas from the flow channel 51b, and recovering the gas with the flow channel 51a.

The control to supply gas to the flow channel 51a or the flow channel 51b is performed with the volume control unit or the gas supply control unit. The volume control unit or the gas supply control unit controls the pump of the pressure generating unit 47 connected to the flow channel 51a or the flow channel 51b that is the gas supply flow channel, thereby controlling a volume of gas to be supplied to the flow channel 51a or the flow channel 51b. The control to recover the gas from the flow channel 51a or the flow channel 51b is performed with the volume control unit or the intake control unit. The volume control unit or the intake control unit controls the pump of the pressure generating unit 47 connected to the flow channel 51a or the flow channel 51b that is the gas recovery flow channel, thereby controlling a volume of gas to be taken in from the flow channel 51a or the flow channel 51b.

FIG. 21(a) and (c) illustrate cross-sectional views of the nozzle 49 and the air bubble 256 in the length direction of the nozzle 49 (the Z direction), and FIG. 21(b) and (d) illustrate cross-sectional views of an edge portion (a portion near the bottom portion 25a) of the air bubble 256 in a direction parallel to the bottom portion 25a of the vessel 25 (the XY direction). In addition, in FIG. 21(a) to (d), arrows F6 and F7 illustrated inside the air bubble 256 show flows of an airflow generated inside the air bubble 256.

Note that, FIG. 21(a) to (d) illustrate the example of supplying and recovering gas by using the flow channel 51a and the flow channel 51b in a state where the edge portion of the air bubble 256 is in contact with the bottom portion 25a of the vessel 25, but the gas may be supplied and recovered by using the flow channel 51a and the flow channel 51b in a state where the edge portion of the air bubble 256 is not in contact with the bottom portion 25a of the vessel 25.

As illustrated in FIG. 21(a), when performing a control to supply gas from the flow channel 51a, an airflow where gas supplied from an upper end portion of the flow channel 51a flows through the air bubble 256 toward an upper end portion of the flow channel 51b is generated. In this case, an airflow F6 rising along the gas-liquid interface 255 is generated inside the air bubble 256 formed on the end part 254 of the nozzle 49. As illustrated in FIG. 21(b), when seen from the + Z direction, the airflow F6 is in a direction toward a radially outward of the air bubble 256.

As illustrated in FIG. 21(c), when performing a control to supply gas from the flow channel 51b, an airflow where gas supplied from an upper end portion of the flow channel 51b flows through the air bubble 256 toward an upper end portion of the flow channel 51a is generated. In this case, an airflow F7 descending along the gas-liquid interface 255 is generated inside the air bubble 256 formed on the end part 254 of the nozzle 49. As illustrated in FIG. 21(d), when seen from the + Z direction, the airflow F7 is in a direction toward a radially inward of the air bubble 256.

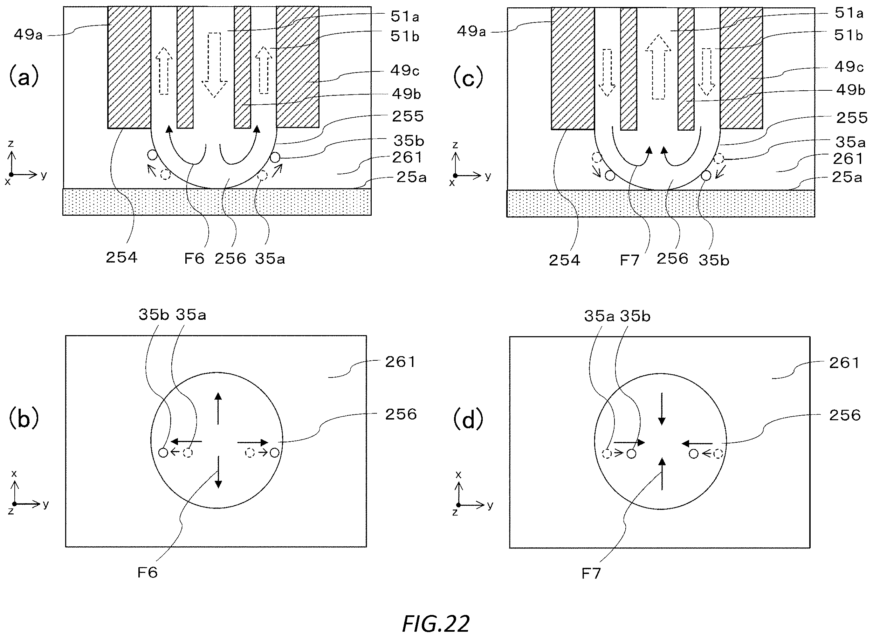

FIG. 22 illustrates an example of a schematic view describing a moving direction of the manipulation target 35 when the double tube nozzle 49a is used in the present embodiment. FIG.22(a) and (c) illustrate partially enlarged views near the air bubble 256 in FIG. 21(a) and (c), and FIG. 22(b) and (d) are drawings similar to FIG. 21(b) and (d). FIG. 22(a) and (b) illustrate the manipulation target 35 attached to the gas-liquid interface 255. In FIG. 22(a) and (b), the manipulation target 35 before movement is shown with the circle 35a in a broken line, and the manipulation target 35 after movement is shown with the circle 35b in a solid line.

As illustrated in FIG. 22(a) and (b), when performing a control to supply gas from the flow channel 51a, since the airflow F6 rising along the gas-liquid interface 255 is generated inside the air bubble 256, the manipulation target 35 moves to the position 35b in the + Z direction that is radially outward of the air bubble 256 due to an influence of the airflow F6. As illustrated in FIG. 22(c) and (d), when performing a control to supply gas from the flow channel 51b, since the airflow F7 descending along the gas-liquid interface 255 is generated inside the air bubble 256, the manipulation target 35 moves to the position 35b in the - Z direction that is radially inward of the air bubble 256 due to an influence of the airflow F7.

As described above, by performing a control to circulate gas in the double tube nozzle 49a, the airflow F6 in a radially outward direction or the airflow F7 in a radially inward direction is generated inside the air bubble 256, and the manipulation target 35 attached to the gas-liquid interface 255 can be moved in the radially outward direction or the radially inward direction.

As described above, by generating the airflows F1 to F7 in the air bubble 256, the position of the manipulation target 35 can be freely moved in the horizontal direction and/or the vertical direction. By moving the position of the manipulation target 35 in the horizontal direction and/or the vertical direction, the following effects can be exerted. For example, by moving the position of the manipulation target 35 in the upward direction, the manipulation target 35 will no longer contact the bottom portion 25a of the vessel 25, and there will be no friction when moving the manipulation target 35. Thus, for example, it is possible to improve manipulativeness at the time of attachment and/or separating of the manipulation target 35 in S422, S432, and S442 of FIG. 11A, and taking in of the manipulation target 35 to the nozzle 49 in S435 of FIG. 11A and S500 of FIG. 6. In addition, by moving the position of the manipulation target 35 in the upward direction, the gas-liquid interface 255 in an edge part of the air bubble 256 can be used as an attachment surface of the manipulation target 35 by gathering the manipulation targets 35 to the end part 254 of the nozzle 49. By moving the position of the manipulation target 35 in the upward direction, the manipulation targets 35 bump into each other by moving the manipulation target 35 to an edge of the nozzle 49 of the air bubble 256, and the manipulation target 35 can be removed from the gas-liquid interface 255. Accordingly, at the time of release of the manipulation targets 35 in S645 of FIG. 6, a release success rate of the manipulation targets 35 is improved.

By moving the position of the manipulation target 35 in the downward direction, compression of the manipulation target 35 can be made easy by moving the manipulation target 35 to an edge of the air bubble 256, and for example, cell compression and/or observation in S452 and S454 of FIG. 11A can be made easy. By moving the position of the manipulation target 35 in the downward direction, at the time of observation of the manipulation target 35 in S444, an observation of the manipulation target 35 can be made easy by moving the manipulation target 35 to an edge of the air bubble 256. By moving the position of the manipulation target 35 in the downward direction, the manipulation target 35 can be brought into contact with the bottom portion 25a of the vessel 25 by moving the manipulation target 35 to an edge of the air bubble 256. By moving the position of the manipulation target 35 in the downward direction, the manipulation target 35 is less likely to contact a wall surface of the vessel 25 or an inner wall of the flow channel 51 at the time of recovery by moving the manipulation target 35 to an edge of the air bubble 256. Accordingly, at the time of taking in of the manipulation target 35 to the nozzle 49 in S435 of FIG. 11A and S500 of FIG. 6, a success rate of taking in the manipulation target 35 is improved. By moving the position of the manipulation target 35 in the downward direction, the manipulation targets 35 bump into each other by moving the manipulation target 35 to an edge of the air bubble 256, and the manipulation target 35 can be removed from the gas-liquid interface 255. Accordingly, at the time of release of the manipulation targets 35 in S645 of FIG. 6, a release success rate of the manipulation targets 35 is improved. By moving the position of the manipulation target 35 in the downward direction, the manipulation target 35 can be brought into contact with and/or pushed against other manipulation targets not attached to an air bubble existing in the liquid 261.

By moving the position of the manipulation target 35 in the horizontal direction, the manipulation targets 35 can be gathered to a partial region, and the gas-liquid interface 255 where the manipulation target 35 do not exist can be used for attachment of the manipulation target 35. Accordingly, at the time of attachment of the air bubble 256 to the manipulation target 35 in S422, S432, and S442 of FIG. 11A, a manipulation time can be shortened by efficiently performing the attachment. By moving the position of the manipulation target 35 in the horizontal direction, the manipulation targets 35 bump into each other by bringing the manipulation target 35 to a partial region, and the manipulation target 35 can be removed from the gas-liquid interface 255. Accordingly, at the time of release of the manipulation targets 35 in S645 of FIG. 6, a release success rate of the manipulation targets 35 is improved. By moving the position of the manipulation target 35 in the horizontal direction, the manipulation target 35 can be brought into contact with other manipulation targets not attached to an air bubble existing in the liquid 261.

Note that, in S720, the step of attaching the manipulation target 35 to the gas-liquid interface 255 of the air bubble 256 may include a control to move the gas-liquid interface 255 such that it contacts the manipulation target 35 that is particular. In this case, the air bubble forming unit 200 acquires a horizontal position (XY position) of the gas-liquid interface 255 and the manipulation target 35 to become a target from an image that is captured with the camera 60 or the camera 70, and acquires a vertical position (Z position) of the gas-liquid interface 255 and the particular manipulation target 35 to be the target from an image that is captured with the flow channel imaging camera 42, thereby identifying a relative positional relationship between the gas-liquid interface 255 and the particular manipulation target 35. In addition, the vertical position (Z position) may be acquired from information on a load, contact or proximity of the actuator, and information on an inner pressure of an air bubble, by using the sensor unit 48.

Subsequently, the air bubble forming unit 200 causes the nozzle actuator 40 to move the nozzle 49 and/or the stage based on an identified relative positional relationship, and brings the gas-liquid interface 255 into contact with the target manipulation target 35. In addition, with the manipulator inputting an operation amount of the nozzle 49 and/or the stage based on the identified relative positional relationship, the nozzle 49 and/or the stage may be moved to bring the gas-liquid interface 255 into contact with the particular manipulation target 35.

Furthermore, the step of attaching the manipulation target 35 to the gas-liquid interface 255 of the air bubble 256 in S720 may be performed while detecting a pressure of the air bubble 356. In this case, the sensor unit 48 detects a pressure generated in the air bubble 256, and the sensor unit 48 sends this information to the air bubble forming unit 200. The air bubble forming unit 200 may perform a feedback control of the pressure generating unit 47 based on the detected pressure.

In addition, in the step of attaching the manipulation target 35 to the gas-liquid interface 255 of the air bubble 256 in S720, the air bubble forming unit 200 may acquire an image of contact between the gas-liquid interface 255 and the manipulation target 35 with the camera 60, the camera 70, and the flow channel imaging camera 42, thereby understanding a contact situation between the gas-liquid interface 255 and the manipulation target 35.

Furthermore, after the step of controlling the airflow in S730, a step of taking the manipulation target 35 in the nozzle 49 from the liquid 261 may be further included. In this case, with the pump taking in gas in the air bubble 256 to which the manipulation target 35 is attached, the gas-liquid interface 225 is taken in the flow channel 51, and by taking the manipulation target 35 in the flow channel 51, the manipulation target 35 is taken in the nozzle 49 from the liquid 261. This recovery step is the same processing as the processing performed in the step of attaching a cell, a cytoplasm, or a cell membrane to the air bubble 256 and taking it in the nozzle 49, shown in S435 of FIG. 11A and S500 of FIG. 6.

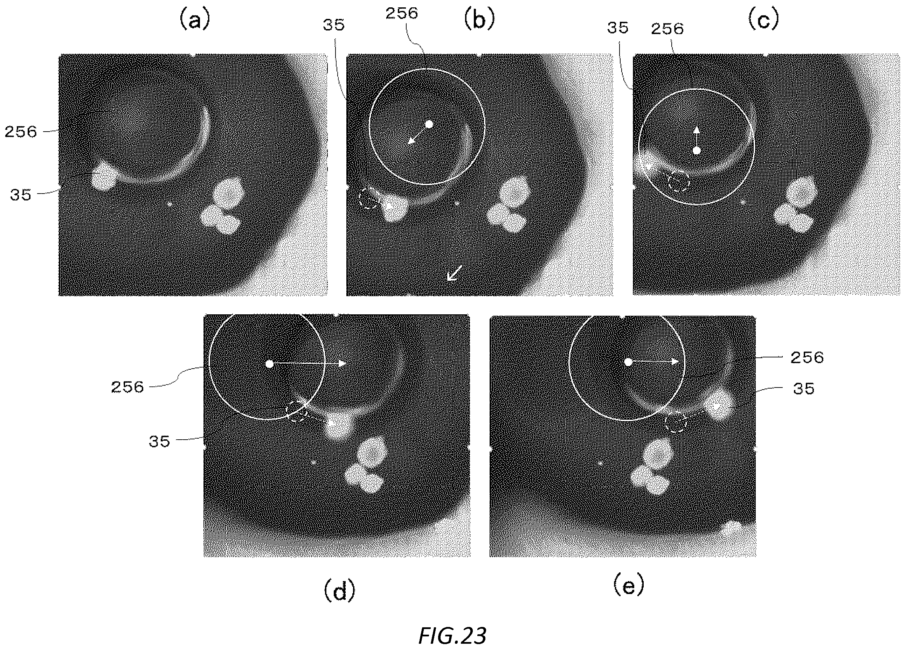

FIG. 23 is an experimental image illustrating a movement of the manipulation target 35 due to a movement of the air bubble 256 in the present embodiment. FIG. 15 to FIG. 22 above described the embodiments in which the manipulation target 35 moves due to an airflow in the air bubble 256, but the manipulation target 35 moves in liquid also with a movement of the air bubble 256 itself. The manipulation target 35 in FIG. 23 represents a spheroid created with a HT29 cell, and a white solid line and an arrow of the white solid line represent a moving direction and a distance of an air bubble from the original position, whereas a white broken line and an arrow of the white broken line represent a moving direction and a distance of the spheroid from the original position on the gas-liquid interface 255 of the air bubble 256. For example, when the air bubble 256 is moved from the state in which the manipulation target 35 is attached to the gas-liquid interface 255 of the air bubble 256 as illustrated in FIG. 23(a) to a lower left direction as illustrated in FIG. 23(b), the manipulation target 35 attached to a lower left part of the air bubble 256 moves along the gas-liquid interface 255 of the air bubble 256 in an opposite direction to the moving direction of the air bubble 256 as illustrated. At this time, the manipulation target 35 remains near the bottom portion 25a by receiving an influence of an airflow in the - Z direction generated on the front side of the moving direction of the air bubble 256 as illustrated in FIG. 15(a), and moves by receiving an influence of an airflow in an opposing direction (the + Y direction) of the moving direction of the air bubble 256 as illustrated FIG. 15(b). As illustrated in FIG. 23(c), when the air bubble 256 is moved in the upward direction, the manipulation target 35 attached to a lower left part of the air bubble 256 moves along the gas-liquid interface 255 of the air bubble 256, in the same direction as the moving direction of the air bubble 256 while rising so as to become away from the bottom portion 25a, as illustrated. At this time, the manipulation target 35 rises so as to become away from the bottom portion 25a by receiving an influence of an airflow in the + Z direction generated on the back side of the moving direction of the air bubble 256 as illustrated in FIG. 15(a), and moves by receiving an influence of an airflow in the same direction (the - Y direction) as the moving direction of the air bubble 256 generated in a middle portion of the air bubble 256 as illustrated in FIG. 15(c). With the manipulation target 35 becoming out of the focus, the manipulation target 35 can be confirmed as becoming away from the bottom portion 25a. Accordingly, the nozzle position control unit may perform a control to move the flow channel 51 in an opposing direction of an organism that is the manipulation target 35 with the central axis of the flow channel 51 being a reference, in a vertical directional view.

As illustrated in FIG. 23(d) and (e), when the air bubble 256 is moved in the rightward direction, the manipulation target 35 attached to a lower left part of the air bubble 256 moves in the rightward direction along the gas-liquid interface 255 of the air bubble 256, and moves in the same direction as the moving direction of the air bubble 256, as illustrated. At this time, since the manipulation target 35 rose so as to become away from the bottom portion 25a in the prior step, as illustrated in FIG. 15(c), the manipulation target 35 moves by receiving an influence of an airflow in the same direction (the - Y direction) as the moving direction of the air bubble 256 generated in a middle portion of the air bubble 256. Such movement of the manipulation target 35 due to a movement of the air bubble 256 can be combined with a movement of the manipulation target 35 due to an airflow in the air bubble 256.

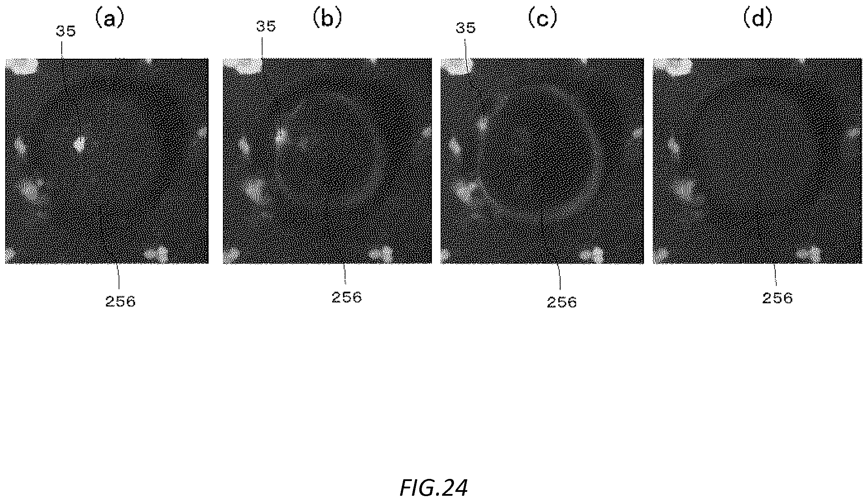

FIG. 24 is an experimental image illustrating a position control on the manipulation target 35 due to an enlargement of the air bubble 256 in the present embodiment. The manipulation target 35 in FIG. 24 represents an iPS cell-derived nerve cell. As illustrated in FIG. 24(a), the nozzle 49 is adjusted such that a vicinity of an outer side of an inside of the flow channel 51 of the nozzle 49 is positioned in a top portion of the manipulation target 35. At this time, the air bubble 256 is not formed, and the gas-liquid interface 255 of the air bubble 256 and the manipulation target 35 are not in contact with each other. As illustrated in FIG. 24(b), the air bubble 256 is enlarged, and the manipulation target 35 is attached to the gas-liquid interface 255 of the air bubble 256. At this time, as illustrated in FIG. 20(a) and (b), the manipulation target 35 rises so as to become away from the bottom portion 25a, and moves slightly to a top portion (becomes out of the focus), by receiving an influence of an airflow in the + Z direction generated in the air bubble 256. As illustrated in FIG. 24(c), when the air bubble 256 is further enlarged, the manipulation target 35 further moves to a top portion (becomes out of the focus), and a network connecting nerve cells which are the manipulation target 35 is cut. Thus, as illustrated in FIG. 24(d), by taking the air bubble 256 in the flow channel 51, the manipulation target 35 can be taken inside the nozzle 49.

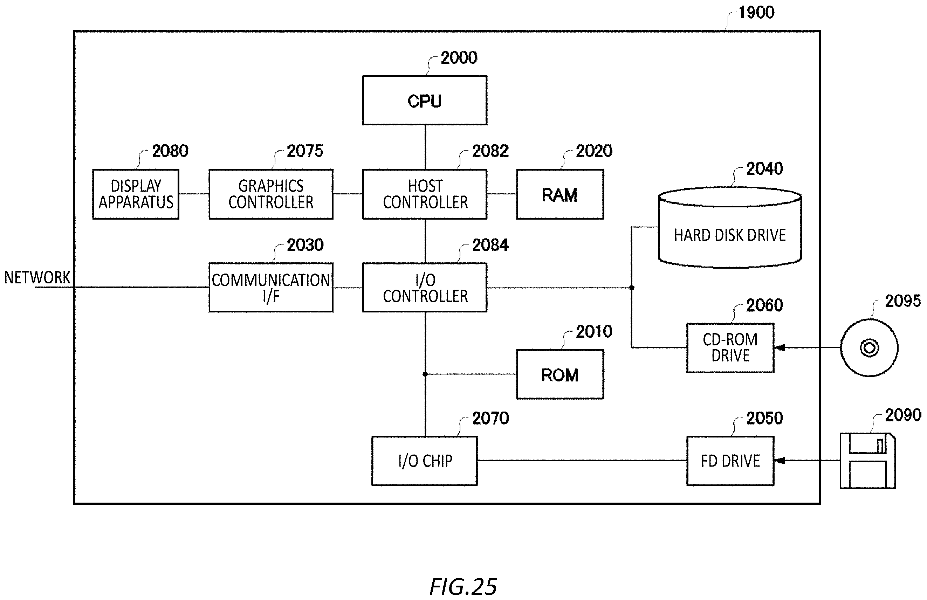

Fig. 25 illustrates an example of a hardware configuration of a computer 1900 functioning as the information processing device 170. The computer 1900 according to the present embodiment includes: a CPU-peripheral portion having a CPU 2000, a RAM 2020, a graphics controller 2075, and a display apparatus 2080 interconnected by a host controller 2082; and an input/output unit having communication interface 2030, a hard disk drive 2040, and a CD-ROM drive 2060 connected to the host controller 2082 by an input/output controller 2084; and a legacy input/output unit having a ROM 2010, a flexible disk drive 2050, and an input/output chip 2070 connected to the input/output controller 2084.

The host controller 2082 connects the RAM 2020 with the CPU 2000 and the graphics controller 2075 accessing the RAM 2020 at a high transfer rate. The CPU 2000 operates based on programs stored in the ROM 2010 and the RAM 2020, and controls each unit. The graphics controller 2075 is configured to acquire image data generated by the CPU 2000 or the like on a frame buffer provided inside the RAM 2020 and display the image data on the display apparatus 2080. Alternatively, the graphics controller 2075 may include therein a frame buffer storing the image data generated by the CPU 2000 or the like. Various types of information generated inside the information processing device 170 (for example, an image, position information on the manipulation target 35, and the like) can be displayed on the display apparatus 2080.

The input/output controller 2084 connects the communication interface 2030, the hard disk drive 2040, and the CD-ROM drive 2060 which are relatively fast input/output apparatuses to the host controller 2082. The communication interface 2030 is configured to communicate with other apparatuses via a network by wire or wirelessly. In addition, the communication interface is configured to function as a hardware to perform communications. The hard disk drive 2040 stores a program and data to be used by the CPU 2000 in the computer 1900. The CD-ROM drive 2060 is configured to read a program or data from the CD-ROM 2095 and provide the hard disk drive 2040 through the RAM 2020.

In addition, the ROM 2010, and the flexible disk drive 2050 and input/output chip 2070, which are relatively low-speed input/output apparatuses, are connected to the input/output controller 2084. The ROM 2010 stores a boot program performed when the computer 1900 starts up, and/or a program relying on the hardware of the computer 1900, and the like. The flexible disk drive 2050 is configured to read out a program or data from the flexible disk 2090, and provide it to the hard disk drive 2040 via the RAM 2020. The input/output chip 2070 connects the flexible disk drive 2050 to the input/output controller 2084, and connects various types of input/output apparatuses to the input/output controller 2084, for example, via a parallel port, a serial port, a keyboard port, a mouse port, or the like.

The program provided to the hard disk drive 2040 via the RAM 2020 is stored in a recording medium, such as the flexible disk 2090, the CD-ROM 2095, or an IC card, and provided by a user. The program is read out from the recording medium, installed on the hard disk drive 2040 in the computer 1900 via the RAM 2020, and executed in the CPU 2000.

A program that is installed on the computer 1900 for causing the computer 1900 to function as the information processing device 170 includes an air bubble forming module, an energy controlling module, and a manipulation module. These programs or modules may act on the CPU 2000 and the like, and cause the computer 1900 to function as the air bubble forming unit 200, the liquid control unit 260, and the like.

Information processing written in these programs are read into the computer 1900, thereby functioning as the air bubble forming unit 200, the liquid control unit 260 and the like, which are specific means in which a software and the various hardware resources described above are in cooperation. With these specific means, an operation or processing of information according to an intended use of the computer 1900 in the present embodiment is achieved, and the information processing device 170 that is particular according to the intended use is constructed.

By way of example, when communication is performed between the computer 1900 and an external apparatus or the like, the CPU 2000 is configured to execute the communication program loaded on the RAM 2020, and provide the communication interface 2030 with communication processing instructions based on the content of the processing written in the communication program. In response to the control by the CPU 2000, the communication interface 2030 is configured to read out the transmission data stored in the transmission buffer region or the like provided on the storage apparatus, such as the RAM 2020, the hard disk drive 2040, the flexible disk 2090, the CD-ROM 2095, or the like, and transmit this transmission data to the network, and write reception data received from the network onto a reception buffer region or the like provided on the storage apparatus. In this manner, the communication interface 2030 may transfer transmission/reception data to the storage apparatus through DMA (Direct Memory Access) scheme, and alternatively, the CPU 2000 may transfer the transmission/reception data by reading the data from the storage apparatus or communication interface 2030 that are the origins of transfer, and writing the data onto the communication interface 2030 or the storage apparatus that are the destinations of transfer.

In addition, the CPU 2000 causes all or necessary portions of files or database stored in an external storage apparatus such as the hard disk drive 2040, the CD-ROM drive 2060 (CD-ROM 2095), and the flexible disk drive 2050 (flexible disk 2090) to be read into the RAM 2020 by means of DMA transfer or the like, and then performs various types of processing on the data in the RAM 2020. The CPU 2000 writes back the data on which processing is completed into an external storage apparatus by DMA transfer or the like. In such processing, the RAM 2020 can be regarded as carrying contents of the external storage apparatus temporarily, and thus the RAM 2020, the external storage apparatus and the like are collectively called a memory, a recording unit, a storage apparatus or the like in the present embodiment.

The storage apparatus or the like stores therein, as necessary, necessary information for information processing of the information processing device 170 such as, for example, motion image data, and supplies them to each component of the information processing device 170 as necessary.

Various types of information such as various types of programs, data, tables, databases or the like in the present embodiment according to the present embodiment are stored on such a storage apparatus, and are subjected to information processing. Note that, the CPU 2000 can carry a part of the RAM 2020 in a cache memory and read from or write to the cache memory. In such a configuration as well, the cache memory serves a part of the function of the RAM 2020, and therefore the cache memory is also included with the RAM 2020, the memory, and/or the storage apparatus in the present embodiment, except when it is shown with distinction.

In addition, the CPU 2000 is configured to execute various types of processing including various types of computations, information processing, conditional determination, information search/replacement, or the like described in the present embodiment for the data read from the RAM 2020, as specified by the instruction sequence of the program, and writes the result back onto the RAM 2020. For example, when performing conditional determination, the CPU 2000 compares various types of variables shown in the present embodiment to determine whether they satisfy conditions such as being larger than, smaller than, equal to or greater than, less than or equal to, equal to or like other variables or constants, and if a condition is satisfied (or if it is not satisfied) branches to a different instruction sequence or calls up a subroutine.

In addition, the CPU 2000 can search for information stored in a file in the storage apparatus or the database, or the like. For example, if a plurality of entries, each having an attribute value of a second attribute associated with an attribute value of a first attribute, are stored in a storage apparatus, the CPU 2000 searches, from among the plurality of entries stored in the storage apparatus, an entry having an attribute value of the first attribute that matches a specified condition, and reads out the attribute value of the second attribute stored in the entry, and it is thereby possible to obtain the attribute value of the second attribute associated with the first attribute that satisfies a predetermined condition.

The programs or modules shown above may also be stored in an external recording medium. As a recording medium, other than the flexible disk 2090 and the CD-ROM 2095, an optical recording medium such as DVD or CD, a magneto-optical recording medium such as MO, a tape medium, a semiconductor memory, such as IC card, or the like can be used. Also, a storage apparatus such as a hard disk or RAM that is provided with a server system connected to the Internet or a specialized communication network may be used as the recording medium to provide the programs to the computer 1900 via the network.

The present disclosure presented a configuration that the information processing device 170 has the CPU 2000 as a processor, but the type of the processor is not particularly limited. For example, as a processor, GPU, ASIA, FPGA or the like can be used appropriately. In addition, the present disclosure presented a configuration that the information processing device 170 has the hard disk drive 2040 as an auxiliary storage apparatus, but the type of the auxiliary storage apparatus is not particularly limited. For example, instead of the hard disk drive 2040 or in addition to the hard disk drive 2040, another storage apparatus such as a solid state drive may be used.

While the present invention has been described with the embodiments, the technical scope of the present invention is not limited to the above-described embodiments. It is apparent to persons skilled in the art that various alterations and improvements can be added to the above-described embodiments. It is also apparent from the scope of the claims that the embodiments added with such alterations or improvements can be included in the technical scope of the present invention.

The operations, procedures, steps, and stages of each process performed by a device, system, program, and method shown in the claims, embodiments, or diagrams can be performed in any order as long as the order is not indicated by "prior to," "before," or the like and as long as the output from a previous process is not used in a later process. Even if the operation flow is described by using phrases such as "first" or "next" in the scope of the claims, specification, or drawings, it does not necessarily mean that the process must be performed in this order.

### (Additional Remarks)

(Claim 1) A manipulating method of an organism, including:
   forming an air bubble in liquid where the organism is immersed;
   attaching the organism to the air bubble; and
   controlling an airflow by generating the airflow in the air bubble and manipulating a position of the organism with the airflow.
(Claim 2) The manipulating method according to claim 1, wherein
   the forming the air bubble is performed by immersing an end part of a flow channel in liquid, and introducing gas from the end part into the liquid, and
   the controlling the airflow includes generating the airflow in the air bubble by moving a relative position between the flow channel and the liquid.
(Claim 3) The manipulating method according to claim 2, wherein
   the forming the air bubble includes forming and maintaining the air bubble on the end part of the flow channel.
(Claim 4) The manipulating method according to claim 2 or 3, wherein
   the controlling the airflow includes generating the airflow in the air bubble by moving the relative position between the flow channel and the liquid, in a direction within a range of ± 20° from a horizontal direction.
(Claim 5) The manipulating method according to any one of claims 2 or 4, wherein
   the controlling the airflow includes generating the airflow in the air bubble by moving the relative position between the flow channel and the liquid, in a direction within a range of ± 20° from a vertical direction.
(Claim 6) The manipulating method according to any one of claims 2 to 5, wherein
   the liquid and the organism are contained in a vessel, and
   the controlling the airflow includes moving the relative position between the flow channel and the liquid in a state where the air bubble is in contact with a bottom portion of the vessel.
(Claim 7) The manipulating method according to any one of claims 1 to 6, wherein
   the controlling the airflow includes generating the airflow in the air bubble by changing a volume of the air bubble.
(Claim 8) The manipulating method according to claim 7, wherein
   the controlling the airflow includes generating the airflow in the air bubble by increasing the volume of the air bubble.
(Claim 9) The manipulating method according to claim 7, wherein
   the controlling the airflow includes generating an airflow in the air bubble by reducing the volume of the air bubble.
(Claim 10) The manipulating method according to any one of claims 1 to 9, wherein
   the forming the air bubble is performed by immersing an end part of a flow channel in liquid, and introducing gas from the end part into the liquid,
   the flow channel includes a gas supply flow channel for supplying the gas, and a gas recovery flow channel for recovering the gas, and
   the controlling the airflow includes generating an airflow where the gas supplied from an end part of the gas supply flow channel passes through the air bubble, and flows toward an end part of the gas recovery flow channel.
(Claim 11) The manipulating method according to claim 10, wherein
   the flow channel includes a double tube structure,
   the gas supply flow channel is one flow channel on an inner side or an outer side in the double tube structure, and
   the gas recovery flow channel is an other flow channel on the inner side or the outer side in the double tube structure.
(Claim 12) The manipulating method according to any one of claims 1 to 11, further including recovering the organism from the liquid after the controlling the airflow.
(Claim 13) An organism manipulation device for manipulating an organism, including:
   a flow channel that can introduce gas into liquid, an end part of the flow channel being immersed in the liquid where the organism is immersed, and
   an air bubble control unit which generates an airflow in an air bubble formed by introducing the gas from the end part into the liquid and manipulating a position of the organism with the airflow.
(Claim 14) The organism manipulation device according to claim 13, wherein
   the air bubble control unit controls to maintain the air bubble formed on the end part.
(Claim 15) The organism manipulation device according to claim 13 or 14, wherein
   the air bubble control unit includes a flow channel position control unit which controls an actuator for moving the flow channel, thereby controlling the airflow in the air bubble.
(Claim 16) The organism manipulation device according to any one of claims 13 to 15, wherein
   the air bubble control unit includes a stage position control unit which controls an actuator for moving a stage that is equipped with a vessel containing the organism, thereby controlling the airflow in the air bubble.
(Claim 17) The organism manipulation device according to any one of claims 13 to 16, wherein
   the air bubble control unit includes a volume control unit which controls a pump that is connected to the flow channel, thereby controlling a volume of the air bubble in the liquid.
(Claim 18) The organism manipulation device according to any one of claims 13 to 17, wherein:
   the flow channel includes a gas supply flow channel for supplying the gas and a gas recovery flow channel for recovering the gas, and
   the air bubble control unit includes
   a gas supply control unit which controls a pump that is connected to the gas supply flow channel, thereby controlling an amount of the gas to be supplied to the gas supply flow channel, and
   an intake control unit which controls a pump that is connected to the gas recovery flow channel, thereby controlling an amount of the gas to be recovered from the gas recovery flow channel.
(Claim 19) A computer program having an instruction inside, wherein
   upon execution of the instruction in a processor or a programmable circuit,
   the processor or the programmable circuit controls operations including:
      forming an air bubble in liquid where an organism is immersed;
      attaching the organism to the air bubble; and
      controlling an airflow by generating an airflow in the air bubble and manipulating a position of the organism with the airflow.
(Claim 20) An organism manipulation device, including:
   a flow channel having an end part arranged in liquid including an organism, the liquid being stored in a vessel;
   a pump for introducing gas into the flow channel to form an air bubble; and
   a position control unit being able to change a position of the vessel or the flow channel,
   wherein the pump or the position control unit attaches the organism to a gas-liquid interface of the air bubble, and
   the position control unit moves the flow channel in an opposing direction of the organism with a central axis of the flow channel as a reference, in a vertical directional view.

### EXPLANATION OF REFERENCES

1: fluorescence image observation light source;
2: dichroic mirror;
3: optical deflector;
4: relay lens;
5: dichroic mirror;
6: objective lens;
7: condenser lens;
8: condensing lens;
9: band pass filter;
10: transmission image observation light source;
11: barrier filter;
12: projection lens;
13: barrier filter;
14: projection lens;
15: pinhole;
16: light source;
17: light source;
25: vessel;
25a: bottom portion;
35: manipulation target;
40: nozzle actuator;
41: sample actuator;
42: flow channel imaging camera;
45: light source;
46: light source;
47: pressure generating unit;
48: sensor unit;
49: nozzle;
49a: double tube nozzle;
49b: inner tube;
49c: outer tube;
50: microscope unit;
51: flow channel;
51a: first flow channel;
51b: second flow channel;
53: flow channel exchange unit;
54: liquid storage part;
60: camera;
70: camera;
100: organism manipulation device;
101: manipulation unit;
111: display area;
112: display area;
113: display area;
114: display area;
115: display area;
160: output unit;
170: information processing device;
171: imaging control unit;
180: input unit;
190: recording unit;
200: air bubble forming unit;
250: flow channel control unit;
251: pump;
251a: first pump;
251b: second pump;
253: tubular portion;
253a: outer tube;
253b: inner tube;
254: end part;
255: gas-liquid interface;
256: air bubble;
260: liquid control unit;
261: liquid;
300: image processing unit;
1900: computer,
2000: CPU;
2010: ROM;
2020: RAM;
2030: communication interface,
2040 hard disk drive,
2050: flexible disk drive,
2060: CD-ROM drive,
2070: input/output chip,
2075: graphics controller,
2080: display apparatus;
2082: host controller,
2084: input/output controller,
2090: flexible disk,
2095: CD-ROM.

## Claims

1. A manipulating method of an organism, comprising:
forming an air bubble in liquid in which the organism is immersed;
attaching the organism to the air bubble; and
controlling an airflow by generating the airflow in the air bubble and manipulating a position of the organism with the airflow.

2. The manipulating method according to claim 1, wherein
the forming the air bubble is performed by immersing an end part of a flow channel in liquid, and introducing gas from the end part into the liquid, and
the controlling the airflow comprises generating the airflow in the air bubble by moving a relative position between the flow channel and the liquid.

3. The manipulating method according to claim 2, wherein
the forming the air bubble comprises forming and maintaining the air bubble on the end part of the flow channel.

4. The manipulating method according to claim 2 or 3, wherein
the controlling the airflow comprises generating the airflow in the air bubble by moving a relative position between the flow channel and a surface of a vessel storing the liquid that is in contact with the organism, in a direction within a range of ± 20° from a horizontal direction.

5. The manipulating method according to any one of claims 2 to 4, wherein
the controlling the airflow comprises generating the airflow in the air bubble by moving a relative position between the flow channel and a surface of a vessel storing the liquid that is in contact with the organism, in a direction within a range of ± 20° from a vertical direction.

6. The manipulating method according to any one of claims 2 to 5, wherein
the liquid and the organism are contained in a vessel, and
the controlling the airflow comprises moving the relative position between the flow channel and the liquid, in a state where the air bubble is in contact with a surface of the vessel that is in contact with the organism.

7. The manipulating method according to any one of claims 1 to 6, wherein
the controlling the airflow comprises generating the airflow in the air bubble by changing a volume of the air bubble.

8. The manipulating method according to claim 7, wherein
the controlling the airflow comprises generating the airflow in the air bubble by increasing the volume of the air bubble.

9. The manipulating method according to claim 7, wherein
the controlling the airflow comprises generating the airflow in the air bubble by reducing the volume of the air bubble.

10. The manipulating method according to any one of claims 1 to 9, wherein
the forming the air bubble is performed by immersing an end part of a flow channel in liquid, and introducing gas from the end part into the liquid,
the flow channel comprises a gas supply flow channel for supplying the gas and a gas recovery flow channel for recovering the gas, and
the controlling the airflow comprises generating an airflow where the gas supplied from an end part of the gas supply flow channel passes through the air bubble, and flows toward an end part of the gas recovery flow channel.

11. The manipulating method according to claim 10, wherein
the flow channel comprises a double tube structure,
the gas supply flow channel is one flow channel on an inner side or an outer side in the double tube structure, and
the gas recovery flow channel is an other flow channel on the inner side or the outer side in the double tube structure.

12. The manipulating method according to any one of claims 1 to 11, further comprising
recovering the organism from the liquid after the controlling the airflow.

13. An organism manipulation device for manipulating an organism, comprising:
a flow channel that can introduce gas into liquid, an end part of the flow channel being immersed in the liquid where the organism is immersed, and
an air bubble control unit which generates an airflow in an air bubble formed by introducing the gas from the end part into the liquid and manipulates a position of the organism with the airflow.

14. The organism manipulation device according to claim 13, wherein
the air bubble control unit controls to maintain the air bubble formed on the end part.

15. The organism manipulation device according to claim 13 or 14, wherein
the air bubble control unit comprises a flow channel position control unit which controls an actuator for moving the flow channel, thereby controlling the airflow in the air bubble.

16. The organism manipulation device according to any one of claims 13 to 15, wherein
the air bubble control unit comprises a stage position control unit which controls an actuator for moving a stage that is equipped with a vessel containing the organism, thereby controlling the airflow in the air bubble.

17. The organism manipulation device according to any one of claims 13 to 16, wherein
the air bubble control unit comprises a volume control unit which controls a pump that is connected to the flow channel, thereby controlling a volume of the air bubble in the liquid.

18. An organism manipulation device, comprising:
a flow channel having an end part arranged in liquid including an organism, the liquid being stored in a vessel;
a pump which introduces gas into the flow channel to form an air bubble; and
a position control unit being able to change a position of the vessel or the flow channel,
wherein the pump or the position control unit attaches the organism to a gas-liquid interface of the air bubble, and
the position control unit moves the flow channel in an opposing direction of the organism with a central axis of the flow channel as a reference, in a vertical directional view.
